# EUROPEAN PATENT SPECIFICATION

(11) **EP 2 121 939 B1**
(45) Date of publication and mention of the grant of the patent: **04.12.2013**
(21) Application number: 08737478.1
(22) Date of filing: 18.01.2008
(51) Int. Cl.: C12N 15/82

(54) **METHODS FOR MODULATING THE SIRNA AND RNA-DIRECTED-DNA METHYLATION PATHWAYS**
VERFAHREN ZUR MODULIERUNG DER SIRNA- UND RNA-GERICHTETEN DNA-METHYLIERUNGSPFADE
PROCÉDÉS DESTINÉS À MODULER LES VOIES DE MÉTHYLATION DE L'ADN DIRIGÉ PAR L'ARN ET LES PETITS ARNI

(30) Priority: 19.01.2007 US 881418 P
(43) Date of publication of application: 25.11.2009
(73) Proprietor: Plant Bioscience Limited, Norwich, Norfolk NR4 7UH (GB); Navarro, Lionel, 67084 Strasbourg Cedex (FR); Voinnet, Olivier, 67084 Strasbourg Cedex (FR)
(72) Inventor: NAVARRO, Lionel, F-67084 Strasboug Cedex (FR); VOINNET,Oliver, F-67084 Strasbourg Cedex (FR)
(74) Representative: Novagraaf Technologies
(86) International application number: PCT/IB2008/000954
(87) International publication number: WO 2008/087561

(56) References cited:
- EP-A- 0 480 236
- WO-A-02/20791
- MATZKE ET AL: "Targets of RNA-directed DNA methylation" CURRENT OPINION IN PLANT BIOLOGY, QUADRANT SUBSCRIPTION SERVICES, GB, vol. 10, no. 5, 4 October 2007 (2007-10-04), pages 512-519, XP022286650 ISSN: 1369-5266
- ZHU J. ET AL.: "The DNA glycosylase/lyase ROS1 functions in pruning DNA methylation patterns in Arabidopsis" CURRENT BIOLOGY, vol. 17, 9 January 2007 (2007-01-09), pages 54-59, XP002498460
- ZHU J. ET AL: "The DNA glycosylase/lyase ROS1 functions in pruning DNA methylation patterns in Arabidopsis" CURRENT BIOLOGY, vol. 17, 9 January 2007 (2007-01-09), pages S1-S8, XP002498461
- DUNOYER P ET AL: "The complex interplay between plant viruses and host RNA-silencing pathways" CURRENT OPINION IN PLANT BIOLOGY, QUADRANT SUBSCRIPTION SERVICES, GB, vol. 8, no. 4, 1 August 2005 (2005-08-01), pages 415-423, XP004944570 ISSN: 1369-5266
- AGIUS F. ET AL.: "Role of the Arabidopsis DNA glycosylase/lyase ROS1 in active DNA demethylation" PNAS, vol. 103, 1 August 2006 (2006-08-01), pages 11796-11801, XP002498462
- CHAN S.W.L. ET AL: "Gardening the genome: DNa methylation in arabidopsis thaliana" NATURE, vol. 6, May 2006 (2006-05), pages 351-360, XP002498463
- STOKES T.L. ET AL: "Epigenetic variation in Arabidopsis disease resistance" GENES AND DEVELOPMENT, vol. 16, 2002, pages 171-182, XP002498464
- GONG Z. ET AL.: "ROS1, a repressor of transcriptional gene silencing in Arabidopsis, encodes a DNA glycosylase/lyase" CELL, vol. 111, 13 December 2002 (2002-12-13), pages 803-814, XP002498465
- MORALES-RUIZ T. ET AL: "DEMETER and REPRESSOR OF SILENCING encode 5-methylcytosine DNA glycosylase" PNAS, vol. 103, no. 18, 2 May 2006 (2006-05-02), pages 6853-6858, XP002498466

## Description

### FIELD OF THE INVENTION

Compositions and methods for conferring broad spectrum pathogen resistance, against plant pathogens.

### BACKGROUND OF THE INVENTION

In recent years, there has been an ever increasing appreciation of the complexity and pleiotropic effects of gene silencing and components of the gene silencing machinery. From effects observed initially via transgene suppression of endogenous gene expression in petunia plants, has emerged an understanding of a penumbra of effects in plants and animals spanning maintenance of control over transposons to control over the methylation state, and indeed transcriptional activity, of chromatin.

### Small RNA. Dicers and Argonautes: the biochemical core of RNA silencing

"RNA silencing" refers collectively to diverse RNA-based processes that all result in sequence-specific inhibition of gene expression, either at the transcription, mRNA stability or translational levels. Those processes share three biochemical features: (i) formation of double-stranded (ds)RNA, (ii) processing of dsRNA to small (s) 20-26nt dsRNAs with staggered ends, and (iii) inhibitory action of a selected sRNA strand within effector complexes acting on partially or fully complementary RNA/DNA. While several mechanisms can generate dsRNA, the sRNA processing and effector steps have a common biochemical core. sRNAs are produced by RNAseIII-type enzymes called Dicers¹ with distinctive dsRNA binding, RNA helicase, RNAseIII and PAZ (Piwi/Argonaute/Zwille) domains. One of the two sRNA strands join effector complexes called RISCs (RNA-induced silencing complex) that invariably contain a member of the Argonaute (Ago) protein family. Agos have an sRNA binding PAZ domain and also contain a PIWI domain providing endonucleolytic ('slicer') activity to those RISCs programmed to cleave target RNAs^{2,3}. In fact, sRNA-loaded human Ago2 alone constitutes a cleavage-competent RISC *in vitro,* but many additional proteins may be functional components of RISCs *in vivo*⁴*.*

Here, we review recent evidence that several pathways built over the Dicer-Ago core execute a diverse set of sRNA-directed biological functions in higher plants. These include regulation of endogenous gene expression, transposon taming, viral defense and heterochromatin formation. Our focus is primarily on plants because they exhibit a nearly full spectrum of known RNA silencing effects, but similarities and differences with other organisms are also discussed. Exogenously triggered RNA silencing pathways resulting in transcript cleavage

### dsRNA-producin transgenes and IR-PTGS: useful, but mysterious

Post-transcriptional gene silencing (PTGS) was discovered in transgenic *Petunia* as loss of both transgene (in either sense or antisense configuration) and homologous endogenous gene expression⁵. The transgene loci often produced dsRNA because they formed arrays with complex integration patterns^{6,7}. Accordingly, PTGS efficacy was greatly enhanced by simultaneous sense and antisense expression⁸ or by direct production of long dsRNA from inverted-repeat (IR) transgenes⁹. The latter process, IR-PTGS, currently forms the basis of experimental RNAi in plants, and involves at least two distinct sRNA classes termed short interfering (si)RNAs. 21 nt siRNAs are believed to guide mRNA cleavage, while 24nt siRNAs may exclusively mediate chromatin modifications^{10, 11}. Both siRNA classes accumulate as populations along the entire sequence of IR transcripts¹². Although widely used as a research tool, IR-PTGS remains one of the least understood plant RNA silencing processes. An inverted repeat (IR) transgene construct, typically employed for RNAi in plants, produces double-stranded (ds) transcripts with perfectly complementary arms. Two distinct Dicer-like (DCL) enzymes process the ds transcripts. DCL3 most likely produces siRNAs of the 24nt size class, which may direct DNA/histone modification at homologous loci and appear dispensable for RNA cleavage. Figure 3 illustrates two of many non-mutually exclusive scenarios that possibly account for siRNA-directed chromatin modifications at endogenous loci. Note that both scenarios are based on circular and amplified schemes in which siRNA production and chromatin modification reinforce one another. DCL4 is likely the preferred enzyme for production of 21nt-long siRNAs from the dsRNA. One siRNA strand incorporates into AGO1-loaded RISC to guide endonucleolytic cleavage of homologous RNA, leading to its degradation. Both siRNA species are protected from degradation by addition of methyl groups at the 3' termini of each RNA strand, by the methyl-transferase HEN1. Hence, until recently, no mutant defective in this pathway had been recovered, despite considerable efforts in several laboratories. One likely explanation is that the high dsRNA levels produced in IR-PTGS promote the activities of different Dicers and RISCs, which would normally act in distinct pathways, to redundantly mediate silencing. Recent analyses of combinatorial Dicer knockouts in *Arabidopsis* support this idea^{13,14}. Nonetheless, Dicer-like 4 (DCL4) seems a preferred enzyme for IR-PTGS because it was specifically required for 21nt siRNA accumulation and silencing from a moderately expressed, phloem-specific IR transgene¹⁵. DCL2 might also be involved in RNAi, because it processes some endogenous DCL4 substrates into 22nt-long siRNAs in the absence of DCL4^{13,14}, although it remains unclear if those molecules can functionally substitute for the 21nt siRNA products of DCL4.

### S-PTGS and transitive silencing: enter RDR

There are several examples in which single-copy transgene insertions producing sense transcripts trigger PTGS. This pathway, sense (S)-PTGS, has been dissected using *Arabidopsis* forward-genetic screens that provided insights into how dsRNA is produced. The pathway is shown here as being elicited by RNAs with aberrant features, although there might be alternative triggers. The RNA aberrations could include lack of a poly-A tail or lack of 5' capping. The latter would normally lead to RNA degradation through the activity of the 5'-3' exonuclease XRN4. Lack of XRN4 would promote accumulation of uncapped mRNA, thereby triggering their conversion into dsRNA by the combined action of RDR6, SGS3, SDE3 and, possibly, WEX. The resulting dsRNA is then processed by a DCL, most likely DCL4 (see text), producing siRNAs that are exclusively of the 21nt size class and methylated by HEN1. These molecules can engage into two sets of reactions. First, they can be used as primers by RDR6 to reinforce production of dsRNA from single-stranded templates through a phenomenon known as 'transitivity'. In transitive RNA silencing, a dsRNA source of primary siRNAs promotes production of secondary siRNAs both 5' and 3' of the initially targeted interval of a transcript. Production of 5' secondary siRNAs (case 1) can be explained by RDR6/SGS3/SDE3-dependent complementary strand synthesis that is primed by one of the primary siRNAs. Production of 3' secondary siRNAs (case 2) cannot be explained by a primed reaction, and it is possible that RNA fragments resulting from primary siRNA-directed transcript cleavage are recognized as aberrant, thereby initiating dsRNA synthesis as in S-PTGS. The 5' and 3' reactions should not be considered mutually exclusive, as siRNAs produced in (2) could prime further dsRNA synthesis according to the scheme depicted in (1). DCL4 is shown as putatively involved in 5' and 3' secondary siRNA biogenesis. Unlike primary siRNAs (which can be 21nt and 24nt in size), secondary siRNA are exclusively of the 21nt size class. It remains unclear whether 24nt primary siRNAs can trigger transitive RNA silencing. They can also incorporate into AGO1-loaded RISC to guide sequence-specific cleavage of homologous RNA. The resulting cleavage products could be perceived as aberrant RNAs and, thus, could promote further production of dsRNA, resulting in an amplified reaction. These screens converged on the identification of the RNA-dependent RNA polymerase RDR6, one of six putative *Arabidopsis* RDRs^{16, 17}. RDR6 is thought to recognize and to use as templates certain transgene transcripts with aberrant features that include lack of 5' capping. For instance, mutation of *Arabidopsis* XRN4, a 5'-3' exonuclease that degrades uncapped mRNAs, enhanced accumulation of uncapped transgene mRNAs. This favored their conversion into dsRNA by RDR6 and the subsequent degradation of all transgene transcripts through the S-PTGS pathway¹⁸. RDR6 most likely synthesizes complementary strands from its RNA templates, resulting in dsRNA production, because a missense mutation in the GDD motif, essential for the catalytic activity of all characterized RDRs, is sufficient to alleviate S-PTGS¹⁷.

Although the Dicer producing siRNAs from RDR6 products remains to be normally identified, S-PTGS siRNA accumulation in *Arabidopsis* requires the coiled-coil protein of unknown function SGS3¹⁷, the RNAseD exonuclease WEX¹⁹, the sRNA-specific methyl transferase HEN1²⁰ and the putative RNA helicase SDE3²¹. Unlike RDR6, SDE3 is not stringently required for transgene silencing, and so could accessorily resolve the secondary structures found in RDR templates²¹. Accordingly, an SDE3 homologue is part of the *Schizosaccharomyces pombe* RDR complex²². SDE3 could also act at other RNA silencing steps because the homologous protein Armitage is required for RISC assembly in *Drosophilia,* an organism deprived of *RDR* genes²³. WEX is related to the exonuclease domain of *mut-7.* required for transposon silencing and RNAi in *C*. *elegans* but its role in S-PTGS remains elusive²⁴. HEN1-catalyzed methylation of free hydroxy termini protects *Arabidopsis* sRNAs, including S-PTGS siRNAs, from oligo-uridylation, a modification promoting their instability (see the miRNA section of this review)²⁵.

In one S-PTGS mutant screen, an extensive allelic series of *ago1* was recovered, arguing that among the 10 *Arabidopsis AGO* paralogs, AGO1 is specifically involved in this pathway^{26, 27}. Even weak *ago1* alleles completely lost S-PTGS siRNAs, initially suggesting a role for AGO1 in siRNA production rather than action²⁷. However, since AGO1 is now recognized as a slicer activity of the plant miRNA- and siRNA-loaded RISCs^{28, 29}, loss of siRNAs in *ago1* may also result from their poor incorporation into RISC, enhancing their turnover. Nevertheless, a role for AGO1 in siRNA production - possibly linked to RDR6-dependent dsRNA synthesis - cannot be excluded because some *ago1* mutants defective in S-PTGS siRNA accumulation show no defects in IR-PTGS³⁰.

RDR6, and perhaps other S-PTGS components, is also involved in the related silencing phenomenon, transitivity^{31, 32}. Transitivity is the "transition" of primary siRNAs (corresponding to a sequence interval of a targeted RNA) to secondary siRNAs targeting regions outside the initial interval. In plants, this transition may occur both 5' and 3' to the primary interval, possibly reflecting primer-dependent and primer-independent RDR6 activities. Transitivity serves as a siRNA amplification mechanism that also accounts for extensive movement of silencing throughout transgenic plants³³. Secondary siRNAs are exclusively of the 21nt size class³³. Thus, given that S-PTGS siRNAs seem to accumulate as 21nt species³², that DCL4 produces the 21nt siRNAs from IR transcripts¹⁵, and that DCL4 and RDR6 activities are coupled for 21nt *trans*-acting siRNA biogenesis (see below), it is tempting to speculate that DCL4 is also the preferred Dicer for siRNA production in both S-PTGS and transitivity.

What would be the biological function of an amplified and non-cell autonomous pathway based on 21nt siRNAs? At least one answer is antiviral defense. Virus-derived 21 nt siRNAs accumulate in infected cells³⁴ and plants compromised for RDR6 function are hypersusceptible to several viruses^{17, 35}. An RDR-amplified response primed by viral siRNAs (transitivity) and/or elicited by viral-derived aberrant RNAs (S-PTGS pathway) would ensure that the silencing machinery keeps pace with the pathogen's high replication rates. The systemic nature of the response would immunize cells that are about to be infected, resulting, in some cases, in viral exclusion. Consistent with this idea, the meristems of *Nicotiana benthamiana* with compromised RDR6 activity became invaded by several viruses, whereas those tissues are normally immune to infection³⁶.

### Endogenous RNA silencing pathways involved in post-transcriptional regulations

### MicroRNAs

In plants, miRNAs are produced as single-stranded, 20-24nt sRNA species, excised from endogenous non-coding transcripts with extensive fold-back structure. siRNAs act in *trans* on cellular target transcripts to induce their degradation *via* cleavage, or to attenuate protein production. Primary (pri) miRNA transcripts with fold-back structures are products of RNA polymerase II (Pol II). The position of the mature miRNA is boxed. The combined nuclear action of DCL1, HYL1 and HEN1 produces a mature, methylated miRNA. Upon nuclear export, possibly mediated by the *Arabidopsis* exportin 5 homolog HASTY, the mature miRNA incorporates into AGO1-loaded RISC to promote two possible sets of reactions that are not mutually exclusive. A first reaction would lead to endonucleolytic cleavage of homologous RNA, as directed by 21nt siRNAs. This would result in a poly-urydilated 5' cleavage fragment - a modification that might promote its rapid turnover - and a more stable 3' fragment that could be degraded by the XRN4 exonuclease. The scheme also accommodates die possibility that mature miRNAs could have sequence-specific effects in the nucleus (see text). Those nuclear activities include RNA cleavage (upon incorporation into a putative nuclear RISC) as well as DNA methylation. Currently, approximately 100 *Arabidopsis MIRNA* genes falling into 25 distinct families have been identified³⁸, but many more are likely to exist (Box 1). miRNAs have important biological roles in plant and animal development, as evidenced by the strong developmental defects of several miRNA overexpression and loss-of-function mutants³⁷. For instance, key regulatory elements of the plant response to the hormone auxin, which specifies organ shape and the axes of the plant body, are controlled by miRNAs^{39,40}. miRNAs also regulate accumulation of transcription factors (TFs) involved in floral organ identity/number^{41,42}, leaf shape⁴³, abaxial/adaxial leaf asymmetry^{44,45} and lateral root formation⁴⁶. In addition, DCL1 and AGO1, involved in the miRNA pathway, are themselves regulated by miRNAs^{47,48}. Nonetheless, plant miRNAs with validated targets involved in primary and secondary metabolism have been identified^{39.49}, indicating that their roles are not confined to developmental regulations. miRNAs might, indeed, have broad implications in plant physiology and environmental adaptation (Box 1).

### miRNA transcription and biogenesis

Most plant and animal miRNA genes reside between protein coding genes or within introns⁵⁰. Most are likely to be independent transcription units and their expression patterns often show exquisite tissue- or even cell-type specificity, in agreement with a role in patterning and maintenance of differentiated cell states^{51,52}. Nonetheless, transcription factors or post-transcriptional mechanisms that specify plant miRNA gene expression remain unknown. Many human primary miRNA transcripts (pri-miRNAs) are synthesized by RNA polymerase II (Pol II), because pri-miRNAs have typical Pol II 5'caps and poly-A tails, their synthesis is inhibited by PolII-inhibiting drugs, and PolII is found at their promoters *in vivo*⁵³. Similar, though less extensive, evidence also points to PolII as the major polymerase producing plant pri-miRNAs³⁸.

Upon transcription, mammalian pri-miRNAs are processed via a well-defined biosynthetic pathway. The RNAseIII Drosha and its essential cofactor DGCR8/Pasha - both constituents of the nuclear Microprocessor complex - catalyze initial cuts at the basis of pri-miRNAs stem-loop to produce pre-miRNAs. Pre-miRNAs are processed by Dicer into mature miRNAs upon Exportin-5-dependent nuclear export⁵⁴. Plants have no direct equivalent of Microprocessor. In *Arabidopsis,* miRNA biosynthesis depends specifically upon DCL1^{55, 56}, required for the nuclear stepwise processing of pri-miRNAs, but whether DCL1 itself catalyzes all of the reactions involved is uncertain⁵⁷. The plant exportin-5 homolog HASTY is involved in miRNA biogenesis⁵⁸, but its exact role is not as clear as in mammals where the Microprocessor pre-miRNA product is an experimentally verified cargo⁵⁹. *Hasty* mutants exhibit decreased accumulation of some, albeit not all, miRNAs in both nuclear and cytoplasmic fractions⁵⁸. These observations support the existence of HASTY-independent miRNA export systems and question whether miRNAs or miRNA-containing complexes are even direct cargoes of HASTY.

In plants and animals, Dicer processing occurs in association with specific dsRNA-binding proteins. First observed with the Dcr2-R2D2 complex required for RISC loading in the *Drosophilia* RNAi pathway⁶⁰, this has now also been found for the Dcr1-Loqs complex involved in the *Drosophila* miRNA pathway⁶¹, and Dicer-TRBP as well as Dicer-PACT in human cells^{62, 63}. DCL1-HYL1 constitutes a sinular complex that acts in pri-miRNA processing in the *Arabidopsis* miRNA pathway.⁶⁴⁻⁶⁷. In all cases, Dicer produces a duplex between the mature miRNA (miR) and its complementary strand (miR*)⁶⁸. The miR strand is generally least stably base-paired at its 5'-end and is, consequently, loaded as the guide strand into RISC, whereas the miR* strand is degraded⁶⁹. In the *Drosophila* RNAi pathway, R2D2 acts as a thermodynamic asymmetry sensor of siRNA duplexes, and Loqs, TRBP, PACT and HYL1 could possibly perform similar roles.

HEN1 is an *S*-adenosyl methionine (SAM)-binding methyl transferase that methylates the 2' hydroxy termini of miR/miR* duplexes, a reaction apparently specific to the plant kingdom^{70, 71}. Methylation protects miRNAs from activities that uridylate and degrade plant sRNAs from the 3'-end²⁵, but it is not required for RISC-dependent miRNA-guided cleavage in *Arabidopsis* extracts²⁸. All known classes of plant sRNAs are methylated by HEN1²⁵, but this modification seems to impact differentially on sRNA stability, perhaps reflecting variable interactions between HEN1 and distinct protein complexes or distinct sRNA populations. For example, the viral silencing suppressor Hc-Pro prevents methylation of virus derived siRNAs, but not of miRNAs ⁷² and several *hen1* mutant alleles exist, in which accumulation of miRNA, but not of S-PTGS siRNAs, is impaired²⁰.

### Plant miRNA activities

Most identified plant miRNAs have near-perfect complementarity to their targets and promote their cleavage. This is followed by oligo-uridylation and rapid degradation of the 5'-cleavage fragment⁷³, and slower degradation of the 3'-cleavage fragment mediated, at least in some cases, by XRN4⁷⁴. Animal miRNAs generally exhibit imperfect complementarity and repress protein production from intact target mRNAs. However, it is possible that the action of both plant and animal miRNAs results from a combination of both processes, whose respective contributions probably vary depending on the extent of the miRNA:target complementarity (Box 2). Although the RISC(s) acting in the plant miRNA pathway remain ill defined, AGO1 associates with miRNAs and miRNA targets are cleaved *in vitro* by immunoaffinity-purified AGO1^{28,29}. Thus, in plants, the same Argonaute appears to function as a Slicer for both miRNA- and siRNA-loaded RISCs, contrasting with the situations in *Drosophila* and *C. elegans.* Plant RISC components other than AGO1 await identification and it may well be that several alternative RISCs exist, given the number of AGO-like genes in *Arabidopsis.*

Mature plant miRNAs are detected in both nuclear and cytosolic cell fractions⁵⁸. Likewise, RISC programmed with the *let-7* miRNA can be immuno-purified from nuclear human cell fractions⁷⁵, indicating that plant and animal miRNAs may have nuclear functions. These may include RNA cleavage, as suggested by the intron-targeting activity of the plant miR173⁷⁶, but could also comprise modifications of homologous DNA⁷⁷. Thus, in *Arabidopsis,* miR165 recognition of the spliced *PHB* transcript apparently directs *cis-*methylation on the *PHB* template DNA. This methylation is enigmatic, however, as it occurs several kb downstream of the miRNA binding site⁷⁷. It is conceivable that miRNA-induced cleavage of the nascent *PHB* transcript triggers dsRNA formation initiated at the 3'-end of the transcript through a primer-independent RDR activity with moderate processivity. The resulting production of siRNA would thus be confined to the 3'-end and could mediate DNA methylation according to the schemes discussed in a further section of this review. Intriguingly, some, albeit few, siRNAs corresponding to downstream parts of several miRNA targets have been detected in *Arabidopsis,* although none were directly complementary to the methylated *PHB* sequence⁷⁸. Direct miRNA-guided DNA methylation in *cis* and/or *trans* has also been suggested from the observation that some 21nt miRNAs of *Arabidopsis* accumulate as a second, 24nt species at specific developmental stages⁶⁸.

### Transacting siRNAs: mixing up miRNA and siRNA actions

Transacting (ta) siRNAs are a recently discovered class of plant endogenous sRNAs. They derive from non-coding, single-stranded transcripts, the pri-tasiRNAs, which are converted into dsRNA by RDR6-SGS3, giving rise to siRNAs produced as discrete species in a specific 21 nt phase^{79, 80}. Primary (pri) trans-acting siRNA transcripts are non-coding RNAs devoid of extensive fold-back structures. A miRNA incorporated into AGO1-loaded RISC guides endonucleolytic cleavage of the pri-tasiRNA. This cut generates two cleavage fragments, one of which acts as an RDR6 template, leading to the production of dsRNA. DCL4 initiates processing exclusively from the dsRNA ends corresponding to the initial miRNA cut site, to produce phased tasiRNAs that are methylated by HEN1. tasiRNA subsequently guide cleavage of homologous mRNAs, once incorporated into AGO1-loaded RISC. The colored reactions depicted in the inlay illustrate the importance of the initial miRNA-directed cut in determining the appropriate phase for tasiRNAs (1). Incorrect phasing (2) would result in the production of off-target small RNAs. The RDR6-SGS3 involvement is reminiscent of siRNA biogenesis in S-PTGS, but the genetic requirements of those pathways are not identical, because tasiRNA accumulation is normal in the hypomorphic *ago1-27* mutant and in mutants defective in SDE3 and WEX⁷⁹. Much like plant miRNAs, mature tasiRNAs guide cleavage and degradation of homologous, cellular transcripts. To date, tasiRNA generating loci (*TAS1-3*) have been only identified in *Arabidopsis*⁷⁶, but they are likely to exist in other plant species and possibly in other organisms that contain RDRs such as *C*. *elegans* or *N. crassa.*

tasiRNA Production involves an interesting mix of miRNA action and the siRNA biogenesis machinery (Box 3). Pri-tasiRNAs contain a binding site for a miRNA that guides cleavage at a defined point. The initial miRNA-guided cut has two important consequences. First, it triggers RDR6-mediated transitivity on the pri-tasiRNA cleavage products, allowing dsRNA production either 5' or 3' of the cleavage site⁷⁶. Second, it provides a well-defined dsRNA terminus crucial for the accuracy of a phased dicing reaction, performed by DCL4, which produces mature tasiRNAs.

What is the biological role of tasiRNAs? *rdr6, sgs3,* and *dcl4* all exhibit accelerated juvenile-to-adult phase transition^{13,14,80,81}, indicating that tasiRNAs could regulate this trait. The tasiRNA targets include two auxin response factor (ARF) TFs and a family of pentatricopeptide repeat proteins, although there is no evidence for the involvement of the only functionally characterized target (ARF3/ETTIN) in juvenile-to-adult phase transition⁸², nor were heterochronic defects noticed in insertion mutants disrupting the *TAS1* or *TAS2* loci^{79,81}. Mutants in *AGO7*/*ZIPPY* display a similar phase transition defect⁸³, suggesting that AGO7 could be part of a specific tasiRNA-programmed RISC, although tasiRNAs do co-immunoprecipitate with AGO1 to form a cleavage competent RISC¹⁸.

### Natural antisense transcript siRNAs

An example has been recently described in which a pair of neighboring genes on opposite DNA strands (cis-antisense genes) gives rise to a single siRNA species from the overlapping region of their transcripts⁸⁴. This 24nt siRNA species - dubbed natural antisense transcript siRNA (nat-siRNA)- guides cleavage of one of the two parent transcripts, and is produced in a unique pathway involving DCL2, RDR6, SGS3 and the atypical DNA dependent RNA polymerase-like subunit NRPD1a (see paragraph on chromatin targeted RNA silencing pathways below). nat-siRNA Guided cleavage triggers production of a series of secondary, phased 21nt siRNAs, a reaction similar to tasiRNA biogenesis except that the Dicer involved is DCL1. The role of secondary nat-siRNAs is currently unclear, but primary nat-siRNA-guided cleavage contributes to stress adaptation, and, given the large number of *cis* antisense gene pairs in plant and other genomes^{85, 86}, this isolated example may reflect a widespread mechanism of gene regulation.

### Chromatin targeted RNA silencing pathways

In addition to acting on RNA, siRNAs can guide formation of transcriptionally silent heterochromatin in fungi, animals and plants. Plant heterochromatin is characterized by two sets of modifications: methylation of cytosines and of specific histone lysine residues (histone 3 Lys9 (H3K9) and histone 3 Lys27 (H3K27) in *Arabidopsis*)⁸⁷. In some organisms, these modifications act as assembly platforms for proteins promoting chromatin condensation. *Arabidopsis* cytosine methyl-transferases include the closely homologous DRM 1/2 required for all *de novo* DNA methylation, MET1 required for replicative maintenance of methylation at CG sites, and CMT3 required for maintenance at CNG and asymmetrical CNN sites (reviewed in ^{88, 89}). Histone methyl-transferases involved in H3K9 and H3K27 methylation belong to the group of Su(Var)3-9 homologues and include KYP/SUVH4 and SUVH2 in *Arabidopsis*⁹⁰.

In several organisms, siRNAs corresponding to a number of endogenous silent loci, including retrotransposons, 5S rDNA and centromeric repeats, have been found⁸⁸. They are referred to as cis-acting siRNAs (casiRNAs) because they promote DNA/histone modifications at the loci that generate them. In plants, casiRNAs are methylated by HEN1 and are predominantly 24nt in size (Box 4)^{25,91}. Their accumulation is specifically dependent upon DCL3 and, in many instances, upon RDR2¹. casiRNA Accumulation also requires an isoform (containing subunits NRPD1a and NRPD2) of a plant-specific and putative DNA-dependent RNA polymerase, termed PolIV⁹²⁻⁹⁴. PolIV may act as a silencing-specific RNA polymerase that produces transcripts to be converted into siRNAs by the actions of RDR2 and DCL3. However, many aspects of PolIV silencing-related activities remain obscure. Hence, it is uncertain whether PolIV even possesses RNA polymerase activity. Additionally, a distinct PolIV isoform with subunits NRPD1b and NRPD2 is required for methylation directed by IR-derived siRNAs with transgene promoter homology, suggesting that the action of PolIV complexes may not be confined to siRNA biogenesis⁹⁵. Finally, the requirement of NRPD1a for nat-siRNA accumulation in the presence of both antisense mRNAs (produced by PolII) suggests that PolIV may have silencing-related functions independent of DNA-dependent RNA polymerase activity⁸⁴. Other factors involved in IR-derived siRNA-directed promoter methylation include the chromatin remodeling factor DRD1⁹⁶ and the putative histone deacetylase HDA6⁹⁷ whose activity may be required to provide free histone lysines for methylation by KYP/SUVH enzymes. It is currently uncertain whether DRD1 and HDA6 are also implicated in silencing of endogenous loci. 24nt siRNAs May act in a RISC-like complex, perhaps akin to the RNA-induced transcriptional silencing complex, RITS, characterized in fission yeast⁹⁸. This complex could contain AGO4 because *ago4* mutants have phenotypes overlapping with those of *rdr2, dcl3, nrpd1a* and *nrpd2*¹¹. At loci affected by the above mutations, CNG and particularly CNN methylation is strongly reduced, whereas loss of CG methylation is less pronounced, consistent with the observation that MET1-dependent promoter CG methylation could be maintained in the absence of a viral-encoded RNA trigger of TGS⁹⁹.

DNA itself or nascent transcripts are both possible targets of casiRNAs. The dsRNA is then processed by DCL3 into 24nt siRNAs that direct further cleavage of nascent transcripts and may possibly guide sequential activities of histone deacetylases (*e.g.,* HDA6), histone methyl transferases (*e.g.,* KYP, SUVH2) and/or DNA methyl-transferases (CMT3/DRM). It is unclear whether histone modification precedes DNA methylation or not. The process might also involve siRNA-directed chromatin remodeling factors such as DRD1. The positions of PolIVa and PolIVb in those reactions are currently ill defined. In the *S*. *pombe* heterochromatic RNAi pathway resulting in H3K9 (but not cytosine) methylation, target transcription by PolII is required for siRNA action, and Ago I associates with nascent transcripts¹⁰⁰. siRNA Directed histone methylation of the human EF1A promoter was also dependent on active PolII transcription¹⁰¹. However, direct siRNA-DNA base-pairing cannot be excluded. For instance, in experiments involving virus derived promoter directed siRNAs, the methylated DNA interval on targeted promoters matched the primary siRNA source and did not extend any further into transcribed regions⁹⁹. If siRNAs indeed interact directly with DNA, how does the double helix become available for siRNA pairing? PolIV could facilitate this access, for instance by moving along the DNA with associated helicases. The precise molecular mechanisms underlying sequence-specific recruitment of cytosine and histone methyl-transferases to silent loci also remains elusive, as associations between sRNA and such enzymes have been reported in only one single case, in human cells¹⁰¹. In fact, a self-sustaining loop in which siRNA production and DNA/histone methylation are mutually dependent appears to exist at endogenous silent loci, raising the possibility that production of chromatin-directed siRNAs *in vivo* might even be a consequence, rather than a cause, of DNA/histone methylation.

The RDR2/DCL3/NRPD1/AGO4 pathway has clear roles in transposon taming and maintenance of genome integrity in plants, because loss of casiRNA caused by mutations in the above factors reactivates transposon activity^{11, 91}. This pathway may also maintain heterochromatin at centromeric repeats, which appears mandatory for accurate chromosome segregation in *S. pombe*¹⁰²*.* The 24nt siRNA-generating machinery may also act to silence protein-coding genes. For example, expression of the key negative regulator of flowering *FLC* is maintained at a low level in an early-flowering *Arabidopsis* ecotype due the presence of an intronic transposon that causes repressive chromatin modifications through the action of an NRPD1a/AGO4-dependent pathway¹⁰³. Nevertheless, several additional mechanisms, not necessarily mediated by siRNAs, account for epigenetic regulation of gene expression in plants. For example, in *Arabidopsis,* mutation of the chromatin-remodeling factor DDM1 has much broader consequences on chromatin silencing than any known single mutant in the RNA silencing machinery^{104,105}. In addition, gene regulation by polycomb-like proteins in *Arabidopsis* has not been linked to RNA silencing¹⁰⁶.

**Table 1 Overview of proteins with roles in Arabidopsis small RNA pathways.**

| Protein | Domains and motifs | Biochemical activity | Pathway | Ref. |
|---|---|---|---|---|
| DCL1 | RNase III | miRNA synthesis | miRNA | 55,85 |
| | dsRNA bd | | nat-siRNA | |
| | DEAD-box helicase | | | |
| | PAZ | | | |
| | DUF283 (unknown function) | | | |
| HYL1 | dsRNA bd | dsRNA bd | miRNA | 64, 65 |
| HST | RanGTP bd | Putative exportin | miRNA | 58 |
| AGO1 | PAZ | siRNA Slicer | miRNA | 26-29 |
| | Piwi | miRNA Slicer | S-PTGS | |
| | | | tasiRNA | |
| | | | Chromatin (?) | |
| HENI | dsRNA bd | sRNA methyl transferase | All SRNA | 20.25, 56, 70 |
| | Lupus La RNA bd | | pathways | |
| | S-adenosyl bd | | | |
| RDR6 | RdRP-specific GDD | RNA-dependent RNA polymerase | S-PTGS | 16, 17, 32, 33, 76, 79, 85 |
| | | | Transitivity | |
| | | | tasiRNA | |
| | | | nat-siRNA | |
| SGS3 | Coiled-coil | Unknown | S-PTGS | 17, 79, 85 |
| | Putative Zn^{II}-bd | | Transitivity | |
| | | | tasiRNA | |
| | | | nat-siRNA | |
| DCL4 | RNase III | 21nt siRNA synthesis | tasiRNA | 13-15 |
| | dsRNA bd | | IR-PTGS | |
| | Helicase | | S-PTGS? | |
| | PAZ | | | |
| WEX | 3'-5' exonuclease | Putative 3'-5' exonuclease | S-PTGS | 19 |
| SDE3 | DEAD box | Putative RNA helicase | S-PTGS | 21, 33 |
| | Helicase | | Transitivity | |
| DCL2 | RNaseIII | 22/24 nt siRNA synthesis | nat-siRNA | 85 |
| | dsRNA bd | | | |
| | PAZ | | | |
| DCL3 | RNase III | 24nt siRNA synthesis | Chromatin | 28, 91 |
| | DEAD box helicase | | | |
| | PAZ | | | |
| RDR2 | RdRP | Putative RNA dependent | Chromatin | 91 |
| | | RNA polymerase | | |
| AGO4 | PAZ | Unclear | Chromatin | 11 |
| | Piwi | | | |
| NRPD1a | RNA polymerase | Putative DNA dependent | Chromatin | 85, 92-95 |
| | | RNA polymerase | nat-siRNA | |
| NRPD1b | RNA polymerase | Putative DNA dependent | Chromatin | 93, 95 |
| | | RNA polymerase | | |
| NRPD2 | RNA polymerase | Putative DNA dependent | Chromatin | 92-95 |
| | | RNA polymerase | | |
| HDA6 | Histone deacetylase | Putative histone deacetylase | Chromatin | 97 |
| DRD1 | SNF2-related DNA and ATP bd Helicase | Putative chromatin remodeling | Chromatin | 96 |
| CMT3 | Cytosine DNA methyl transf. | Cytosine DNA methyl transferase | Chromatin | 88 |
| | Chromodomain | | | |
| | Bromo-adjacent domain | | | |
| DRM1/2 | Cytosine DNA methyl transf. | Cytosine DNA methyl transferase | Chromatin | 88 |
| MET1 | Cytosine DNA methyl transf. | Cytosine DNA methyl transferase | +Chromatin | ⁸⁸ |
| | Bromo-adjacent domain | | | |
| KYP | SET domain | H3K9 methyl transferase | Chromatin | ⁹⁰ |
| | Zn^{II}-bd pre-SET domain | | | |
| | Post-SET domain | | | |
| | YDG domain | | | |
| | EF-hand | | | |
| SUVH2 | SET domain | H3K9 methyl transferase | Chromatin | ¹²⁸ |
| | Zn^{II}-bd pre-SET domain | | | |
| | YDG domain | | | |

### Antiviral Dicer activities in plants

There is extensive evidence that the plant RNAi pathway plays essential roles in antiviral defense {Voinnet, 2005 #5046}. Double-stranded RNA derived from viral genomes is diced into siRNAs by the redundant activities of both DCL4 (the major antiviral Dicer) and DCL2 (a surrogate of DCL4) {Deleris, 2006 #5858}. These siRNAs then incorporate into an RISC to mediate slicing of viral transcripts and thereby reduce the overall viral load into plant cells {Deleris, 2006 #5858}. AGO1 is the likely effector protein of the siRNA loaded RISC, although other AGO paralogs might be also involved {Zhang, 2006 #5861}. A cell-to-cell and long distance signal for RNA silencing also accounts for the systemic spread of the antiviral innate immune response throughout plants {Voinnet, 2005 #5046}. As a counter-defensive strategy, viruses encode suppressor proteins that are targeted against key processor and effector of antiviral silencing. For instance, the P19 protein of tombusviruses sequesters siRNAs and prevents their use by RISC {Vargason, 2003 #4872}, the 2b protein of *Cucumber mosaic virus* physically interacts with AGO1 and inhibits its cleavage activity {Zhang, 2006 #5861}, and the P38 protein of *Turnip crinckle virus* strongly inhibits DCL4 activity{Deleris, 2006 #5858}. DCL3 (producing heterochromatic siRNAs) and DCL1 (producing miRNAs) do not appear to have a significant impact on plant virus accumulation.

### Disease resistance in plants

Apart from antiviral defense, there is currently scant information available on the role of small RNA pathways in defense against other types of pathogens including bacteria and fungi, which account for major yield losses worldwide. In plants, fungal and bacterial resistance has been most thoroughly studied in the context of race-specific interactions, in which a specific resistance (R) protein protects the plant against a particular pathogen's race {Dang1, 2001 #4961}. This highly specific recognition leads to activation of defense responses and local cell death referred to as 'hypersensitive response' (HR). A well-characterized example of HR elicitation through race-specific interaction is provided by the *Arabidopsis RPS2* gene that confers resistance to *Pseudomonas syrinage* pv. *tomaro* strain DC3000 (*Pst* DC3000) producing the corresponding *AvrRpt2* elicitor protein (REF1). The presence of both RPS2 and AvrRpt2 components leads to resistance, whereas the absence of either component leads to disease {Dang1, 2001 #4961}.

Beside the race-specific interaction is a basal defense mechanism referred to as "non-host resistance", which accounts for the fact that most plants are resistant to most pathogens. Basal defense relies on both constitutive and inducible responses. The inducible basal defense occurs through the perception of general elicitors known as 'pathogen-associated molecular patterns' (PAMPs). One such PAMP is a conserved 22 amino acid motif (flg-22) of the bacterial flagellin, which is recognized in several plant species, including *A. thaliana* (REF2). Perception of flg-22 in *Arabidopsis* triggers an immune response which elevates resistance to the virulent Pto DC3000 (REF3). This basal resistance is thought to rely on the induction of a set of 'defense-related genes', some of which are up-regulated within minutes of elicitation and therefore might play a preponderant role in PAMP-triggered immunity (REF4). Nonetheless, the molecular basis orchestrating the transcriptional activation of such defense-related genes remains largely unknown.

### CasiRNAs, transposon taming and epigenetic regulation of gene expression

Large-scale small RNA cloning and sequencing carried out in *Arabidopsis,* rice and maize indicates that the vast majority of those molecules is 24nt in size and, therefore, likely derives from the activity of DCL3. Genomic mapping of these abundant small RNA species shows that many originate from centromeric repeats as well as transposon and retrotransposon loci that are scattered along the chromosomes. Based on circumstantial evidence, these transposon-derived siRNAs appear to act in *cis* to repress their transcription by promoting sequence-specific DNA methylation and chromatin condensation. Accordingly, those molecules have been named *cis-*acting (ca)siRNAs. A popular assumption is that casiRNAs are important for taming the expression and mobilization of transposable elements TEs, thereby preventing genome instability due to random insertions. Nonetheless, *dcl3* mutant plants do not show any sign of obvious developmental defects and set seeds normally. Another idea comes from the proposal, by Barbara McClintock, that the epigenetic state of TEs might influence the expression of genes located in their vicinity. According to this idea, casiRNA-repressed TEs might dampen expression of neighboring genes and, conversely, transcriptionally de-repressed TEs (*e.g*., in the *dcl3* mutant background) might promote gene expression.

Given the density and diversity of TEs in plants, and the potential flexibility of epigenetic regulations in guiding the adaptation of organisms to their direct environment, we tested if the casiRNAs pathway could be involved in plant defense responses to biotic stress, in particular to bacterial and fungal infections.

Approaches to knock-out or knock-down both *DCL2* and *DCL3* genes in various plant species, including crops are used to enhanced pathogen resistance without altering plant development and seed yields. These approaches include, but are not restricted to, Targeted Induced Local Lesions in Genomes (TILLING) of the *DCL2* and *DCL3* genes from non-transgenic plant species (*DCL2* and *DCL3* are conserved across most plant species including crops), RNAi of both DCL2 and DCL3 mRNAs using a hairpin construct that carries a portion of 150bp of *DCL2* gene and a portion of 150bp of *DCL3* gene to allow combinatorial silencing of both DCL2 and DCL3 mRNAs, the generation of an artificial microRNA that target both DCL2 and DCL3 transcripts. These approaches hereby disclosed are known by those skilled in the art and are used to specifically knock-out or knock-down the expression of both *DCL2* and *DCL3* in various plants, including crops, and to obtain crops that are significantly more resistant to both fungal and bacterial pathogens. Such plants can then be transformed with constructs carrying either the strong 35S promoter or a pathogen-inducible promoter (*e.g*., WRKY6, PR1) fused to the *DCL4* coding sequence to allow, additionally, enhanced resistance to viral pathogens (see introduction).

The European Patent Application EP 0480236 and International Application WO 02/20791 disclose a method to confer resistance to pathogen attack in a plant comprising modifying said plant to contain as transgene a gene which is upregulated when resistance to a pathogen is elicited.

Zhu et al. (Curr. Biol., 17: 54-59, 2007; Curr. Biol., 17: 51-58, 2007) disclose that plant defence related genes such as "extensin", "plant defensine gene PDF1a, 2a", "beta glucosidase" have a reduced expression in plant mutant for ROS1.

Agius et al. (PNAS USA., 103: 11796-11801, 2006) disclose production of ROS1 under a constitutive promoter and that its overexpression leads to a reduced cytosine methylation level and an increased expression of the target gene.

Chan et al. (Nature, 6: 351-360, 2006) and Stokes et al. (Genes Dev., 16: 171-182, 2002) disclose that a gene cluster involved in plant resistance (BAL) is silenced by DNA methylation.

### DISCLOSURE OF THE INVENTION

The invention relates in general to genes, pathways, and silencing mechanisms that modulate the response of plants, including crop plants, to infection by pathogens. Methods for identifying compounds or endogenous factors that repress or enhance an undesired or desired pathway or activity respectively comprise providing an expression system wherein the control sequences associated with the gene which generates a desired or undesired response is operatively linked to a reporter whose production is detectable. The influence of compounds on the expression mediated by these control sequences as determined by the level of reporter produced can be used to identify compounds that modulate such activities or pathways. In addition, endogenous repressors or enhancers can be assessed by mutagenizing organisms that contain the foregoing expression systems and analyzing the genome for differences in those organisms where the desired affect has been achieved.

In addition, genes the expression of which is desired because enhancement of resistance is desirable may be supplied in constructs containing constitutive or pathogen responsive control sequences and introduced into plants to effect better resistance. Alternatively, sequences that are designed to interfere with the expression of genes that deplete resistance to pathogen infection may be similarly placed under control of such promoters and introduced into plants so as to inhibit the activities which interfere with pathogen resistance.

As shown herein, plants lacking both Dicer-like enzymes (DCL) DCL2 and DCL3 are more resistant to fungal and bacterial pathogens, and both DCL2 and DCL3 mRNAs are down-regulated in response to Pto DC3000 and flg-22, a flagellin protein that elicits resistance based on pathogen associated molecular patterns (PAMP). Also plants lacking components involved in cytosine DNA-methylation, *i.e*., the RNA directed DNA methylation (RdDM pathway) are more resistant to pathogens, whereas plants lacking the Repressor of transcriptional gene silencing-1 (ROS1), which encodes a DNA-glycosylase involved in active DNA-demethylation, are more susceptible to the same pathogens. Key defense related genes are negatively regulated by casiRNAs, which trigger RNA-directed DNA methylation. These results provide important new insight into epigenetic regulation of activators of the PAMP-triggered immune response.

Method for inhibiting expression of both DCL2 and DCL3 in various plant species including crops, by introducing into a plant a nucleic acid construct comprising a constitutive or pathogen responsive promoter operatively linked to a hairpin directed against both DCL2 and DCL3 or to an artificial miRNA precursor carrying a mature miRNA directed against both DCL2 and DCL3, is disclosed. This also comprises targeted induced local lesions in genes (TILLING) of DCL2 and DCL3 genes that are conserved across plant species.

Methods for identifying repressors of DCL2 and DCL3 transcription by introducing into a plant a nucleic acid construct comprising either DCL2 or DCL3 promoter sequences fused to a reporter gene (*e.g*., a fluorescent protein, *e.g*., Green Fluorescence Protein : GFP or other indicator including mRNA). Plants that express GFP are mutagenized and those with decreased reporter expression are examined for genetic differences to identify upregulated genes, are disclosed also.

Alternatively, plants or cells that constituitively produce a reporter such as GFP wherein the expression is downregulated by DCL2 or DCL3 will have enhanced levels of GFP when the plant or cell is mutagenized to produce repressors of DCL2 or DCL3.

As used herein, "reporter" refers to any sequence whose expression can be monitored. Convenient monitors of expression are fluorescent proteins of many colors, and green fluorescent protein is most commonly used. Other indicators include various enzyme activities or even characteristic mRNA.

In another aspect, resistance is conferred when the identified genes are further fused to a constitutive promoters or pathogen-inducible promoters to repress DCL2 and DCL3 expression in various plant species including crops. Chemical compounds involved in repressing DCL2 and DCL3 transcription can be identified by screening for chemical components that inhibit expression of the reporter of the above transgenic plants that report DCL2 and DCL3 transcriptional activity and these compounds can be used to confer resistance to bacterial or fungal infection.

Methods for identifying positive regulators of DCL4 transcription follow similar approaches are disclosed. These regulators enhance resistance to virulent viruses.

Compositions and methods to isolate genes involved in plant and animal innate immunity and that are regulated by casiRNAs contained in their promoter, coding or 3'UTR regions, are disclosed. This method employs microarray analysis coupled with bioinformatic analysis to retrieve remnant transposons located in the vicinity of, or within, positive regulators of the plant and animal defense response.

Enhanced pathogen resistance may also be achieved by introducing into a plant a nucleic acid construct comprising a constitutive promoter operatively linked to the coding sequence of genes that are hyper-induced in PAMP-elicited *dcl2-dcl3* double mutant and a list of such candidates is provided herein.

Precursors of miRNA or siRNA that are involved in plant or animal innate immunity that are regulated by casiRNA-directed DNA-methylation, are determined by a method using microarray analysis coupled with bioinformatic analysis to retrieve remnant transposons located within the upstream regions of PAMP-responsive miRNA or siRNA precursors that are likely involved in pathogen resistance. Plants are provided enhanced pathogen resistance by introducing into a plant a nucleic acid construct comprising a constitutive promoter, or pathogen-responsive promoter, operatively linked to the identified PAMP-responsive pre-miRNA or pre-siRNA sequences. The sequences of such PAMP-responsive pre-miRNA/siRNA are provided herein.

The invention is directed to a method for modulating expression of the ROS1 DNA-demethylase in various plant species including crops, comprise introducing into a plant a nucleic acid construct that comprises a constitutive or pathogen responsive promoter operatively linked to the coding sequence of the *A rabidopsis* DNA-demethylase ROS1.

Methods for identifying repressors of DNA-methyltransferase transcription by introducing into a plant a nucleic acid construct comprising either DRM1, DRM2, CMT3 or MET1 promoter sequences fused to a reporter gene (*e.g*., Green Fluorescence Protein : GFP) are disclosed. The resulting plants are mutagenized to retrieve plants that have diminished expression of reporter, and analyzing the genome to identify modified genes. The identified genes are further fused to a constitutive promoter or pathogen-inducible promoter to repress constitutively or conditionally DNA-methyltransferase expression in various plant species including crops. Similarly, chemical compounds involved in repressing transcription of DNA-methyltransferase genes may be identified by screening for chemical components that inhibit reporter expression of the transgenic plants described above. A similar approach is used to identify positive regulators of ROS1 transcription that are further overexpress, conditionally or constitutively, *in planta* to confer enhanced resistance to bacterial and fungal pathogens in various plant species including crops, and to identify chemical compounds that enhance ROS1 transcription, which are also used to confer resistance to unrelated pathogens.

Mechanisms of gene regulation similar to those described for plants herein occur in animals including humans. Using the methods disclosed herein genes that are induced by lipopolysaccharide (LPS), flagellin or other PAMPs, are analyzed for the presence of remnant transposons within their promoter, coding or 3' UTR regions. Similar analyses are performed in promoters from PAMP-induced miRNAs (*e.g*., miR146). These protein-coding and non-coding genes contribute to the mammalian innate immune response and can be constitutively expressed in mammalian cells to confer broad spectrum resistance to pathogens.

### BRIEF DESCRIPTION OF THE DRAWINGS

Figures 4A-4E present results demonstrating that DCL2 and DCL3 act as negative regulators of the antifungal and antibacterial defense response.
Figures 5A-5B show results demonstrating that DCL2 and DCL3, but not DCL4 transcripts are down-regulated in response to flg-22 or a Pto DC3000.

### MODES OF CARRYING OUT THE INVENTION

As described in the examples below, it has been found that resistance to fungal and bacterial pathogens in plants is enhanced in the absence of expression of DCL2 and DCL3, although enhancing expression of DCL4 enhances resistance to viral pathogens. Additional genes whose expression is helpful in providing resistance to pathogens are described below. These genes are upregulated in response to producers of PAMP, such as flg-22. Cis acting siRNA sequences (casiRNAs) have been located by virtue of their proximity to songs and have been found to repress PAMP responses by effecting methylation of some pre-miRNA/p-siRNA promoter DNA sequences which would otherwise generate miRNA or siRNA to combat the pathogen. Thus, the expression of the pmi-RNA/siRNA sequences described can be provided in expression systems to plants to confer resistance. In addition, it has been found desirable to deplete DNA methyltransferases that regulate the response, and these include MET1, DRM1, DRM2, CMT3 and DDM1.

Finally, using the method of the invention, it has been shown that enhanced expression of the DNA-demethylase ROS1 enhances plant resistance two pathogens or other stimuli.

Various embodiments disclosed herein include:
1. A method for repressing the casiRNA pathway in plants which comprises introduction into a plant of a nucleic acid construct comprising a constitutive or pathogen responsive promoter operatively linked to a hairpin directed against both DCL2 and DCL3 mRNAs or an artificial miRNA precursor carrying a mature miRNA directed against both DCL2 and DCL3 mRNAs. This also comprises, but is not restricted to, TILLING of DCL2 and DCL3 genes.
   The foregoing method is completed by an approach that allows the constitutive or conditional overexpression of the viral-derived siRNA pathway in the said plants that do not, or less, express DCL2 and DCL3 genes. This comprises introduction into a plant of a nucleic acid construct comprising a constitutive or pathogen responsive promoter operatively linked to the *Arabidopsis* DCL4 coding sequence to confer resistance to viruses. This is applied in various plant species including crops where the *Arabidopsis* DCL4 protein should be functional.
   In these methods, adverse effects on plant development and physiology are avoided. These methods can be applied to various plant species including crops where the DCL2, DCL3 and DCL4 orthologs are also present.
2. A method for identifying repressors of DCL2 and DCL3 transcription as well as positive regulators of DCL4 transcription. A genetic approach involving transgenic lines which report DCL2, DCL3 or DCL4 transcriptional activities which are mutagenized to identify mutants that (i) constitutively express lower DCL2 or DCL3 transcription and (ii) enhance DCL4 transcription. This allows the identification of repressors of both DCL2 and DCL3 transcription as well as activators of DCL4 transcription.
   The method allows the identification of repressors of DCL2 and DCL3 transcription as well as activators of DCL4 transcription that are likely conserved across plants species and therefore can be constitutively or conditionally overexpressed in various plants species including crops to confer enhance resistance to unrelated pathogens. This comprises introduction into a plant of a nucleic acid construct comprising a constitutive or pathogen-responsive promoter operatively linked to the *Arabidopsis* DNA sequence coding for the DCL2 or DCL3 transcriptional repressors or DCL4 transcriptional activators in various plant species including crops.
   The method further allows constitutive or conditional expression of the viral-derived siRNA pathway to confer resistance to viruses, by introduction into a plant of a nucleic acid construct comprising a constitutive or pathogen responsive promoter operatively linked to the *Arabidopsis* DCL4 coding sequence to confer resistance to viruses. This is applied in various plant species including crops where the *Arabidopsis* DCL4 protein should be functional.
3. A method to identify chemical compounds that efficiently repress DCL2 and DCL3 transcription to allow antibacterial and antifungal resistance to pathogens in various plant species. This is achieved by using the said transgenic lines described above and screening for a library of compounds. A similar approach is used to identify chemical agents that enhance DCL4 transcription and will additionally confer antiviral resistance.
4. A method for identifying genes (including protein-coding genes and miRNA/siRNA genes) involved in plant and animal innate immunity, using microarray technology coupled to a bioinformatic analysis in order to retrieve remnant transposons within plant and animal genomes that are located in promoter, coding and 3' UTR regions from the said defense-related genes (including protein-coding genes as well as miRNA/siRNA genes).
   This method allows constitutive or conditional overexpression of key defense-related genes (protein-coding genes) that are likely regulated by transcriptional gene silencing, by introducing a nucleic acid construct comprising a constitutive or pathogen responsive promoter operatively linked to *Arabidopsis* coding sequences corresponding to genes that are hyper-induced in *dcl2-dcl3*-elicited mutant.
   This method allows constitutive or conditional overexpression of key PAMP-responsive miRNA- or siRNA-precursors that are regulated by transcriptional gene silencing, by introducing into a plant of a nucleic acid construct comprising a constitutive or pathogen responsive promoter operatively linked to the PAMP-induced miRNA or siRNA precursor sequences (40nt upstream and downstream of the miRNA or siRNA stem loops).
5. A method for repressing the RdDM pathway in plants which comprises introduction into a plant of a nucleic acid construct comprising a constitutive or pathogen responsive promoter operatively linked to a hairpin directed against all DRM1, DRM2, CMT3 and MET1 or an artificial miRNA precursor carrying a mature miRNA directed against all these transcripts. This also comprises, but is not restricted to, TILLING of *MET1* and *DDM1* genes in various plant species including crops. Methods for repressing DNA-methyltransferase transcription are provided, by introduction into a plant of a construct carrying the control sequences from DNA-methyltransferase genes operatively linked to reporter sequences and mutagenesis of the said transgenic lines to identify transcriptional repressors of such DNA-methyltransferases. These repressors are further overexpressed, conditionally or constitutively, in various plants species including crops to confer enhanced resistance to pathogens. Chemical agents that repress the transcription of DNA-methyltransferases to confer enhanced resistance to pathogens can be thus identified. This is achieved by using the same transgenic lines that report transcriptional activities of DNA-methyltransferases.

The method can also be supplemented by the constitutive or conditional overexpression of the viral-derived siRNA pathway in the above plants that do not, or less, express DNA-methyltransferase genes.

Finally, the method of the invention is directed to a method for constitutively or conditionally overexpressing the *Arabidopsis* DNA-glycosylase ROS1 in various plant species including crops. This comprises introduction into a plant of a nucleic acid construct comprising a constitutive or pathogen-responsive promoter operatively linked to the *Arabidopsis* ROS1 coding sequence to confer broad spectrum resistance to pathogens.

This method is completed by the constitutive or conditional overexpression of the viral-derived siRNA pathway in the above plants that, constitutively or conditionally, overexpress the said *Arabidopsis* ROS1 gene using DCL4 as above.

The methods described for identification of transcriptional activators set forth above may also be applied to ROS1.

The following examples are offered to illustrate but not to limit the invention.

### EXAMPLES

All the results below were generated in the model species *Arabidopsis thaliana*, as illustrative of plants in general including crops. While the specifics of the examples that follow are provided to fully enable those skilled in the art to understand and practice this invention, to provide the best mode for practicing this invention, and to supply a thorough written description of the invention, the invention should not be construed as being limited to the specifics as outlined in these examples.

### Example 1

### The dcl2-dcl3 mutant displayed enhanced disease resistance to bacterial and fungal biotrophic pathogens through potentiation of the SA-dependent defense pathway

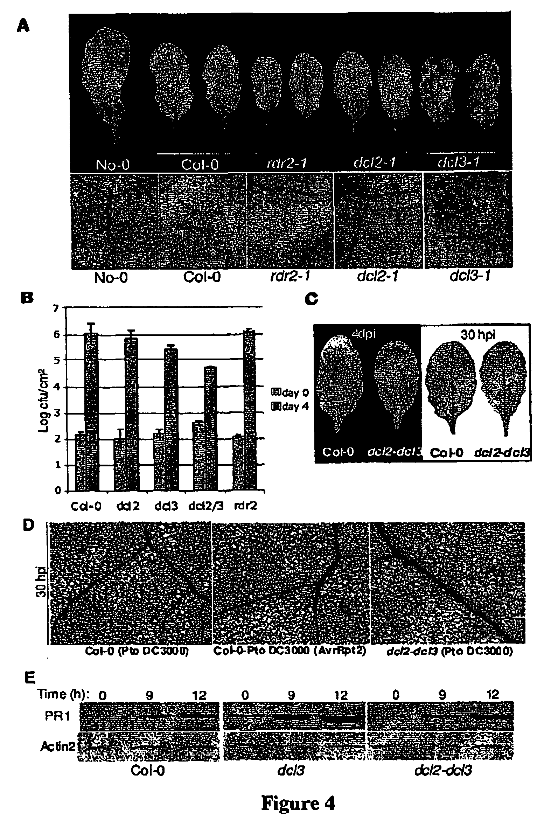

We challenged *rdr2-1, dcl2* and *dcl3* casiRNA-deficient single mutants with the powdery mildew *Erysiphe cichoracearum* (isolate UEA). The *dcl3-1* mutant, but not the *rdr2-1* nor *dcl2-1* mutants, was partially more resistant to this fungus as compared to the Col-0-infected control (Figure 4A, upper panel). Figure 4A shows pathtests carried out with *Arabidopsis* mutants deficient in casiRNA biogenesis. Leaves from five week-old plants (Col-0: *dc12-1, dcl3-1, rdr2-1,* No-0) were inoculated with the powdery mildew *Erysiphe cichoracearum* (isolate UEA) and fungal growth was assessed visually 10 days post-inculcation (upper panel). Trypan blue staining of the above infected leaves (4 days post infection) reveals the presence of micro-HR in No-0 (carrying the functional RPW8 resistance gene), *dcl3-1* and *dcl2-1*.

This enhanced disease resistance phenotype was correlated with the appearance of micro lesions (so-called microHRs) as observed by trypan blue staining (a classical approach used to visualize cell death as well as fungal structures) of the *dcl3*-infected leaves (Figure 4A, bottom panel). Similar microHRs were observed on the *Arabidopsis* accession Nossen that carries a functional *RPW8* resistance gene involved in the recognition of this fungus (Figure 4A, bottom panel). We also observed microHRs in the *dcl2*-infected leaves, however no significant enhanced disease resistance was obtained in this mutant background as compared to Col-0-infected control (Figure 4A, bottom panel).

These results indicate that (i) DCL3 negatively regulates the *Arabidopsis* resistance to *E. cichoracearum* and that (ii) both DCL2 and DCL3 repress the hypersensitive response triggered by this fungus.

To test whether general disease resistance pathways rather than specific pathogen compatibility factors are affected by the *dcl2* and *dcl3* mutations, we further analyzed the resistance of such mutants to the virulent bacterium Pto DC3000. We found that the *dcl2-dcl3* double mutant plants had ~15 fold less bacterial titer and attenuated bacterial disease symptoms as compared to wildtype infected plants (Figure 4B, C). Figure 4B shows bacterial growth on *Arabidopsis* mutants deficient in casiRNA biogenesis. Leaves from five-week old plants (Col-0: *dcl2-1, dcl3-1, rdr2-1*) were inoculated with 10⁵ cfu/ml and bacterial titers assessed four days post-inoculation. Figure 4C shows the *dcl2-dcl3* double mutant displays attenuated disease symptoms (left panel) as well as the presence of microHRs (right panel).

Moreover, trypan blue staining of *dcl2-dcl3-infected* leaves revealed the presence of microHRs at 30 hour post inoculation (hpi) that were absent in Col-0-infected leaves (Figure 4C/D). Figure 4D shows trypan blue staining of the leaves from *dcl2-dcl3* double mutants shows the presence of microHRs.

These nucroHRs were also present in wildtype leaves treated for 30 hours with a low bacterial inoculum of the avirulent Pto DC3000 (AvrRpt2) strain (Figure 4D), which is known to trigger a RPS2-dependent race-specific resistance in *Arabidopsis* Col-0 accession.

These results indicate that both DCL2 and DCL3 act as negative regulators of plant resistance against biotrophic fungal and bacterial pathogens.

Salicylic acid (SA) is the major signaling molecule implicated in plant resistance to biotrophic pathogens. Based on the above results, we investigated whether DCL2 and DCL3 proteins could interfere with the SA signaling pathway during Pto DC3000 infection. We monitored the expression of the *PR1* SA-dependent marker gene in both the *dcl3* and *dcl2-dcl3* plants challenged with high inoculum of the virulent Pto DC3000 over a timecourse experiment, and found that the PR1 transcript displayed an earlier induction in both the *dcl3* and *dcl2-dcl3* infected plants *versus* Col-0 infected plants (Figure 4E). Figure 4E shows PR1 expression is induced earlier in both *dcl3-1* and *dcl2-dcl3*-bacterially infected plants. Leaves from four-week old plants (Col-0: *dc13-1. dcl2-dcl3*) were inoculated with 2x10⁷ cfu/ml and PR1 accumulation was assayed by semi-quantitative RT-PCR over a 9 hour timecourse.

However, sinular PR1 mRNA levels were observed in non-treated *dcl3, dcl2-dcl3* and Col-0 plants (Figure 4E, time 0), which is consistent with a normal developmental phenotype of both *dcl3* and *dcl2-dcl3* mutants in the absence of pathogen challenge (as opposed to *mpk4* or *cpr* mutants that display a severe dwarfism as a result of a constitutive activation of the SA-dependent defense pathway).

Thus, the enhanced disease resistance observed in both *dcl3* and *dcl2-dcl3* mutants is likely due to a potentiation, but not constitutive activation, of the SA-dependent defense pathway during pathogen infection.

Coding as well as protein sequences from DCL2, DCL3 and DCL4 are as follows, which permit generating RNAi constructs, artificial miRNA constructs, DCL4 overexpressor constructs and retrieving DCL orthologs in other plant species in order to use similar knock-down strategies in various plant species including crops.

### -Arabidopsis DCL2 (At3 g03300) coding sequence is:

ATGACCATGGATGCTGATGCGATGGAAACTGAGACCACTGATCAAGTCTCTGCTTCTCCTCTACATTTTGC
CAGAAGTTATCAGGTAGAGGCACTTGAGAAAGCTATCAAGCAGAACACTATTGTCTTCTTGGAGACTGGTT
CTGGCAAGACCCTTATTGCTATTATGCTTCTTCGTAGCTATGCCTACCTTTTCCGCAAGCCTTCACCATGC
TTCTGTGTCTTCTTGGTTCCTCAAGTGGTTCTTGTCACTCAGCAAGCAGAAGCCCTGAAGATGCATACTGA
TCTAAAAGTTGGTATGTATTGGGGAGACATGGGGGTGGACTTTTGGGATTCTTCAACATGGAAACAAGAAG
TTGATAAATATGAGGTTCTGGTGATGACCCCTGCCATTTTGCTCGACGCGTTGAGGCATAGTTTTCTGAGC
TTGAGCATGATCAAGGTTCTAATAGTTGATGAGTGTCATCATGCAGGGGGAAAGCACCCTTATGCTTGTAT
CATGAGGGAGTTCTATCATAAGGAGTTAAATTCTGGAACTTCCAATGTTCCACGGATATTTGGGATGACTG
CTTCACTTGTGAAAACAAAGGGTGAAAATCTGGATAGCTACTGGAAAAAAATTCATGAACTCGAAACTCTA
ATGAATTCAAAGGTCTATACCTGTGAGAATGAGTCTGTGCTGGCTGGGTTTGTCCCCTTTTCTACACCAAG
CTTCAAGTATTACCAGCACATAAAAATACCAAGTCCCAAACGAGCAAGCTTGGTAGAGAAGCTAGAAAGAC
TAACGATAAAGCATCGCTTATCCCTTGGAACCTTGGATCTCAACTCCTCTACTGTTGATTCTGTAGAGAAG

AGACTGTTGAGGATAAGTTCAACTCTAACATATTGTTTGGATGATCTCGGAATTTTGCTGGCCCAGAAGGC
TGCTCAGTCATTGTCAGCCAGTCAGAATGACTCTTTCTTGTGGGGCGAACTAAATATGTTTAGCGTGGCCT
TGGTAAAAAAATTCTGCTCTGATGCTTCACAGGAGTTTTTGGCTGAGATACCTCAAGGTCTTAATTGGAGT
GTTGCAAACATAAATGGAAATGCGGAGGCAGGTCTCCTAACTTTAAAAACTGTCTGCCTCATTGAGACTCT
TCTTGGTTATAGCTCCTTGGAGAACATACGGTGCATCATTTTTGTGGATAGGGTGATAACAGCCATCGTTC
TGGAATCCCTTTTGGCTGAGATTCTTCCAAACTGTAATAACTGGAAAACCAAGTACGTTGCAGGAAATAAC
TCTGGTCTGCAAAATCAAACTCGGAAGAAGCAAAATGAAATTGTGGAAGACTTCCGGAGAGGCTTGGTTAA
CATCATTGTAGCAACATCTATTCTAGAGGAGGGTCTAGATGTTCAAAGTTGCAACCTGGTTATCAGATTTG
ACCCTGCATCCAACATTTGCAGTTTCATACAGTCTCGTGGGCGTGCTAGAATGCAAAATTCAGATTATTTG
ATGATGGTGGAAAGCGGAGATCTGTTAACACAATCTCGATTAATGAAATATCTTTCTGGTGGGAAAAGAAT
GCGCGAAGAGTCTTTGGATCATTCTCTTGTTCCCTGTCCACCTCTTCCAGATGATTCAGATGAACCACTCT
TCCGTGTCGAAAGTACTGGAGCAACTGTAACTCTTAGCTCAAGCGTCAGCTTAATATATCATTACTGCTCA
AGGCTTCCTTCAGATGAGTACTTCAAACCAGCCCCTAGATTTGATGTAAACAAGGATCAGGGGAGTTGCAC
CCTTTACCTTCCTAAGAGTTGCCCAGTAAAAGAAGTTAAAGCTGAAGCAAATAATAAAGTGTTAAAACAAG
CTGTCTGTCTTAAAGCTTGCATTCAACTGCACAAAGTTGGAGCTCTAAGTGATCATCTTGTGCCTGACATG
GTTGTGGCGGAAACTGTCTCACAAAAACTCGAGAAAATCCAATATAACACAGAGCAGCCATGTTACTTCCC
CCCAGAGCTAGTCTCCCAGTTTTCAGCACAGCCGGAGACAACATACCACTTCTACTTAATAAGAATGAAGC
CAAACTCTCCAAGAAATTTTCATTTAAACGATGTTTTACTAGGCACCAGAGTTGTGCTTGAAGATGACATT
GGGAACACAAGCTTCCGGTTGGAAGATCATAGGGGTACAATAGCTGTGACATTGAGTTATGTGGGAGCTTT
TCACCTTACACAAGAAGAGGTCCTTTTCTGTAGAAGATTTCAGATAACTCTATTCCGAGTTCTTTTAGATC
ACAGTGTGGAAAATTTGATGGAGGCATTGAATGGATTGCATCTCAGAGATGGGGTGGCACTTGATTATCTA
CTAGTTCCATCCACTCATTCACATGAAACATCTCTTATTGATTGGGAAGTGATCAGATCCGTGAATCTAAC
TTCTCATGAGGTTTTGGAAAAACACGAAAATTGTTCTACCAACGGTGCTTCTCGCATTCTACACACAAAAG
ACGGCTTGTTTTGTACTTGTGTCGTACAAAATGCATTGGTTTACACACCACATAATGGATACGTCTACTGC
ACAAAAGGTGTTCTCAACAATCTAAACGGAAATTCATTATTGACCAAGAGAAATTCTGGCGATCAGACTTA
CATTGAGTACTACGAGGAAAGGCATGGATTCAATTAAATTTTGTGGATGAACCTCTTCTAAATGGAAGAC
ACATTTTCACGTTGCATAGTTATCTTCACATGGCCAAGAAGAAGAAGGAGAAAGAGCATGACAGGGAATTT
GTTGAACTACCTCCTGAGCTTTGTCATGTCATTTTGTCCCCAATATCAGTTGATATGATCTATTCATATAC
TTTTATCCCATCTGTTATGCAACGCATTGAATCTTTGCTTATAGCATACAACCTGAAGAAAAGCATCCAA
AAGTCAATATTCCAACCATTAAGGTTTTGGAAGCTATTACGACAAAGAAGTGCGAAGATCAGTTCCACTTG
GAATCACTAGAAACTCTTGGTGACTCTTTTCTGAAATATGCTGTTTGTCAGCAACTATTCCAACACTGTCA
TACTCACCATGAGGGTCTTCTTAGCACGAAGAAAGATGGAATGATTTCAAATGTCATGCTCTGCCAATTTG
GATGTCAGCAGAAACTTCAGGGATTTATCCGCGATGAGTGTTTTGAACCCAAAGGTTGGATGGTTCCAGGT
CAATCATCTGCAGCTTATTCACTTGTAAACGATACTCTACCCGAGTCTAGAAACATATACGTTGCTAGTAG
GAGGAATCTGAAACGCAAGAGTGTGGCCGATGTTGTAGAATCATTAATTGGAGCATATCTCAGCGAGGGAG
GTGAACTTGCAGCTTTGATGTTCATGAATTGGGTTGGAATAAAGGTCGACTTTACAACTACGAAGATCCAG
AGAGATTCCCCAATACAAGCAGAGAAGCTTGTGAATGTAGGTTATATGGAGTCGCTGTTGAATTACAGTTT
TGAGGATAAGTCTCTTCTAGTTGAAGCATTGACTCATGGTTCATACATGATGCCTGAAATTCCAAGATGCT
ATCAGCGGTTGGAGTTCCTCGGTGACTCTGTATTGGATTATCTCATAACCAAGCATCTATACGACAAATAT
CCTTGTCTGTCCCCTGGACTATTAACCGACATGCGATCAGCTTCTGTTAACAATGAATGTTATGCCCTAGT
GGCGGTGAAAGCAAACCTGCACAAACACATCCTGTACGCCTCTCATCATCTCCATAAGCACATCTCTAGAA
CTGTCAGTGAGTTTGAACAGTCTTCTTTGCAATCCACTTTCGGATGGGAATCCGATATATCTTTTCCAAAG
GTTCTTGGAGATGTGATAGAATCTCTAGCAGGCGCGATATTTGTTGACTCAGGTTACAACAAGGAAGTAGT
GTTTGCAAGTATTAAACCACTTTTGGGTTGTATGATAACTCCAGAGACTGTCAAGTTGCATCCTGTGAGAG
AGTTGACAGAATTATGTCAGAAGTGGCAGTTCGAGTTGAGTAAAGCTAAAGATTTCGATTCTTTCACGGTT
GAGGTGAAAGCTAAGGAGATGAGTTTTGCTCACACAGCAAAGGCCTCTGATAAGAAAATGGCCAAGAAATT
GGCTTACAAAGAAGTCTTGAACTTACTTAAGAACAGCCTGGACTACTAA

- *Arabidopsis* DCL2 protein sequence is: 
MTMDADAMETETTDQVSASPLHFARSYQVEALEKAIKQNTIVFLETGSGKTLIAIMLLRSYAYLFRKPSPC
FCVFLVPQVVLVTQQAEALKMHTDLKVGMYWGDMGVDFWDSSTWKQEVDKYEVLVMTPAILLDALRHSFLS
LSMIKVLIVDECHHAGGKHPYACIMREFYHKELNSGTSNVPRIFGMTASLVKTKGENLDSYWKKIHELETL
MNSKVYTCENESVLAGFVPFSTPSFKYYQHIKIPSPKRASLVEKLERLTIKHRLSLGTLDLNSSTVDSVEK
RLLRISSTLTYCLDDLGILLAQKAAQSLSASQNDSFLWGELNMFSVALVKKFCSDASQEFLAEIPQGLNWS
VANINGNAEAGLLTLKTVCLIETLLGYSSLENIRCIIFVDRVITAIVLESLLAEILPNCNNWKTKYVAGNN
SGLQNQTRKKQNEIVEDFRRGLVNIIVATSILEEGLDVQSCNLVIRFDPASNICSFIQSRGRARMQNSDYL
MMVESGDLLTQSRLMKYLSGGKRMREESLDHSLVPCPPLPDDSDEPLFRVESTGATVTLSSSVSLIYHYCS
RLPSDEYFKPAPRFDVNKDQGSCTLYLPKSCPVKEVKAEANNKVLKQAVCLKACIQLHKVGALSDHLVPDM
VVAETVSQKLEKIQYNTEQPCYFPPELVSQFSAQPETTYHFYLIRMKPNSPRNFHLNDVLLGTRVVLEDDI
GNTSFRLEDHRGTIAVTLSYVGAFHLTQEEVLFCRRFQITLFRVLLDHSVENLMEALNGLHLRDGVALDYL
LVPSTHSHETSLIDWEVIRSVNLTSHEVLEKHENCSTNGASRILHTKDGLFCTCVVQNALVYTPHNGYVYC
TKGVLNNLNGNSLLTKRNSGDQTYIEYYEERHGIQLNFVDEPLLNGRHIFTLHSYLHMAKKKKEKEHDREF
 
VELPPELCHVILSPISVDMIYSYTFIPSVMQRIESLLIAYNLKKSIPKVNIPTIKVLEAITTKKCEDQFHL
ESLETLGDSFLKYAVCQQLFQHCHTHHEGLLSTKKDGMISNVMLCQFGCQQKLQGFIRDECFEPKGWMVPG
QSSAAYSLVNDTLPESRNIYVASRRNLKRKSVADVVESLIGAYLSEGGELAALMFMNWVGIKVDFTTTKIQ
RDSPIQAEKLVNVGYMESLLNYSFEDKSLLVEALTHGSYMMPEIPRCYQRLEFLGDSVLDYLITKHLYDKY
PCLSPGLLTDMRSASVNNECYALVAVKANLHKHILYASHHLHKHISRTVSEFEQSSLQSTFGWESDISFPK
VLGDVIESLAGAIFVDSGYNKEVVFASIKPLLGCMITPETVKLHPVRELTELCQKWQFELSKAKDFDSFTV
EVKAKEMSFAHTAKASDKKMAKKLAYKEVLNLLKNSLDY
- *Arabidopsis* DCL3 (At3g43920) coding sequence is: 
ATGCATTCGTCGTTGGAGCCGGAGAAAATGGAGGAAGGTGGGGAAGCAATTCGCTTAAGAGAAAATTCTC
TGAAATCGATGGAGATCAAAATCTTGATTCTGTCTCTTCTCCTATGATGACTGACTCTAATGGTAGTTATG
AATTGAAAGTGTACGAGGTTGCTAAGAACAGGAACATAATTGCTGTTTTGGGGACAGGGATTGATAAGTCA
GAGATCACTAAGAGGCTTATCAAAGCTATGGGTTCTTCTGATACAGACAAAAGATTGATAATTTTCTTGGC
CCCAACTGTGAATCTTCAATGCTGTGAGATCAGAGCACTTGTGAATTTGAAAGTTGAAGAGTACTTTGGAG
CTAAAGGAGTTGATAAATGGACATCTCAGCGCTGGGATGAGGAATTTAGCAAGCACGATGTTTTAGTTATG
ACTCCTCAAATATTATTGGATGTCCTTAGAAGTGCATTCCTGAAACTAGAGATGGTATGTCTTCTAATAAT
AGATGAATGCCACCATACCACTGGCAATCATCCCTATGCGAAGTTAATGAAGATTTTTAATCCTGAAGAGC
GTGAAGGAGTGGAAAAGTTTGCTACAACGGTTAAAGAAGGTCCAATATTGTATAACCCATCACCATCCTGT
AGTTTGGAATTGAAAGAAAAGTTAGAAACTTCACACCTCAAGTTTGATGCTTCTCTTAGAAGGCTTCAAGA
GTTGGGAAAAGACAGTTTTCTGAATATGGATAATAAGTTTGAGACATATCAAAAGAGATTGTCTATCGACT
ACAGAGAGATTTTGCATTGCCTTGATAATCTTGGCCTGATTTGCGCACACTTGGCGGCTGAAGTCTGCTTG
GAGAAAATCTCAGATACGAAAGAGGAAAGTGAAACTTATAAAGAATGCTCAATGGTGTGCAAGGAATTTCT
TGAGGATATTTTATCCACCATTGGGGTGTATTTGCCGCAAGATGATAAGAGTCTGGTAGATTTGCAGCAAA
ACCATCTGTCAGCAGTAATTTCTGGGCATGTATCTCCAAAGCTAAAAGAACTCTTCCATCTATTGGATTCC
TTTAGAGGTGACAAGCAAAAGCAGTGCCTTATTTTAGTTGAGAGAATTATAACTGCGAAAGTGATCGAAAG
ATTCGTTAAGAAAGAAGCCTCTTTGGCTTACCTTAATGTCTTGTATTTAACCGAAAACAACCCCTCCACCA
ATGTATCGGCACAGAAAATGCAAATTGAAATCCCTGATTTATTTCAACATGGCAAGGTGAATCTTTTATTC
ATCACAGATGTGGTTGAAGAGGGATTTCAGGTTCCAGATTGCTCATGCATGGTTTGTTTTGACCTGCCCAA
AACAATGTGTAGTTACTCGCAGTCTCAAAAACATGCCAAACAGAGTAATTCTAAGTCTATCATGTTTCTTG
AAAGAGGGAACCCGAAGCAAAGAGACCATCTGCATGACCTTATGCGAAGAGAAGTCCTAATTCAAGATCCA
GAAGCTCCAAACTTGAAATCGTGTCCACCTCCAGTGAAAAATGGACACGGTGTGAAGGAGATTGGATCCAT
GGTTATCCCAGATTCTAACATAACTGTATCTGAGGAAGCAGCTTCCACACAAACTATGAGTGATCCTCCTA
GCAGAAATGAGCAGTTACCACCGTGTAAAAAGTTACGCTTGGATAACAATCTCTTACAATCCAACGGCAAA
GAGAAGGTTGCCTCTTCTAAAAGTAAATCATCTTCATCGGCTGCAGGTTCAAAAAAACGTAAGGAGTTGCA
CGGAACAACCTGTGCAAACGCATTGTCAGGAACCTGGGGAGAAAATATTGATGGCGCCACCTTTCAGGCTT
ATAAGTTTGACTTCTGTTGTAATATTTCTGGCGAAGTATACTCGAGTTTCTCTCTTTTGCTTGAGTCAACT
CTCGCCGAGGATGTTGGTAAAGTTGAGATGGACCTTTACTTGGTCAGGAAGCTTGTCAAGGCTTCTGTCTC
ACCTTGTGGCCAGATACGTTTGAGTCAAGAGGAGCTGGTCAAAGCAAAATATTTTCAGCAGTTTTTCTTTA
ATGGCATGTTTGGAAAGTTGTTTGTTGGATCTAAGTCACAGGGAACAAAGAGAGAATTTTTGCTTCAAACT
GACACTAGTTCTCTTTGGCACCCTGCCTTTATGTTTCTACTGCTACCAGTTGAAACAAATGATCTAGCTTC
GAGTGCGACAATTGATTGGTCAGCTATCAACTCCTGTGCCTCAATAGTTGAGTTCTTGAAGAAAAATTCTC
TTCTTGATCTTCGGGATAGTGATGGAATCAGTGCAATACCTCATCCGGTCAGGAAGTCTTACTAGACGAT
AAAATGGAAGAAACGAATCTGATTCATTTTGCCAATGCTTCGTCTGATAAAAATAGTCTCGAAGAACTTGT
GGTCATTGCAATTCATACTGGACGGATATACTCTATAGTTGAAGCCGTAAGCGATTCTTCTGCTATGAGCC
CCTTTGAGGTGGATGCCTCATCAGGCTATGCTACTTATGCAGAATATTTTAACAAAAAGTATGGATTGTT
TTAGCGCACCCGAACCAGCCGTTGATGAAGTTGAAGCAGAGTCACCATGCGCACAACCTTTAGTCGACTT
CAATGAAGAGATGGTTGTGAAGACAGAACCAAAAGCTGGCAATGTTAGGAAAAGAAAACCGAATATCCATG
CGCATTTGCCTCCAGAGCTTTTGGCTAGAATTGATGTACCGCGTGCTGTGCTAAAATCAATCTACTTGCTG
CCTTCAGTGATGCACCGCCTAGAGTCTCTAATGTTGGCCAGCCAGCTTAGGGAAGAGATTGATTGTAGCAT
AGATAACTTCAGTATATCAAGTACATCGATTCTTGAAGCAGTTACAACACTTACATGCCCCGAATCATTTT
CAATGGAGCGGTTGGAACTGCTCGGGGATTCAGTCTTGAAGTATGTTGCGAGCTGTCATCTATTCCTTAAG
TATCCTGACAAAGATGAGGGGCAACTATCACGGCAGAGACAATCGATTATATCTAACTCAAATCTTCACCG
CTTGACAACCAGTCGCAAACTACAGGGATACATAAGAAATGGCGCTTTTGAACCGCGTCGCTGGACTGCAC
CTGGTCAATTTTCTCTTTTTCCTGTTCCTTGCAAGTGTGGGATTGATACTAGAGAAGTACCATTGGACCCA
AAATTCTTCACAGAAAACATGACTATCAAAATAGGCAAGTCTTGCGACATGGGTCATAGATGGGTAGTTTC
AAAATCTGTATCAGATTGCGCTGAGGCCCTGATTGGTGCCTATTATGTAAGCGGTGGATTGTCTGCTTCTC
TCCATATGATGAAATGGCTCGGTATTGACGTCGATTTTGACCCAAACCTAGTCGTTGAAGCCATCAATAGA
GTTTCTCTACGGTGTTACATTCCTAAAGAAGATGAGCTCATAGAGTTGGAGAGAAAGATCCAACATGAATT
CTCTGCAAAGTTTCTTTTAAAAGAGGCTATCACACACTCCTCTCTTCGTGAATCCTATTCATACGAGAGAT
TAGAGTTTCTTGGCGATTCTGTACTGGATTTTCTAATAACCCGTCATCTTTTTAACACCTACGAACAAACT
GGGCCTGGAGAGATGACCGATCTTCGTTCTGCATGTGTAAACAATGAAAATTTTGCGCAAGTTGCAGTGAA
AAATAACCTGCATACCCACCTTCAACGCTGTGCTACGGTTCTCGAGACTCAAATAAACGACTATCTGATGT
 
CCTTTCAAAAGCCAGATGAGACTGGTAGATCAATCCCTTCAATACAGGGCCCTAAGGCTCTTGGAGATGTT
GTGGAGAGTATCGCTGGAGCATTGCTGATCGATACGAGGTTAGATCTCGATCAAGTGTGGAGAGTCTTTGA
GCCGTTGCTTTCTCCACTTGTAACTCCAGATAAACTTCAGCTTCCTCCATACCGGAGCTCAATGAGCTAT
GCGACTCTCTTGGGTATTTCTTTCGAGTGAAATGTTCAAATGATGGTGTCAAAGCACAAGCCACGATCCAG
TTGCAGCTGGATGATGTTCTTTTAACTGGAGATGGATCTGAACAGACAAATAAACTGGCCTTGGGAAAAGC
AGCTTCACATCTGCTTACACAACTTGAGAAGAGAAACATTTCACGTAAAACCTCGCTCGGGATAATCAAA
GTTCCATGGATGTCAATCTTGCTTGCAATCATAGCGACAGAGAAACTCTGACTTCAGAGACTACTGAAATC
CAGAGTATAGTGATTCCATTTATTGGACCTATAAACATGAAGAAAGGCGGGCCTCGTGGAACTCTACATGA
GTTTTGCAAGAAGCATCTGTGGCCAATGCCTACTTTCGATACCTCGGAAGAGAAATCCAGAACTCCGTTTG
AATTCATAGATGGCGGTGAGAAGCGGACTAGCTTCAGCAGTTTCACATCGACCATAACCCTAAGGATACCC
AATCGTGAGGCTGTGATGTATGCTGGAGAAGCAAGGCCTGACAAGAAGAGTTCCTTCGACTCTGCAGTCGT
GGAATTGCTTTATGAGCTCGAGCGCCGCAAGATCGTCATAATACAAAAGTAG
- *Arabidopsis* DCL3 protein sequence is: 
MHSSLEPEKMEEGGGSNSLKRKFSEIDGDQNLDSVSSPMMTDSNGSYELKVYEVAKNRNIIAVLGTGIDKS
EITKRLIKAMGSSDTDKRLIIFLAPTVNLQCCEIRALVNLKVEEYFGAKGVDKWTSQRWDEEFSKHDVLVM
TPQILLDVLRSAFLKLEMVCLLIIDECHHTTGNHPYAKLMKIFNPEEREGVEKFATTVKEGPILYNPSPSC
SLELKEKLETSHLKFDASLRRLQELGKDSFLNMDNKFETYQKRLSIDYREILHCLDNLGLICAHLAAEVCL
EKISDTKEESETYKECSMVCKEFLEDILSTIGVYLPQDDKSLVDLQQNHLSAVISGHVSPKLKELFHLLDS
FRGDKQKQCLILVERIITAKVIERFVKKEASLAYLNVLYLTENNPSTNVSAQKMQIEIPDLFQHGKVNLLF
ITDVVEEGFQVPDCSCMVCFDLPKTMCSYSQSQKHAKQSNSKSIMFLERGNPKQRDHLHDLMRREVLIQDP
EAPNLKSCPPPVKNGHGVKEIGSMVIPDSNITVSEEAASTQTMSDPPSRNEQLPPCKKLRLDNNLLQSNGK
EKVASSKSKSSSSAAGSKKRKELHGTTCANALSGTWGENIDGATFQAYKFDFCCNISGEVYSSFSLLLEST
LAEDVGKVEMDLYLVRKLVKASVSPCGQIRLSQEELVKAKYFQQFFFNGMFGKLFVGSKSQGTKREFLLQT
DTSSLWHPAFMFLLLPVETNDLASSATIDWSAINSCASIVEFLKKNSLLDLRDSDGNQCNTSSGQEVLLDD
KMEETNLIHFANASSDKNSLEELVVIAIHTGRIYSIVEAVSDSSAMSPFEVDASSGYATYAEYFNKKYGIV
LAHPNQPLMKLKQSHHAHNLLVDFNEEMVVKTEPKAGNVRKRKPNIHAHLPPELLARIDVPRAVLKSIYLL
PSVMHRLESLMLASQLREEIDCSIDNFSISSTSILEAVTTLTCPESFSMERLELLGDSVLKYVASCHLFLK
YPDKDEGQLSRQRQSIISNSNLHRLTTSRKLQGYIRNGAFEPRRWTAPGQFSLFPVPCKCGIDTREVPLDP
KFFTENMTIKIGKSCDMGHRWVVSKSVSDCAEALIGAYYVSGGLSASLHMMKWLGIDVDFDPNLVVEAINR
VSLRCYIPKEDELIELERKIQHEFSAKFLLKEAITHSSLRESYSYERLEFLGDSVLDFLITRHLFNTYEQT
GPGEMTDLRSACVNNENFAQVAVKNNLHTHLQRCATVLETQINDYLMSFQKPDETGRSIPSIQGPKALGDV
VESIAGALLIDTRLDLDQVWRVFEPLLSPLVTPDKLQLPPYRELNELCDSLGYFFRVKCSNDGVKAQATIQ
LQLDDVLLTGDGSEQTNKLALGKAASHLLTQLEKRNISRKTSLGDNQSSMDVNLACNHSDRETLTSETTEI
QSIVIPFIGPINMKKGGPRGTLHEFCKKHLWPMPTFDTSEEKSRTPFEFIDGGEKRTSFSSFTSTITLRIP
NREAVMYAGEARPDKKSSFDSAVVELLYELERRKIVIIQK
- *Arabidopsis* DCL24 (At5g20320) coding sequence is: 
ATGCGTGACGAAGTTGACTTGAGCTTGACCATTCCCTCGAAGCTTTTGGGGAAGCGAGACAGAGAACAAAA
AAATTGTGAAGAAGAAAAAAACAAAAACAAAAAAGCTAAAAAGCAGCAAAAGGACCCAATTCTTCTTCACA
CTAGTGCTGCCACTCACAAGTTTCTTCCTCCTCCTTTGACCATGCCGTACAGTGAAATCGGCGACGATCTT
CGCTCACTCGACTTTGACCACGCCGATGTTTCTTCCGACCTTCACCTCACTTCTTCTTCCTCTGTTTCTTC
GTTTTCCTCTTCTTCGTCTTCTTTGTTCTCCGCGGCTGGTACGGATGATCCTTCACCGAAAATGGAGAAAG
ACCCTAGAAAAATCGCCAGGAGGTATCAGGTGGAGCTGTGTAAGAAAGCAACGGAGGAGAACGTTATTGTA
TATTTGGGTACAGGTTGTGGGAAGACTCACATTGCAGTGATGCTTATATATGAGCTTGGTCATTTGGTTCT
TAGTCCCAAGAAAAGTGTTTGTATTTTTCTTGCTCCCACCGTGGCTTTGGTCGAACAGCAAGCCAAGGTCA
TAGCGGACTCTGTCAACTTCAAAGTTGCAATACATTGTGGAGGCAAGAGGATTGTGAAGAGCCACTCGGAG
TGGGAGAGAGAGATTGCAGCGAATGAGGTTCTTGTTATGACTCCACAAATACTTCTGCATAACTTACAGCA
CTGTTTCATCAAGATGGAGTGTATCTCCCTTCTAATATTTGATGAGTGTCACCATGCTCAACAACAAAGCA
ACCATCCTTATGCAGAAATCATGAAGGTTTTCTATAAATCGGAAAGTTTACAACGGCCTCGAATATTTGGA
ATGACTGCATCTCCAGTTGTTGGCAAAGGGTCTTTTCAATCAGAGAATTTATCGAAAAGCATTAATAGCCT
TGAAAATTTGCTCAATGCCAAGGTTTATTCAGTGGAAAGCAATGTCCAGCTGGATGGTTTTGTTTCATCTC
CTTTAGTCAAAGTATATTATTATCGGTCAGCTTTAAGTGATGCATCTCAATCGACCATCAGATATGAAAAC
ATGCTGGAGGACATCAAACAGCGGTGCTTGGCATCACTTAAGCTGCTGATTGATACTCATCAAACACAAAC
CCTCCTAAGTATGAAAAGGCTTCTCAAAAGATCTCATGATAATCTCATATATACTCTGCTGAATCTTGGCC
TCTGGGGAGCAATACAGGCTGCTAAAATCCAATTGAATAGTGACCATAATGTACAAGACGAGCCTGTGGGA
AAGAATCCTAAGTCAAAGATATGTGATACATATCTTTCTATGGCTGCTGAGGCCCTCTCTTCTGGTGTTGC
TAAAGATGAGAATGCATCTGACCTCCTCAGCTTAGCGGCGTTGAAGGAACCATTATTCTCTAGAAAGCTAG
TTCAATTGATTAAGATCCTTTCGGTATTCAGGCTAGAGCCACACATGAAATGTATAATATTTGTCAATCGG
ATTGTGACTGCAAGAACATTGTCATGCATACTAAATAACTTGGAACTGCTACGGTCTTGGAAGTCTGATTT
CCTTGTTGGACTTAGTTCTGGACTGAAGAGCATGTCAAGAAGGAGTATGGAAACAATACTTAAACGGTTCC
 
AATCTAAAGAGCTCAATTTACTGGTTGCCACTAAAGTTGGTGAAGAAGGCCTTGATATTCAGACATGCTGT
CTTGTGATCCGTTATGATTTACCAGAGACTGTTACCAGCTTCATACAGTCCAGAGGTCGTGCTCGAATGCC
TCAGTCTGAATATGCGTTTCTAGTGGACAGCGGAAACGAGAAAGAGATGGATCTTATTGAAAATTTTAAAG
TAAATGAAGATCGAATGAATCTAGAAATTACTTACAGAAGCTCAGAGGAAACTTGTCCTAGACTTGATGAG
GAGTTATACAAAGTTCATGAGACAGGAGCTTGTATCAGTGGTGGAAGCAGCATCTCCCTTCTCTATAAATA
TTGTTCTAGGCTTCCACATGATGAATTTTTCAGCCCAAGCCAGAGTTTCAATTCAAGCCTGTTGACGAAT
TTGGTGGAACTATCTGTCGCATAACTTTACCTGCTAATGCTCCTATAAGTGAAATCGAAAGTTCACTACTA
CCTTCGACAGAAGCTGCTAAAAAGGATGCTTGTCTAAAGGCTGTGCATGAGTTGCACAACTTGGGTGTACT
TAACGATTTTCTGTTGCCAGATTCCAAGGATGAAATTGAGGACGAATTGTCAGATGATGAATTTGATTTTG
ATAACATCAAAGGTGAAGGCTGTTCACGAGGTGACCTGTATGAGATGCGTGTACCAGTCTTGTTTAAACAA
AAGTGGGATCCATCTACAAGTTGTGTCAATCTTCATTCTTACTATATAATGTTTGTGCCTCATCCCGCTGA
TAGGATCTACAAAAAGTTTGGTTTCTTCATGAAGTCACCTCTTCCCGTTGAGGCTGAGACTATGGATATCG
ATCTTCACCTTGCTCATCAAAGATCTGTAAGTGTAAAGATTTTTCCATCAGGGGTCACAGAATTCGACAAC
GATGAGATAAGACTAGCTGAGCTTTTCCAGGAGATTGCCCTGAAGGTTCTTTTTGAACGGGGGGAGCTGAT
CCCGGACTTTGTTCCCTTGGAACTGCAAGACTCTTCTAGAACAAGCAAATCCACCTTCTACCTTCTTCTTC
CACTCTGTCTGCATGATGGAGAAAGTGTTATATCTGTAGATTGGGTGACTATCAGAAACTGCTTGTCATCA
CCAATCTTTAAGACTCCATCTGTTTTAGTGGAAGATATATTTCCTCCTTCGGGCTCTCATTTAAAGCTAGC
AAATGGCTGCTGGAATATTGATGATGTGAAGAACAGCTTGGTTTTTACAACCTACAGTAAACAATTTTACT
TTGTTGCTGATATCTGCCATGGAAGAAATGGTTTCAGTCCTGTTAAGGAATCTAGCACCAAAAGCCATGTG
GAGAGCATATATAAGTTGTATGGCGTGGAACTCAAGCATCCTGCACAGCCACTCTTGCGTGTGAAACCACT
TTGTCATGTTCGGAACTTGCTTCACAACCGAATGCAGACGAATTTGGAACCACAAGAACTTGACGAATACT
TCATAGAGATTCCTCCCGAACTTTCTCACTTAAAGATAAAAGGATTATCTAAAGACATCGGAAGCTCGTTA
TCCTTGTTACCATCAATCATGCATCGTATGGAGAATTTACTCGTGGCTATTGAACTGAAACATGTGCTGTC
TGCTTCGATCCCTGAGATAGCTGAAGTTTCTGGTCACAGGGTACTCGAGGCGCTCACAACAGAGAAATGTC
ATGAGCGCCTTTCTCTTGAAAGGCTTGAGGTGCTTGGTGATGCATTCCTCAAGTTTGCTGTTAGCCGACAC
CTTTTTCTACACCATGATAGTCTTGATGAAGGAGAGTTGACTCGGAGACGCTCTAACGTTGTTAACAATTC
CAACTTGTGCAGGCTTGCAATAAAAAAAAATCTGCAGGTCTACATCCGTGATCAAGCATTGGATCCTACTC
AGTTCTTTGCATTTGGCCATCCATGCAGAGTAACCTGTGACGAGGTAGCCAGTAAAGAGGTTCATTCCTTG
AATAGGGATCTTGGGATCTTGGAGTCAAATACTGGTGAAATCAGATGTAGCAAAGGCCATCATTGGTTGTA
CAAGAAAACAATTGCTGATGTGGTTGAGGCTCTTGTGGGAGCTTTCTTAGTTGACAGTGGCTTCAAAGGTG
CTGTGAAATTTCTGAAGTGGATTGGTGTAAATGTTGATTTTGAATCCTTGCAAGTACAAGATGCTTGTATT
GCAAGCAGGCGCTACTTGCCCCTCACTACTCGCAATAATTTGGAGACCCTTGAAAACCAGCTTGACTATAA
GTTCCTCCACAAAGGTCTACTTGTACAAGCCTTTATCCATCCATCTTACAACAGGCATGGAGGAGGCTGCT
ACCAGAGATTGGAGTTTCTTGGGGATGCTGTTCTGGACTACTTGATGACATCCTATTTTTTCACAGTCTTC
CCGAAACTGAAACCTGGTCAACTGACCGATCTAAGATCTCTCTCAGTAAATAATGAGGCGCTAGCAAATGT
TGCTGTCAGTTTTTCGCTAAAGAGATTTCTATTTTGCGAGTCCATTTATCTTCATGAAGTTATAGAGGATT
ATACCAATTTCCTGGCATCTTCCCCATTGGCAAGTGGACAATCTGAAGGTCCAAGATGCCCAAAGGTTCTT
GGTGACTTGGTAGAATCCTGTTTGGGGGCTCTTTTCCTCGATTGTGGGTTCAACTTGAATCATGTCTGGAC
TATGATGCTATCATTTCTAGATCCGGTCAAAAACTTGTCTAACCTTCAGATTAGTCCTATAAAAGAACTGA
TTGAACTTTGCCAGTCTTACAAGTGGGATCGGGAAATATCAGCGACGAAAAAGGATGGTGCTTTTACTGTT
GAACTAAAAGTGACCAAGAATGGTTGTTGCCTTACAGTTTCTGCAACTGGTCGGAACAAAAGAGAGGGCAC
AAAAAAGGCTGCACAGCTGATGATTACAAACCTGAAGGCTCATGAGAACATAACAACCTCCCATCCGTTGG
AGGATGTTCTGAAGAATGGCATCCGAAATGAAGCTAAATTAATTGGCTACAATGAAGATCCTATAGATGTT
GTGGATCTTGTTGGGCTGGACGTTGAAAACCTAAATATCCTAGAAACTTTTGGCGGGAATAGTGAAAGAAG
CAGCTCATACGTCATCAGACGAGGTCTCCCCCAAGCACCATCTAAAACAGAAGACAGGCTTCCTCAAAAGG
CCATCATAAAAGCAGGTGGACCAAGCAGCAAAACCGCAAAATCCCTCTTGCACGAAACATGTGTTGCTAAC
TGTTGGAAGCCACCACACTTCGAATGTTGTGAAGAGGAAGGACCAGGCCACCTGAAATCATTCGTCTACAA
GGTAATCCTGGAAGTTGAAGATGCGCCCAATATGACATTGGAATGTTATGGTGAGGCTAGAGCAACGAAGA
AAGGTGCAGCAGAGCACGCTGCCCAAGCTGCTATATGGTGCCTCAAGCATTCTGGATTCCTTTGCTGA
- *Arabidopsis* DCL4 protein sequence is:

MRDEVDLSLTIPSKLLGKRDREQKNCEEEKNKNKKAKKQQKDPILLHTSAATHKFLPPPLTMPYSEIGDDL
RSLDFDHADVSSDLHLTSSSSVSSFSSSSSSLFSAAGTDDPSPKMEKDPRKIARRYQVELCKKATEENVIV
YLGTGCGKTHIAVMLIYELGHLVLSPKKSVCIFLAPTVALVEQQAKVIADSVNFKVAIHCGGKRIVKSHSE
WEREIAANEVLVMTPQILLHNLQHCFIKMECISLLIFDECHHAQQQSNHPYAEIMKVFYKSESLQRPRIFG
MTASPVVGKGSFQSENLSKSINSLENLLNAKVYSVESNVQLDGFVSSPLVKVYYYRSALSDASQSTIRYEN
MLEDIKQRCLASLKLLIDTHQTQTLLSMKRLLKRSHDNLIYTLLNLGLWGAIQAAKIQLNSDHNVQDEPVG
KNPKSKICDTYLSMAAEALSSGVAKDENASDLLSLAALKEPLFSRKLVQLIKILSVFRLEPHMKCIIFVNR
IVTARTLSCILNNLELLRSWKSDFLVGLSSGLKSMSRRSMETILKRFQSKELNLLVATKVGEEGLDIQTCC
LVIRYDLPETVTSFIQSRGRARMPQSEYAFLVDSGNEKEMDLIENFKVNEDRMNLEITYRSSEETCPRLDE
ELYKVHETGACISGGSSISLLYKYCSRLPHDEFFQPKPEFQFKPVDEFGGTICRITLPANAPISEIESSLL
PSTEAAKKDACLKAVHELHNLGVLNDFLLPDSKDEIEDELSDDEFDFDNIKGEGCSRGDLYEMRVPVLFKQ

KWDPSTSCVNLHSYYIMFVPHPADRIYKKFGFFMKSPLPVEAETMDIDLHLAHQRSVSVKIFPSGVTEFDN
DEIRLAELFQEIALKVLFERGELIPDFVPLELQDSSRTSKSTFYLLLPLCLHDGESVISVDWVTIRNCLSS
PIFKTPSVLVEDIFPPSGSHLKLANGCWNIDDVKNSLVFTTYSKQFYFVADICHGRNGFSPVKESSTKSHV
ESIYKLYGVELKHPAQPLLRVKPLCHVRNLLHNRMQTNLEPQELDEYFIEIPPELSHLKIKGLSKDIGSSL
SLLPSIMHRMENLLVAIELKHVLSASIPEIAEVSGHRVLEALTTEKCHERLSLERLEVLGDAFLKFAVSRH
LFLHHDSLDEGELTRRRSNVVNNSNLCRLAIKKNLQVYIRDQALDPTQFFAFGHPCRVTCDEVASKEVHSL
NRDLGILESNTGEIRCSKGHHWLYKKTIADVVEALVGAFLVDSGFKGAVKFLKWIGVNVDFESLQVQDACI
ASRRYLPLTTRNNLETLENQLDYKFLHKGLLVQAFIHPSYNRHGGGCYQRLEFLGDAVLDYLMTSYFFTVF
PKLKPGQLTDLRSLSVNNEALANVAVSFSLKRFLFCESIYLHEVIEDYTNFLASSPLASGQSEGPRCPKVL
GDLVESCLGALFLDCGFNLNHVWTMMLSFLDPVKNLSNLQISPIKELIELCQSYKWDREISATKKDGAFTV
ELKVTKNGCCLTVSATGRNKREGTKKAAQLMITNLKAHENITTSHPLEDVLKNGIRNEAKLIGYNEDPIDV
VDLVGLDVENLNILETFGGNSERSSSYVIRRGLPQAPSKTEDRLPQKAIIKAGGPSSKTAKSLLHETCVAN
CWKPPHFECCEEEGPGHLKSFVYKVILEVEDAPNMTLECYGEARATKKGAAEHAAQAAIWCLKHSGFLC

### Example 2

### Both DCL2 and DCL3, but not DCL4, transcripts are repressed during the innate immune response

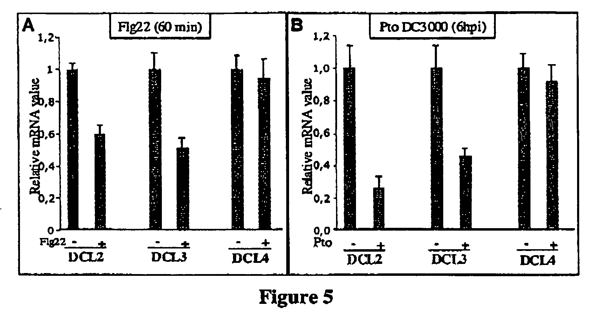

Because both DCL2 and DCL3 negatively regulate the *Arabidopsis* innate immune response, we tested whether their transcript levels were down-regulated during PAMP elicitation or pathogen infection. Quantitative RT-PCR analysis revealed that both DCL2 and DCL3, but not DCL4, mRNAs were indeed ~2-3 fold repressed upon either flg-22 or virulent Pto DC3000 treatments (Figure 5A, B). Figure 5A shows WT Col-0 seedlings were challenged with 1µM of flg-22 for 60 min and DCL2, DCL3 and DCL4 mRNA accumulation assessed by RT-qPCR. Figure 5B shows the same as in Figure 5A except that four week-old plants were challenged with DC3000 at 2x10⁷ cfu/ml for 6h.

These results suggest that both DCL2 and DCL3 are transcriptionally repressed during the plant innate immune response.

### Example 3

### Identification of Endogenous Repressors of DCL2/DCL3 Expression

*Arabidopsis* transgenic lines carrying 1.5 Kb upstream regions from either DCL2 or DCL3 are fused to a *GFP* reporter gene and further mutagenized (using approaches known by those skilled in this art such as Ethyl Methane Sulfonate (EMS)). A screen for a loss of GFP is further performed to identify negative regulators of either DCL2 or DCL3 transcription. The candidate repressor genes are isolated by map-based cloning and further screened for enhanced susceptibility to virulent bacterial and fungal pathogens. The repressors are then expressed under a strong 35S promoter or pathogen-inducible promoters (*e.g*., WRKY6, PR1) and stable transgenic lines generated to confer enhanced disease resistance to pathogens. By constitutively enhancing the expression of negative regulators of *DCL2* and *DCL3* expression, increased resistance to bacterial and fungal pathogens is achieved in a variety of plants, including crops.

Similarly, positive regulators of DCL4 transcription that play a role in antiviral defense are identified. This comprises mutagenesis of *Arabidopsis* transgenic lines that report DCL4 transcription by assessing the effect of DCL4 on a constitutive GFP construct, and further isolate mutants that abolish GFP expression. The corresponding genes are then identified, using methods known by those skilled in the art such as map-based cloning, and their, constitutive or conditional, overexpression in various plant species is implemented to confer antiviral resistance. Transgenic plants overexpressing, conditionally or constitutively, repressors of DCL2 and DCL3 transcription as well as activators of DCL4 transcription are generated to confer broad spectrum resistance to unrelated pathogens.

Furthermore, the same transgenic lines reporting DCL2 and DCL3 transcriptional activities are used to screen for chemical compounds that trigger down-regulation of GFP mRNA. This is achieved by monitoring GFP mRNA levels (using methods known by those skilled in the art such as Northern analysis, semi-quantitative RT-PCR analysis or quantitative RT-PCR analysis) after exposure of these transgenic lines to a library of chemical agents. Molecules that repress GFP mRNA levels are further used to confer antibacterial and antifungal resistance in a variety of plant species including crops.

Additionally, the same library of chemical agents will be used on transgenic lines reporting DCL4 transcriptional activity to identify molecules that enhance GFP expression. These chemical compounds will likely confer enhanced resistance to viral pathogens by promoting DCL4 transcription. Cocktails of chemical agents that promote DCL4 transcription and inhibit both DCL2 and DCL3 transcription are further used to confer broad spectrum resistance to unrelated pathogens.

Sequences from DCL2, DCL3 and DCL4 predicted promoters allow those skilled in the art to generate constructs reporting either DCL2, DCL3 or DCL4 transcription.
- *Arabidopsis* DCL2 promoter sequence is: 
ATTCTTTGGCCTGCTCTATATAGTTTGTTTCTCGTTTTTCTTATCCCCAAATGCATCATCATCGTTTTCAA
GAAGCAGTACACTCTCAAGAAGTTCATTGCCAAGAAAGGACCTATCACACTTGTACTGTGGATTCTCCAAG
ACCTCTGCAGAATGCCTGTGGTTTGGTTCGGTTACATGGCATACTTGTTCTATCTCATATTCTTTCCTTGG
TTCTCCGGTGAAGTGTTTGCTGATTCTGGAGACAGAGCATACATGACTATTATGGGATGGGTGGTGACGAG
CTCAGGCGCAGATAGGAAACATGAATACATTGGACAACCTGATGTAATGGTTGTGGTGATCCCACATGTGG
TCTTTGTTGTTATCCCCAGTGTCTTGGTTGTGTTGTCTGGTTGCTGAGAGAGAAATCTACAAAGATCAC
ATTCGAACTGTCTCTGGTAAGAAAGAGGATGACCATGACCGGGGAAGGAAGAAGAGATCACAACGCCGCTC
ACTGTTATTCTCGAACAGAAGACTGTTTCGGAAATCGGTCTTGCTGGCTTCATTAGCTCTATATTGGAAGC
ATTTCAAGGTACCACTTGTATATTAACTAATGATTCGTTTATTTCCATCTTGCATTGGCAAAAACCTGCGA
TCATGTTCATCCGTTGTCTGTTTTCATTCTGCATTCACTTCTGAAGTATATTTGTTTTTATTGACAGAAT
TGCTGGGCATTAGGTAGAGCTTATGAGATGAATGTGGTTCATTTTCCAGGTTACAGCCTTGTAGTTCCATT
GTTGCTACTATATGTTATCTGCAAAACCCATAAAGTTCCATGAGATTTGAATCTATGGAAGTTTTGGTGTT
TTTATGATTTTGATTATGAAGAAACACATTTATAGGGGGTTTCTTATGTGTTGTTTGATAGAGTTGATAT
ACTATATAGATCAAGAAAGTTAGACCAAATTATTCTGTATCCTTTTGCTTTTATTTTTTTTTACAGCTAGA
CCAAAAAAGTACAAGTGGTTTTGCTCAATTTGGAAAAAAAAACATTAAAAATATTAAAAAACAAAAATGA
ATAATGACGTAAGCGAAAGCTTCTGTCATAGTCTCACAGTCACAACAAAACAGTCAATCCCCCAAAGAATT
ATGGTAGCAGTCAATAATCCCATAAATAATCTTCTTCAACAGTTTTTTTCTCTTAAATTTTTGTTTCGAAA
ACCGCTCACTACTTCTTAATCGAAATGACTGACTGAGAGCTTTAGCTTTGATCGTTCGTCTTCGTGATTC
CTTTCTTCTTCTTCTCTCCGCTACTGTCTGTACCTGAAACTACTGTCCTCGATCGCTGCTTTGTCTTTCGA
GGTTCTCTAATTTGTTTTTTCTTATTATTATCTCTGGGGTTTGCTTTTGCGTTAATCCTCTTTCTGCTCGA
CTTACGGCGATTTTTGATATTTGCAGATTAAAAAGTTAGGTTTTTTTTTCGTAAATCTACTGTATATGTTT
TAGATGACC
- *Arabidopsis* DCL3 promoter sequence is: 
TTATCCGAATTTGACTGGATATAGATCCGACCCATATATCCAGAATCCGTGTGTCAGAATGTGTTAAATTG
CCCATTTTACCCCTTGACGAAATCTCTTAGTTTGCCTTAGTTTGAGAACATTTTATGTAATATTTTACCCT
TTATATAGTTATTTATTTTTGAGTAATTTCCAATTAATATTATAGATCAAACTGTTTCTAACTATTAAGTT
AGTGCGTTTTGTTATCTATTCCTAACCTTAAGTTAAATAAGACATGCTTTGTACACTGTTTTCTTGGTGAA
TAGAGAGGAAAATTCACTTTAGCATTTTGATATATATGGATTTAGTTTTGTGTGTTAAATCTATGAAGGTA
TATGATACTCCTGTGGAGGTGTGGAAAACTTTGTTATCTTTTCTGTCGATTCTATTCTAAATTTGGATAAT
 
CTCTAGCTAATGTACATTAATTGCAACAAGTTCTAGTGTCTATAATAAACTCTTTCATCACTCCCTCTCTA
AGTAAAAATTAGATTATGAATCTTAGGTTTGGTTAAAAGGCCAACCAATTCTTGTATATAGGGACTCTAAC
CATGTGTCTGATCTATGTGATTGATTTTTCTCCACTAAAAATCATTTTCTAATCAAACATATGATTCATGG
TGGTAGTAGATTCTTCACTAGCTAGCGTAGCTTCTTCTTATGTGTTGAATTTTGTCTCCCAACTGAGTTGG
TCATTCTCCGTTTTTTTATATTTATCTAATTATTTTTAAGTTGGATTGTTAATGAAATTTTATTTTATGTT
GATAATGTCGTTCAGCTGAGTCATGTTGGAAAGGTAAAACGTAATATCTCTCTGGGATGTACAATTATGTA
CTTGTTTAGTTAAATAAATATTTAAACTTTTTTGGATTTATTTTTGCATTAAACAAAACAAAAAAAAAAAA
CCTAAAACGTAATAGACGACTAATTTGAACATTAAATAGTGACTCGTTTCGTTGCTTCAGATAATATTTGG
TAGATCACACCAGATTGCAGAGCATATTCTCTCATATCCCAAATTTTCTTACTTCCAATAGTCGATTTCGT
CGTATCTAAAACGATTTAGCTGCGCAAATTTATGTTGAATAAACAAAGCAGTGGAAGAGGAAAAAGAAGTT
AGTTCTAATTAATCTTTCGTTTAAGTAAATATATAGATCTTTGGGAGACGTAAGTTTTTATTGTTTATCTC
TTATATAACCTACACTTTTAGGCTCCAATGAATCTATGACTTATTGTACAAGAAAGTTATGGACATGGGTT
GGTTACATGATTACATAATGGTTTGTTTAGTTCTACGTTTGTGGTCCATTGTTACAAACTTTGTATGAACG
TACGTGAGTAGGTTAATTAGTTTTGTACTTACGTTCCTTACTTCCCTGATTCTTGTAGGTATAACTAAGGG
CAACGACTTTCTCCTCTAAGCTCAAATCTTTCCGTTCTCGATATTTCTCCTTACATGAGAAGGACAAAGAT
AAGGCTTTA
- *Arabidopsis* DCL4 promoter sequence is:

ACGATCAAAATATCAACATATGTGGTCGAACGACTGAAAACCAAACTAGCCAAAAACACACATCAACTTTT
TTCGGTTTGGCTTAGTTACCCTAGAAGATTACCCTCAAACAAACAAAACCCAAAAAAATTAAAATATTAAT
TTTAAAATAGAAGAAACATATTTAAGAAAAAGTCATTTCTTTTAATTGTTACTTTAGTTTTGGACACTAC
AAGCTGACCGAGTAGTCCAACATTCGATATGAAAGAAAGTGGCTCACTGCCTATATAGCTCTCGCCACTGG
GAGAATACAAGGTTCGATCCTAGATTTTGATGGAATGTCAACCAAGATTCATAATTTGTGCACTTGTTTTT
TCCCCATAATTTCTTAGTTAAAGGTACATCAATATAAATTTTAAGTAGTCAAGTGTGTCCAATCAACAAAA
GGTAAATCTATAAAAATTTGGAAATTTATCAAAATGAATCAATGTAATGTTGTATAATATGGAAAAATATG
TAGTTTAGATGTTTTTACTTTATAATACTTATCATTGCTGCATCACCACTCATATTAAAAATGACTAAATA
AATACTCAAGTATGTGTTAACCTCATAAAAATGAAATGAGGTTAAACTTCTGCAATTTCACATATGGTAAA
TTGTTTCTAGAGCTAAAAGACCTGCAACAAGGTGATTCAATCTTCTTCTTCGACATTTAACAAGTCGGAAG
TTCATGCTACAAATATCAGTACAAAAAATCATACTCTATCCATTTCAAATTAAATTTAGTTCAAGTGTATA
CGTAAATTTAAAAAATGAACGCATTTTAGATTTTCAAAATCTGAGATCAAATACCTAATTTAACCTTATCA
AAATCAAGAGTCATCTAGATTGTAAGGGTATAATAGAAATTATAATGTTTTTAATAATTTTTTGTAAAAAT
TTTAAATGACACTTAAAGTAAAACGGAGAGAAGATAACTTAACAGCCACGAAATCGCGACTTGAGATTTCA
AAGAGATAAAGTATTCATCTATGTACTTTGGCACATCAATACTCTTAAAATTTACCAAAATATGTAATATA
ACATCCCTAACCATCAACAACAATCAACATAAATTTTAATATATATGTTTTTGTAATTTTCGTAAAAATGT
TAAAACAACACTTATAGTAAAACAAAGAGAGGATAACTTAACAGCCAAAGAATTGAGATTTGAGATTTCGA
AGAGATAAGATATTCATCTATATATCTTGACACATCAGCACTCTAAAAATTTACCAAAAGATGTATTTTAA
AATCTCTAAACTCAATAACTCCACAAAAATTTTCAGAATCAATGATTGTAGAAACACATGATTTCTGGTTC
AGAATTTCACACACTCCACCCAAAAAAATACCCTTAAAAAGTTATAATTGTATTGATTAGCTGATAAAATC
AATTTATTGGAAAGAAATCCTAATAATAACGCTGTAATAGAAGAGAAGAGAAGAGAGAGGGAGACGTGAGA
TCGTGAATT

### Example 4

### CasiRNAs Trigger DNA-Methylation of Plant Defense-Related Genes to Repress Their PAMP Transcriptional Activation

The above phenotypical analyses indicated that activators of the plant defense response are likely repressed by RdDM. To identify such activators, we isolated genes that are up-regulated by flg-22 peptide, extracted their 2kb upstream regions and performed a blast analysis of these DNA sequences against publically available small RNA databases that contain known casiRNAs. This three-step analysis allowed the identification of genes that are potentially repressed by casiRNAs. Among those candidate genes, we identified At4g01250, a well-characterized WRKY transcription factor that positively regulates the *Arabidopsis* defense response. A casiRNA cluster covered a region of 278 bp within the At4g01250 promoter region and DNA methylation occurs right on the top of the casiRNA cluster (see World Wide Web address epigenomics.mcdb.ucla.edu/DNAmeth/ from Jacobsen Lab, UCLA). These small RNA molecules are majoritarily 24nt to 22nt long and therefore are likely products of DCL3- and DCL2 processing, which is consistent with the enhanced pathogen resistance observed in the *dcl2-dcl3* mutant (Figure 4).

The DNA methylated region of At4g01250 promoter contains 2 copies of the W-box element, which are known binding-sites for the plant defense-related WRKY transcription factors (see At4g01250 promoter sequence hereafter). The presence of casiRNAs matching this promoter region suggested that a RdDM mechanism represses transcriptional activation of At4g01250 by inhibiting the accessibility of the yet unknown, activator of At4g01250 transcription.

To test this hypothesis, we challenged the casiRNA-deficient mutants *dcl2-dcl* with flg-22 and analyzed whether At4g01250 mRNA would be hyper-induced in these mutants. We indeed found that flg-22-treated *dcl2-dcl3* mutants displayed a hyper-induction of the At4g01250 transcript as compared to wildtype-elicited seedlings. This hyper-induction correlated with the loss of asymmetrical methylation in the At4g01250 promoter region *of dcl3* and *dcl2-dcl3* naive mutants (as assayed by bisulfite sequencing analysis, data not shown).

Similar results were obtained with the At3g56710 gene (encoding for SIB1, Sigma-factor binding protein 1), where a cluster of casiRNAs covers a promoter region of 273 bp, which is also methylated right in front of the siRNA cluster (see World Wide Web address epigenomics.mcdb.ucla.edu/DNAmeth/ from Jacobsen Lab, UCLA). This DNA-region also contains key cis-regulatory elements, such as the W-box element, that contribute to transcriptional activation of pathogen-responsive genes (see SIB1 promoter sequence hereafter).

These results indicate that specific casiRNAs negatively regulate the transcriptional activation of a subset of PAMP-responsive genes.
>At4g01250 promoter region that carries the casiRNA cluster directing RdDM: 
CGAGATAAACTTTGAATGGTTACAGCGATCCAAGCGGACAAACCATCTACAAACCGAACCGCTTTTTATTT
ATCATTCACAATCTATAGTTCGTAATGAAGCAGAACCAAAAAAACAAATAAATTTGATGCGGATTGACTTT
AAACTAAGTTTGCAGCGGTTTGTTCTCTGTCCTAAAAAATAGTATAGATTGGACCGTTGACGGATTAGTTC
GCACTAACCACAACATATCCGTCTCAAAAAATAGTATAGACTGTACTGCTGACGGATTATTCCGG
 >At3g56710 promoter region that carries the casiRNA cluster directing RdDM:

GGACATGGTTAGGTCCTTGTTCCCTCAAGAGTACTCGACGAAGTTTATTATTGTTTCATGCGGAATTTGAT
TCCTTGCTATAGACAATGGAATACATGATCTATATTGACGATACTTCTGGCGCTTTTGCTTCCGACTGTTC
AGATCTGATTTTTATCATTGACAATCAAGAAGATTGGCCGACATTCGCAGCGGAATTGGCATCCTATCGCT
CCTTAGTTTGTTTTTCCTTCTTTTCGTATTAGATTTCTTCTTCGTAGTTCTAATACTCGAGCAGA

In bold is the core motif of the defense regulatory element W-box.

By contrast to the results observed when stimulated by flg-22 conditions, neither At4g01250 nor At3g56710 mRNA levels were significantly affected in a naive *dcl2-dcl3* mutant background as compared to the non-challenged wildtype control (data not shown). This suggests that the yet unknown transcriptional activators are not present or not active in naive conditions.

### Example 5

### Association with Transposons

It is known that a large proportion of *Arabidopsis* casiRNAs are derived from transposon-related sequences. We next analyzed whether our candidate promoter set could contain such repeated sequences. We re-annotated most of the *Arabidopsis* transposons and relocated those in the *Arabidopsis* genome, and found that 23% of the flg-22-induced genes contain remnant transposons within their promoter regions. Those are mostly non-autonomous transposons because they are lacking key elements required for their transcription and/or transposition. The distribution of casiRNA clusters coincides precisely with the remnant transposon sequences as exemplified with At3g56710 promoter region where a remnant LINE retrotransposon likely gives rise to the casiRNA cluster.

These small RNA molecules might be produced in *cis* by remnant transposons, or by a few 'mother' autonomous transposons, located elsewhere in the *Arabidopsis* genome that could direct RdDM in *trans* onto any remnant transposons in the genome that would display high sequence homologies with the 'mother' transposon sequences.

We infer that remnant transposons, located within some promoter regions, direct an epigenetic regulation involved in the transcriptional repression of nearby genes. The presence of remnant transposons also likely provides cryptic promoters for the nearby genes in biotic and abiotic stress-conditions. This mechanism of gene regulation seems not to be restricted to promoter regions as we also observed casiRNA clusters in DNA-regions corresponding to coding regions. We also found that several key activators of the defense response are slightly, but reproducibly, more elevated in non-challenged *dcl2-dcl3* double mutant (data not shown). These candidate genes include some resistance genes from the RPP5 cluster (*e.g*., RPP4) and the receptor-like kinase BAK1 that might play a role in the potentiation of the defense response observed in both *dcl3* and *dcl2-dcl3* mutant backgrounds (Figure 4E).

### Example 6

### Identification of Genes Hyper-Induced by PAMP

To identify the whole set of genes that are hyper-induced in flg-22-treated *dcl2-dcl3* mutant background and potentially regulated by casiRNAs, we first performed a large-scale mRNA profiling using standard *Arabidopsis* microarray. For this purpose, we treated Col-0 and *dcl2-dcl3* seedlings for 30min with flg-22 peptide and selected genes (i) that were hyper-induced in *dcl2-dcl3-*elicited mutant as compared to Col-0-elicited control (ii) or genes that were solely up-regulated in *dcl2-dcl3* mutant seedlings (the latter are likely induced earlier in the *dcl2-dcl3* mutant background). For this particular analysis, we selected only the subset of genes that were hyper-induced in the *dcl2-dcl3* mutant by flg-22.. We found that 337 genes were hyper-induced in *dcl2-dcl3-elicited* mutant as compared to Col-0-elicited control.

Among those, we identified the At3g56710 internal control discussed above. Further bioinformatic analysis showed presence of many casiRNA clusters in some promoters, coding and 3' UTR regions that may play a role in transcriptional gene silencing of PAMP-responsive genes, as shown in drawings depicting casiRNA clusters available on the web at nups.gsf.de/cgi-bin/proj/plant/gbrowse/gbrowse/siRNA.

However, several genes carrying casiRNA clusters within their promoters were not hyper-induced in the flg-22-treated *dcl2-dcl3* mutant background, although the corresponding DNA regions were methylated (data not shown). This indicates that RdDM alone is not sufficient to trigger transcriptional silencing of these endogenous genes.

By constitutively enhancing the expression of each of these candidate genes (using the methods described above), increased resistance to a broad spectrum of pathogens is achieved in a variety of plants, including crop species. This approach allows the identification of uncharacterized genes that are likely involved in broad-spectrum resistance to pathogens. The above approach can also be applied to genes undergoing casiRNA-mediated negative regulation, that are involved in response to viruses as well as to non-biotic stresses, including, but not restricted to drought, salinity and cold.

### Example 7

### casiRNAs Trigger DNA-Methylation of Some Pre-miRNA/Pre-siRNA Promoter DNA Sequences and May Repress PAMP Transcriptional Activation

We recently showed that miR393, a canonical miRNA regulating auxin-receptors, is transcriptionally induced upon flg-22 treatment which miRNA contributes to antibacterial resistance. The overexpression of miR393 elevates resistance to the virulent Pto DC3000, whereas overexpression of AFB1, an auxin-receptor that is partially refractory to miR393-directed cleavage, promotes susceptibility to the same bacterium (Navarro, *et al., supra*). We later describe many additional flg-22-induced primary miRNA (pri-miRNA) transcripts also contribute to plant disease resistance. We sought to identify those miRNA expressing genes that were repressed by transcriptional gene silencing as observed with some protein coding genes (*e.g*., At3g56710). The 2kb long sequences located upstream of the PAMP-responsive miRNA precursors and were subjected to a BLAST analysis against several publically available small RNA databases and found that several pre-miRNAs contain siRNA clusters within their putative promoter regions. An example is the miR416 precursor promoter region carrying casiRNAs and a remnant transposon sequence. These casiRNAs are mainly 24 to 22nt long which is consistent with a DCL2 and DCL3 processing as well as with the enhanced disease resistance observed in the *dcl2-dcl3* mutant (Figure 4). Cytosine DNA-methylation (RdDM) often occurs right on the top of these casiRNA clusters (see World Wide Web address epigenomics.mcdb.ucla.edu/DNAmeth/ from Jacobsen Lab, UCLA).

We also found that many early evolving miRNAs or pri-siRNAs (which represent endogenous near-perfect endogenous hairpin structures that give rise to a population of siRNAs. This indicates that miRNA as well as siRNA genes might also be repressed by transcriptional gene silencing.

Sequences from PAMP-responsive pre-miRNAs/siRNAs potentially regulated by RdDM are shown in Table 2.

**Table 2**

| miRsrot506 sequence: |
|---|
| CGAAACTGAACCCGGTTTGTACGTACGGACCGCGTCGTTGGAATCCAAAAGAACCGggttcgtacgtacgc tgttcaTCG |

| miRspot418 sequence: |
|---|
| AGGGTTTAGGGTTTAGGGTTTTGGTTTAAGGGTTTAGGGTTAAAAGTTtatggtttagggtttacggtttTT GGGTTTGGGATTTAGGGTATAGGGGTTAGGGTAAAGAATTTATGATTTTATGTGTAGGATTGAATATAAAA CTAGAACCTCAACAAGATACCGAAGAGTGGACCGAACTGTCTCACGACGTTCTAAACCCAGCTCA |

| miRspot730 sequence: |
|---|
| TTAGATCATCATCCATGGCACTGACGCCGTTCACGGCAACTGCCGTAGACGTTGTTGTTGCCGTGAACGGC GTGAGTGCCGTAGATTATTGGCTTAT |

| miRspot29 sequence: |
|---|
| TCAAAATGGCTAACCCAACTCAACTCAACTCATAATCAAATGAGTTTAGGGTTAAATGAGTTATGGGTTGA CCCAACCCATTTAACAAAATGAGTTGGGTCAACCCATAACTCATTTAATTTGATG |

| miRspot18 sequence: |
|---|
| TAAATGGTTAACCCATTTAACAATTCAACCCATCAAATGAAATGAGTTATGGGTTAGACCCAACTCATTTA ACAAAatgagttgggtctaacccataactCATTTAATTATAAACTCATTTGATTATGAGTTGGGTTGGGTT GGGTTACCCATTTTGA |

| miRspot43 sequence: |
|---|
| TCAAAATGGGTAACCCAACTCAACTCAACTCATAATCAAATGAGTTTAGGGTTAAATGAGTTATGGGTTGA TCCAACCCATTTAACAAAATGAGTTGGGTCAACCCATAACTCATTTAATTTG |

| miRspot1204 sequence: |
|---|
| AGAATTGAAGATGCATGGAATGGTGTGTGGGAAAGGCAAAGCACCATGACTTCACAAGTTGCGTGAGGGCA AAGTATCTATTTTGGGTGAAACCATTTTGCCCTCTCAGCCGTTGGATCTCTTTCTTCCTTCAtcatcattc cgtcatcctcttTGTTC |

| miRspot107 sequence: |
|---|
| TCTCGCTAGAGCTCTTCTCTCCCGGCTGTCTCCTGCTCCTGCCTAAGCGATGGCCTGGAGAGTGCTCTAGT GGTG |

| miRspot199 sequence: |
|---|
| TCTCTTAACTTTGATGAAACCTAGGCAATTGTCTCTTAGTTAAGAGATAAttggtcttggtttcaccaaat tTAAGAGA |

| miRspot1047 sequence: |
|---|
| TCGAAACGAACACAAAACCTGCGGTTGCGACAGCGGCTGCGGCAACGTTGGCGGCGACGAAACGAACAACA ACCTGCGGCAGtgttaccgttgccgctgccgcAACCGCAGCCGCTGCCGC |

| miRspot711 sequence: |
|---|
| CTGTCACTGGACCGCAAGAACATTGATAGGGCACACTCCATCTCTAATGTCTCATGAGGGTCAATGACAC |

| miRspot326 sequence: |
|---|
| CTGGACCGCAAGAGCATTGATAGGGGTCACTCCATCTCCAATGTCTCATGATGCTCCATGA |

The set of pre-miRNAs or pre-siRNAs can be used to elevate resistance to pathogens. Individual or groups of pre-miRNAs/siRNAs are expressed transgenically in plants using methods known by those skilled in the art, using promoters not repressed by RdDM. Thus, a constitutive or pathogen responsive promoter (including but not limited to, for example, the WRKY6 promoter, the PR1 promoter and the like) is operatively linked to a nucleic acid sequence which encodes one or more individual pre-miRNA or pre-siRNA sequences of Table 2. Expression of the above sequences (+ 40 nt upstream and downstream of the miRNA or siRNA hairpins) is either constitutive or, preferably, is driven by promoters that are known to be broadly responsive to bacterial, fungal and viral pathogens. Examples of such promoters include, but are not restricted to, WRKY6 and PR1. This minimizes detriment to plant development and physiology in non-infected conditions.

### Example 8

### DNA-Methyltransferases Negatively Regulate Plant Defense Response

The results of Example 7 indicate that casiRNA-directed DNA methylation negatively regulates the plant defense response. Therefore, *Arabidopsis* mutants lacking key components of the RdDM pathway are more resistant to virulent pathogens. Virulent Pto DC3000 were inoculated on DNA-methyltransferase mutants that are impaired in *de novo* DNA-methylation (*e.g.*, DRM2) or in maintenance of non-CG methylation (CMT3). No enhanced resistance to this bacterium was observed in *drm1, drm2* nor *cmt3* single mutants (data not shown), but *drm1-drm2-cmt3* triple mutants display ~20 fold less bacterial titer and significantly less bacterial disease symptoms as compared to wildtype infected plants. Thus, DRM1, DRM2 and CMT3 act redundantly as repressors of plant defense and programmed-cell death.

We tested whether genes that are repressed by TGS such as At4g01250 and At3g56710 were hyper-induced in the PAMP-treated *drm1-drm2-cmt3* mutant background. We challenged the triple *drm1-drm2-cmt3* mutant for 30 min with the flg-22 peptide and monitored the transcript levels of At4g01250 and At3g56710 by quantitative RT-PCR analysis. We found that both genes were hyper-induced in the *drm1-drm2-cmt3-elicited* mutant as compared to La-er-challenged seedlings. Ten day-old seedlings were elicited with either 100 nM of flg-22 or flg-22^{A.tum} for 30 min and qRT-PCR performed on At4g01250 and At3g56710 mRNAs. Transcriptional repression of both genes implicates DRM1, DRM2 and CMT3.

The above results prompted us to analyze the resistance of *Arabidopsis* mutants that are impaired in MET1 function, the remaining *Arabidopsis* DNA-methyltransferase that is involved in maintenance of symmetrical CG methylation as well as in RdDM. We also tested the resistance of plants altered in decrease in DNA methylation 1 (DDM1) function. DDM1 encodes a protein related to SWI2/SNF2-like chromatin remodeling enzymes that is also involved in CG methylation. Both *met1* and *ddm1* mutants (from the 1^{st} to the 5^{th} generations) were significantly more resistant to Pto DC3000 as indicated by lower bacterial titer and attenuated bacterial-triggered disease symptoms. Five week-old Col-0 and *ddm1* mutant (from 2^{nd} to 5^{th} generations) plants were syringe inoculated with a Pto DC3000 concentration of 10⁵ cfu/ml and pictures taken 4 dpi. Five week-old Col-0 and *ddm1* mutant (from 2^{nd} to 5^{th} generations) plants were syringe inoculated and bacterial growth measured 4 dpi. Therefore, both DDM1 and MET1 act as negative regulators of plant defense. We conclude from these experiments that both symmetrical and non-symmetrical cytosine DNA methylation negatively regulate the plant defense response.

Thus, knock-out or knock-down DDM1, MET1, DRM1, DRM2, CMT3 genes in various plant species, including crops, are able to enhanced pathogen resistance. This may be done by, for example, Targeted Induced Local Lesions in Genomes (TILLING) of the *MET1* and *DDM1* genes from non-transgenic plant species (*MET1* and *DDM1* are conserved across most plant species including crops), RNAi of all MET1, DRM1. DRM2 and CMT3 mRNAs using a hairpin construct that carries a portion of 100bp of each gene to allow combinatorial silencing of all these mRNAs, the generation of an artificial microRNA that target MET1, DRM1, DRM2 and CMT3 transcripts. The resulting plants can optionally be transformed with constructs carrying either the strong 35S promoter or a pathogen-inducible promoter (*e.g.*, WRKY6, PR1) fused to the *DCL4* coding sequence to allow, additionally, enhanced resistance to viral pathogens (see introduction). Backcrosses with wildtype plants at the 3^{rd} to 4^{th} generations of self will be required to avoid transgenerational miss-regulation of genes involved in development/physiology that are also regulated by RdDM.

Coding as well as protein sequences from the *Arabidopsis* MET1, DRM1, DRM2, CMT3 and DDM1 are as follows:
- The *Arabidopsis* DRM1 (At1g28330) coding sequence: 
ATGGTTCTGCTAGAGAAGCTTTGGGATGATGTTGTGGCTGGACCTCAGCCTGACCGTGGCCTTGGCCGCCT
CCGTAAGATCACCACCCAACCCATTAATATCCGAGATATAGGAGAAGGGAGCAGCAGTAAGGTGGTGATGC
ATAGGTCGTTGACCATGCCGGCGGCAGTGAGCCCTGGAACTCCAACGACTCCAACCACTCCGACGACGCCA
CGTAAGGATAACGTGTGGAGGAGCGTCTTTAATCCGGGAAGCAACCTCGCCACTAGAGCCATCGGCTCCAA
CATCTTTGATAAACCCACCCATCCAAATTCTCCCTCCGTCTACGACTGCGTTGATAATGAAGCTCAAAGGA
AGGAACATGTGGCACTGTGTTTAGTGGGCGCGTGGATTAAGTGA
- The *Arabidopsis* DRM I protein sequence: 
MVLLEKLWDDVVAGPQPDRGLGRLRKITTQPINIRDIGEGSSSKVVMHRSLTMPAAVSPGTPTTPTTPTTP
RKDNVWRSVFNPGSNLATRAIGSNIFDKPTHPNSPSVYDCVDNEAQRKEHVALCLVGAWIK
- The *Arabidopsis* DRM2 (At5g14620) coding sequence: 
ATGGTGATTTGGAATAACGATGATGATGATTTTTGGAGATTGATAACTTTCAATCTTCTCCACGGTCATC
TCCAATACATGCAATGCAGTGTAGGGTCGAAAATCTAGCTGGTGTAGCCGTGACAACTAGTTCTTTGAGCT
CTCCTACTGAGACAACTGATTTAGTTCAGATGGCTTCTCAGACGAGGTTTTGCTACATTGTTTGACATG
GGATTTCCTGTTGAGATGATTTCTAGAGCGATCAAGGAAACTGGACCAAATGTAGAAACTTCGGTTATAAT
TGATACTATCTCCAAATACTCAAGCGACTGTGAAGCTGGTTCTTCCAAGTCCAAGGCTATTGATCATTTCC
TTGCTATGGGATTTGATGAAGAAAAAGTTGTCAAAGCCATTCAAGAACATGGAGAAGACAATATGGAAGCA
ATTGCAAATGCATTGCTCTCTTGTCCAGAGGCTAAGAAACTGCCAGCAGCAGTAGAGGAAGAAGATGGCAT
TGACTGGTCATCAAGTGATGATGATACCAATTACACCGATATGTTAAACTCAGATGATGAGAAAGATCCAA
ACTCAAATGAAAATGGCAGCAAAATACGGTCTTTGGTGAAGATGGGTTTCTCAGAGCTTGAAGCTTCTTTA
GCTGTCGAGAGATGTGGAGAAAATGTGGATATTGCAGAGCTCACAGACTTCCTTTGTGCTGCTCAAATGGC
TAGGGAATTTAGTGAGTTTTACACTGAACATGAAGAACAAAAGCCTAGACATAATATTAAGAAAAGGCGGT
TTGAGTCAAAAGGAGAGCCAAGATCATCTGTTGATGACGAGCCGATTCGTCTACCAAATCCAATGATAGGA
TTTGGGGTTCCAAACGAGCCCGGACTCATTACACATAGATCGCTTCCAGAGTTAGCCCGAGGCCACCTTT
TTTCTACTATGAGAATGTCGCCCTCACACCTAAAGGCGTTTGGAGACTATTTCCAGGCACTTGTTCGAGA
TCCCACCTGAGTTTGTGGACTCAAAATATTTCTGTGTTGCAGCGAGGAAGAGAGGCTACATCCACAATCTC
CCCATCAACAACAGATTTCAGATTCAGCCTCCACCAAAATACACCATCCATGATGCATTTCCTTTGAGTAA
GAGATGGTGGCCAGAATGGGATAAAAGGACCAAGCTTAATTGCATTTTGACTTGTACAGGTAGTGCTCAGT
TGACTAACAGGATTCGTGTAGCCCTTGAGCCTTACAATGAAGAACCAGAACCGCCTAAGCATGTACAAAGA
TATGTGATTGACCAGTGCAAAAAATGGAATTTGGTTTGGGTGGGTAAAAACAAAGCTGCCCCACTCGAGCC
AGATGAGATGGAGAGTATTCTGGGATTTCCAAAAAATCATACTCGTGGTGGAGGCATGAGTAGAACTGAGC
GCTTCAAGTCCTTAGGAAATTCGTTTCAGGTTGATACTGTGGCGTATCATCTGTCTGTCCTGAAGCCCATT
TTCCCACATGGAATCAATGTTCTCTCTCTTTTCACGGGTATTGGTGGTGGGGAAGTGGCACTTCATCGTCT
CCAAATCAAAATGAAGCTTGTTGTGTCTGTTGAGATTTCAAAAGTCAACAGAAATATTTTGAAGGACTTTT
GGGAGCAAACTAACCAGACTGGAGAATTGATCGAGTTTTCAGACATCCAACACTTGACTAATGACACAATC
GAAGGGTTGATGGAGAAATATGGTGGATTTGATCTTGTAATTGGAGGAAGTCCTTGTAACAATCTGGCAGG
CGGTAATAGGGTAAGCCGAGTTGGTCTTGAAGGTGATCAATCTTCGTTGTTCTTTGAGTATTGCCGTATTC
TAGAGGTGGTACGTGCGAGGATGAGAGGATCTTGA
- The *Arabidopsis* DRM2 protein sequence: 
MVIWNNDDDDFLEIDNFQSSPRSSPIHAMQCRVENLAGVAVTTSSLSSPTETTDLVQMGFSDEVFATLFDM
GFPVEMISRAIKETGPNVETSVIIDTISKYSSDCEAGSSKSKAIDHFLAMGFDEEKVVKAIQEHGEDNMEA
IANALLSCPEAKKLPAAVEEEDGIDWSSSDDDTNYTDMLNSDDEKDPNSNENGSKIRSLVKMGFSELEASL
AVERCGENVDIAELTDFLCAAQMAREFSEFYTEHEEQKPRHNIKKRRFESKGEPRSSVDDEPIRLPNPMIG
FGVPNEPGLITHRSLPELARGPPFFYYENVALTPKGVWETISRHLFEIPPEFVDSKYFCVAARKRGYIHNL
PINNRFQIQPPPKYTIHDAFPLSKRWWPEWDKRTKLNCILTCTGSAQLTNRIRVALEPYNEEPEPPKHVQR
YVIDQCKKWNLVWVGKNKAAPLEPDEMESILGFPKNHTRGGGMSRTERFKSLGNSFQVDTVAYHLSVLKPI
FPHGINVLSLFTGIGGGEVALHRLQIKMKLVVSVEISKVNRNILKDFWEQTNQTGELIEFSDIQHLTNDTI
EGLMEKYGGFDLVIGGSPCNNLAGGNRVSRVGLEGDQSSLFFEYCRILEVVRARMRGS
- The *Arabidopsis* CMT3 (At1g69770) coding sequence: 
ATGGCGCCGAAGCGAAAGAGACCTGCGACAAAGGATGACACTACCAAATCCATTCCCAAACCGAAGAAGAG
AGCTCCTAAGCGAGCTAAGACGGTGAAAGAAGAGCCGGTGACAGTGGTCGAGGAAGGGGAAAAGCATGTTG
CGAGGTTTCTAGACGAGCCAATTCCAGAATCTGAAGCGAAGAGTACCTGGCCTGACAGATACAAACCGATT
GAGGTACAGCCACCTAAGGCTTCGTCAAGAAAGAAGACGAAGGATGACGAAAAAGTTGAGATCATTCGTGC
TCGATGCCATTATAGACGTGCGATTGTTGATGAGCGTCAGATATATGAGCTGAATGATGATGCTTATGTAC
AGTCTGGTGAGGGAAAGGATCCCTTCATTTGTAAAATCATTGAAATGTTTGAAGGGGCTAATGGAAACTG
TATTTCACGGCTCGGTGGTTTTATAGACCTTCTGATACTGTAATGAAAGAGTTCGAGATTCTGATCAAGAA
AAAGCGTGTGTTTTTCTCTGAGATACAAGATACAAATGAATTGGGATTACTTGAAAAGAAGCTGAACATTT
TGATGATTCCCTTGAATGAAAATACTAAAGAGACTATCCCTGCAACAGAAAACTGTGACTTTTTCTGTGAC
ATGAACTATTTCTTGCCTTACGATACATTTGAAGCTATACAACAAGAAACCATGATGGCTATAAGTGAAAG
TTCAACAATATCCAGTGATACTGATATAAGAGAAGGAGCTGCTGCCATATCAGAGATTGGAGAATGTTCTC
AAGAAACAGAAGGTCACAAAAAGGCAACTTTGCTTGACCTTTACTCCGGCTGTGGAGCTATGTCGACAGGG
TTGTGCATGGGTGCACAACTGTCTGGTTTGAACCTCGTCACTAAATGGGCTGTTGACATGAATGCACATGC
ATGTAAAAGCTTGCAGCATAACCACCCAGAGACAAACGTGAGAAACATGACCGCAGAAGATTTCTTGTTTC
TGCTTAAGGAGTGGGAGAAGCTATGCATTCATTTCTCTTTGAGAAATAGTCCAAATTCAGAAGAATATGCC
AACCTTCACGGTTTGAATAATGTTGAGGACAATGAAGATGTCAGCGAGGAGAGTGAAAATGAAGATGATGG
AGAAGTTTTTACTGTTGACAAGATTGTTGGTATTTCCTTCGGAGTCCCTAAAAAGTTATTGAAACGTGGAC
TTTATTTGAAGGTAAGGTGGCTGAATTATGATGATTCTCATGATACATGGGAGCCTATTGAAGGACTCAGT
AATTGCCGGGGTAAAATTGAAGAGTTCGTTAAACTTGGATATAAATCTGGCATCCTTCCGTTACCAGGAGG
TGTTGATGTTGTCTGCGGTGGGCCACCATGCCAAGGAATCAGTGGTCACAACCGCTTCAGGAACTTATTGG
ACCCTCTAGAAGATCAGAAAAACAAGCAGCTTTTGGTGTATATGAACATTGTAGAATATTTGAAGCCTAAG
TTCGTTTTGATGGAAAACGTCGTTGACATGCTGAAGATGGCTAAGGGCTATCTTGCACGGTTTGCTGTTGG
ACGCCTTCTACAGATGAATTACCAAGTGAGGAATGGAATGATGGCAGCTGGAGCTTATGGCTTGCTCAGT
TTCGTTTGAGGTTCTTTCTATGGGGTGCACTCCCTAGTGAGATAATTCCGCAGTTCCCACTTCCAACACAT
GATCTAGTTCATAGAGGAAATATTGTCAAGGAGTTTCAGGGAAACATAGTAGCCTATGATGAAGGACATAC
TGTGAAGTTAGCAGACAAGCTTTTGTTGAAGGATGTGATTTCTGATCTTCCTGCAGTTGCCAACAGTGAAA
AAAGAGACGAGATTACATATGACAAAGATCCCACAACGCCATTTCAAAAGTTCATCAGATTGAGAAAGGAT
GAAGCGTCAGGTTCACAATCAAAGTCCAAGTCCAAAAAGCATGTCTTATATGATCATCACCCTCTTAATCT
TAATATAAATGACTATGAACGGGTTTGTCAGGTCCCCAAGAGAAAGGGAGCGAATTTAGGGACTTTCCTG
GTGTTATTGTTGGACCTGGTAATGTAGTCAAGTTGGAAGAGGGAAAGGAAAGGTCAAACTTGAATCTGGA
AAAACATTGGTTCCCGATTATGCCTTAACATATGTCGATGGGAAATCATGCAAACCTTTTGGTCGTCTTTG
GTGGGACGAAATTGTCCCCACTGTTGTCACACGGGCAGAACCCCACAACCAGGTGATCATTCATCCAGAGC
AAAATCGGGTTTTATCCATTCGAGAAAATGCGAGACTCCAAGGCTTTCCTGATGACTACAAACTCTTTGGC
CCACCCAAACAGAAGTACATTCAAGTAGGTAACGCTGTAGCTGTGCCAGTAGCGAAGGCCCTTGGATATGC
TTTGGGAACAGCTTTCCAGGGACTCGCAGTTGGAAAGATCCACTTCTTACTCTGCCTGAAGGTTTTGCAT
TCATGAAGCCAACTCTTCCTTCCGAGCTTGCATGA
- The *Arabidopsis* CMT3 protein sequence: 
MAPKRKRPATKDDTTKSIPKPKKRAPKRAKTVKEEPVTVVEEGEKHVARFLDEPIPESEAKSTWPDRYKPI
EVQPPKASSRKKTKDDEKVEIIRARCHYRRAIVDERQIYELNDDAYVQSGEGKDPFICKIIEMFEGANGKL
YFTARWFYRPSDTVMKEFEILIKKKRVFFSEIQDTNELGLLEKKLNILMIPLNENTKETIPATENCDFFCD
MNYFLPYDTFEAIQQETMMAISESSTISSDTDIREGAAAISEIGECSQETEGHKKATLLDLYSGCGAMSTG
LCMGAQLSGLNLVTKWAVDMNAHACKSLQHNHPETNVRNMTAEDFLFLLKEWEKLCIHFSLRNSPNSEEYA
NLHGLNNVEDNEDVSEESENEDDGEVFTVDKIVGISFGVPKKLLKRGLYLKVRWLNYDDSHDTWEPIEGLS
NCRGKIEEFVKLGYKSGILPLPGGVDVVCGGPPCQGISGHNRFRNLLDPLEDQKNKQLLVYMNIVEYLKPK
FVLMENVVDMLKMAKGYLARFAVGRLLQMNYQVRNGMMAAGAYGLAQFRLRFFLWGALPSEIIPQFPLPTH
DLVHRGNIVKEFQGNIVAYDEGHTVKLADKLLLKDVISDLPAVANSEKRDEITYDKDPTTPFQKFIRLRKD
EASGSQSKSKSKKHVLYDHHPLNLNINDYERVCQVPKRKGANFRDFPGVIVGPGNVVKLEEGKERVKLESG
KTLVPDYALTYVDGKSCKPFGRLWWDEIVPTVVTRAEPHNQVIIHPEQNRVLSIRENARLQGFPDDYKLFG
PPKQKYIQVGNAVAVPVAKALGYALGTAFQGLAVGKDPLLTLPEGFAFMKPTLPSELA
- The *Arabidopsis* MET1 (At5g49160) coding sequence: 
ATGGTGGAAAATGGGGCTAAAGCTGCGAAGCGAAAGAAGAGACCACTTCCAGAGATTCAAGAGGTAGAAGA
TGTACCTAGGACGAGGAGACCAAGGCGTGCTGCAGCGTGTACCAGTTTCAAGGAGAAATCTATTCGAGTCT
GTGAGAAATCTGCTACTATTGAAGTAAAGAAACAGCAGATTGTGGAGGAAGAGTTTCTCGCGTTACGGTTA
ACGGCTCTGGAAACTGATGTTGAAGATCGTCCAACCAGGAGACTGAATGATTTTGTTTTGTTTGATTCAGA
TGGAGTTCCACAACCTCTGGAGATGTTGGAGATTCATGACATATTCGTTTCAGGTGCTATCTTACCTTCAG
ATGTGTGTACTGATAAGGAGAAAGAGAAGGGTGTGAGGTGTACATCGTTTGGACGGGTTGAGCATTGGAGT
ATCTCTGGTTATGAAGATGGTTCCCCTGTTATTTGGATCTCAACGGAATTGGCGGATTATGATTGTCGTAA
ACCTGCTGCTAGCTACAGGAAGGTTTATGATTACTTCTATGAGAAAGCTCGTGCTTCAGTGGCTGTGTATA
 
AGAAATTGTCCAAGTCATCTGGTGGGGATCCTGATATAGGTCTTGAGGAGTTACTTGCGGCGGTTGTCAGA
TCAATGAGCAGTGGAAGCAAGTACTTTTCTAGTGGTGCGGCAATCATCGATTTTGTTATATCCCAGGGAGA
TTTTATATATAACCAACTCGCTGGTTTGGATGAGACAGCCAAGAAACATGAATCAAGCTATGTTGAGATTC
CTGTTCTTGTAGCTCTCAGAGAGAAGAGTAGTAAGATTGACAAGCCTCTGCAGAGGGAAAGAAACCCATCT
AATGGTGTGAGGATTAAAGAAGTTTCTCAAGTTGCGGAGAGCGAGGCCTTGACATCTGATCAACTGGTTGA
TGGTACTGATGATGACAGAAGATATGCTATACTCTTACAAGACGAAGAGAATAGGAAATCTATGCAACAGC
CCAGAAAAAACAGCAGCTCAGGTTCTGCTTCAAATATGTTCTACATTAAGATAAATGAAGATGAGATTGCC
AATGATTATCCTCTCCCATCGTACTATAAGACCTCCGAAGAAGAAACAGATGAACTTATACTTTATGATGC
TTCCTATGAGGTTCAATCTGAACACCTGCCTCACAGGATGCTTCACAACTGGGCTCTTTATAACTCTGATT
TACGATTCATATCACTGGAACTTCTACCGATGAAACAATGTGATGATATTGATGTCAACATTTTTGGGTCA
GGTGTGGTGACTGATGATAATGGAAGTTGGATTTCTTTAAACGATCCTGACAGCGGTTCTCAGTCACACGA
TCCTGATGGGATGTGCATATTCCTCAGTCAAATTAAAGAATGGATGATTGAGTTTGGAGCGATGATATTA
TCTCCATTTCTATACGAACAGATGTGGCCTGGTACCGTCTTGGGAAACCATCAAAACTTTATGCCCCTTGG
TGGAAACCTGTTCTGAAAACAGCAAGGGTTGGGATAAGCATTCTTACTTTTCTTAGGGTGGAAAGTAGGGT
TGCTAGGCTTTCATTTGCAGATGTCACAAAAAGACTGTCTGGGTTACAGGCGAATGATAAAGCTTACATTT
CTTCTGACCCCTTGGCTGTTGAGAGATATTTGGTCGTCCATGGGCAAATTATTTTACAGCTTTTTGCAGTT
TATCCGGACGACAATGTCAAAAGGTGTCCATTTGTTGTTGGTCTTGCAAGCAAATTGGAGGATAGGCACCA
CACAAAATGGATCATCAAGAAGAAGAAAATTTCGCTGAAGGAACTGAATCTGAATCCAAGGGCAGGCATGG
CACCAGTAGCATCGAAGAGGAAAGCTATGCAAGCAACAACAACTCGCCTGGTCAACAGAATTTGGGGAGAG
TTTTACTCCAATTACTCTCCAGAGGATCCATTGCAGGCGACTGCTGCAGAAAATGGGGAGGATGAGGTGGA
AGAGGAAGGCGGAAATGGGGAGGAAGAGGTTGAAGAGGAAGGTGAAAATGGTCTCACAGAGGACACTGTAC
CAGAACCTGTTGAGGTTCAGAAGCCTCATACTCCTAAGAAAATCCGAGGCAGTTCTGGAAAAAGGGAAATA
AAATGGGATGGTGAGAGTCTAGGAAAAACTTCTGCTGGCGAGCCTCTCTATCAACAAGCCCTTGTTGGAGG
GGAAATGGTGGCTGTAGGTGGCGCTGTCACCTTGGAAGTTGATGATCCAGATGAAATGCCGGCCATCTATT
TTGTGGAGTACATGTTCGAAAGTACAGATCACTGCAAAATGTTACATGGTAGATTCTTACAAAGAGGATCT
ATGACTGTTCTGGGGAATGCTGCTAACGAGAGGGAACTATTCCTGACTAATGAATGCATGACTACACAGCT
CAAGGACATTAAAGGAGTAGCCAGTTTTGAGATTCGATCAAGGCCATGGGGCATCAGTATAGGAAAAAGA
ACATCACTGCGGATAAGCTTGACTGGGCTAGAGCATTAGAAAGAAAAGTAAAAGATTTGCCAACAGAGTAT
TACTGCAAAAGCTTGTACTCACCTGAGAGAGGGGGATTCTTTAGTCTTCCACTAAGTGATATTGGTCGCAG
TTCTGGGTTCTGCACTTCATGTAAGATAAGGGAGGATGAAGAGAAGAGGTCTACAATTAAACTAAATGTTT
CAAAGACAGGCTTTTTCATCAATGGGATTGAGTATTCTGTTGAGGATTTTGTCTATGTCAACCCTGACTCT
ATTGGTGGGTTGAAGGAGGGTAGTAAAACTTCTTTTAAGTCTGGGCGAAACATTGGGTTAAGAGCGTATGT
TGTTTGCCAATTGCTGGAAATTGTTCCAAAGGAATCTAGAAAGGCTGATTTGGGTTCCTTTGATGTTAAAG
TGAGAAGGTTTTATAGGCCTGAGGATGTTTCTGCAGAGAAGGCCTATGCTTCAGACATCCAAGAATTGTAT
TTCAGCCAGGACACAGTTGTTCTCCCTCCAGGTGCTCTAGAGGGAAAATGTGAAGTAAGAAAGAAAAGTGA
TATGCCCTTATCCCGTGAATATCCAATATCAGACCATATTTCTTCTGTGATCTTTTCTTTGACACCTCCA
AAGGTTCTCTCAAGCAGCTGCCCGCCAATATGAAGCCAAAGTTCTCTACTATTAAGGACGACACACTTTTA
AGAAAGAAAAAGGGAAAGGGAGTAGAGAGTGAAATTGAGTCTGAGATTGTCAAGCCTGTTGAGCCACCTAA
AGAGATTCGTCTGGCTACTCTAGATATTTTTGCTGGTTGTGGTGGCCTGTCTCATGGACTGAAAAAGGCGG
GTGTATCTGATGCAAAGTGGGCGATTGAGTATGAAGAGCCAGCTGGGCAGGCTTTTAAACAAAACCATCCT
GAGTCAACAGTTTTTGTTGACAACTGCAATGTGATTCTTAGGGCTATAATGGAGAAAGGTGGAGATCAAGA
TGATTGTGTCTCTACTACAGAGGCAAATGAATTAGCAGCTAAACTAACTGAGGAGCAGAAGAGTACTCTGC
CACTGCCTGGTCAAGTGGACTTCATCAATGGTGGACCTCCATGTCAGGGATTTTCTGGTATGAACAGGTTC
AACCAAAGCTCTTGGAGTAAAGTTCAGTGTGAAATGATATTAGCATTCTTGTCCTTTGCTGACTATTTCCG
GCCAAGGTATTTTCTTCTGGAGAACGTGAGGACCTTTGTGTCATTCAATAAAGGGCAGACATTTCAGCTTA
CTTTGGCTTCCCTTCTCGAAATGGGTTACCAGGTGAGATTTGGAATCCTGGAGGCCGGTGCATATGGAGTA
TCCCAATCTCGTAAACGAGCTTTCATTTGGGCTGCTGCACCAGAAGAAGTTCTCCCTGAATGGCCTGAGCC
GATGCATGTCTTTGGTGTTCCAAAGTTGAAAATCTCACTATCTCAAGGTTTACATTATGCTGCTGTTCGTA
GTACTGCACTTGGTGCCCCTTTCCGTCCAATCACCGTGAGAGACACAATTGGTGATCTTCCATCAGTAGAA
AACGGAGACTCTAGGACAAACAAAGAGTATAAAGAGGTTGCAGTCTCGTGGTTCCAAAAGGAGATAAGAGG
AAACACGATTGCTCTCACTGATCATATCTGCAAGGCTATGAATGAGCTTAACCTCATTCGATGCAAATTAA
TCCCAACTAGGCCTGGGGCTGATTGGCATGACTTGCCAAAGAGAAAGGTTACGTTATCTGATGGGCGCGTA
GAAGAAATGATTCCTTTTTGTCTCCCAAACACAGCTGAGCGCCACAACGGTTGGAAGGGACTATATGGGAG
ATTAGATTGGCAAGGAAACTTTCCGACTTCCGTCACGGATCCTCAGCCCATGGGTAAGGTTGGAATGTGCT
TTCATCCTGAACAGCACAGAATCCTTACAGTCCGTGAATGCGCCCGATCTCAGGGGTTTCCGGATAGCTAC
GAGTTTGCAGGGAACATAAATCACAAGCACAGGCAGATTGGGAATGCAGTCCCTCCACCATTGGCATTTGC
TCTAGGTCGTAAGCTCAAAGAAGCCCTACATCTCAAGAAGTCTCCTCAACACCAACCCTAG
- The *Arabidopsis* MET1 protein sequence: 
MVENGAKAAKRKKRPLPEIQEVEDVPRTRRPRRAAACTSFKEKSIRVCEKSATIEVKKQQIVEEEFLALRL
TALETDVEDRPTRRLNDFVLFDSDGVPQPLEMLEIHDIFVSGAILPSDVCTDKEKEKGVRCTSFGRVEHWS
ISGYEDGSPVIWISTELADYDCRKPAASYRKVYDYFYEKARASVAVYKKLSKSSGGDPDIGLEELLAAVVR
 
SMSSGSKYFSSGAAIIDFVISQGDFIYNQLAGLDETAKKHESSYVEIPVLVALREKSSKIDKPLQRERNPS
NGVRIKEVSQVAESEALTSDQLVDGTDDDRRYAILLQDEENRKSMQQPRKNSSSGSASNMFYIKINEDEIA
NDYPLPSYYKTSEEETDELILYDASYEVQSEHLPHRMLHNWALYNSDLRFISLELLPMKQCDDIDVNIFGS
GVVTDDNGSWISLNDPDSGSQSHDPDGMCIFLSQIKEWMIEFGSDDIISISIRTDVAWYRLGKPSKLYAPW
WKPVLKTARVGISILTFLRVESRVARLSFADVTKRLSGLQANDKAYISSDPLAVERYLVVHGQIILQLFAV
YPDDNVKRCPFVVGLASKLEDRHHTKWIIKKKKISLKELNLNPRAGMAPVASKRKAMQATTTRLVNRIWGE
FYSNYSPEDPLQATAAENGEDEVEEEGGNGEEEVEEEGENGLTEDTVPEPVEVQKPHTPKKIRGSSGKREI
KWDGESLGKTSAGEPLYQQALVGGEMVAVGGAVTLEVDDPDEMPAIYFVEYMFESTDHCKMLHGRFLQRGS
MTVLGNAANERELFLTNECMTTQLKDIKGVASFEIRSRPWGHQYRKKNITADKLDWARALERKVKDLPTEY
YCKSLYSPERGGFFSLPLSDIGRSSGFCTSCKIREDEEKRSTIKLNVSKTGFFINGIEYSVEDFVYVNPDS
IGGLKEGSKTSFKSGRNIGLRAYVVCQLLEIVPKESRKADLGSFDVKVRRFYRPEDVSAEKAYASDIQELY
FSQDTVVLPPGALEGKCEVRKKSDMPLSREYPISDHIFFCDLFFDTSKGSLKQLPANMKPKFSTIKDDTLL
RKKKGKGVESEIESEIVKPVEPPKEIRLATLDIFAGCGGLSHGLKKAGVSDAKWAIEYEEPAGQAFKQNHP
ESTVFVDNCNVILRAIMEKGGDQDDCVSTTEANELAAKLTEEQKSTLPLPGQVDFINGGPPCQGFSGMNRF
NQSSWSKVQCEMILAFLSFADYFRPRYFLLENVRTFVSFNKGQTFQLTLASLLEMGYQVRFGILEAGAYGV
SQSRKRAFIWAAAPEEVLPEWPEPMHVFGVPKLKISLSQGLHYAAVRSTALGAPFRPITVRDTIGDLPSVE
NGDSRTNKEYKEVAVSWFQKEIRGNTIALTDHICKAMNELNLIRCKLIPTRPGADWHDLPKRKVTLSDGRV
EEMIPFCLPNTAERHNGWKGLYGRLDWQGNFPTSVTDPQPMGKVGMCFHPEQHRILTVRECARSQGFPDSY
EFAGNINHKHRQIGNAVPPPLAFALGRKLKEALHLKKSPQHQP
- The *Arabidopsis* DDM1 (At5g66750) coding sequence: 
ATGGTTAGTCTGCGCTCCAGAAAAGTTATTCCGGCTTCGGAAATGGTCAGCGACGGAAAACGGAGAAAGA
TGCGTCTGGTGATTCACCCACTTCTGTTCTCAACGAAGAGGAAAACTGTGAGGAGAAAAGTGTTACTGTTG
TAGAGGAAGAGATACTTCTAGCCAAAAATGGAGATTCTTCTCTTATTTCTGAAGCCATGGCTCAGGAGGAA
GAGCAGCTGCTCAAACTTCGGGAAGATGAAGAGAAAGCTAACAATGCTGGATCTGCTGTTGCTCCTAATCT
GAATGAAACTCAGTTTACTAAACTTGATGAGCTCTTGACGCAAACTCAGCTCTACTCTGAGTTTCTCCTTG
AGAAAATGGAGGATATCACAATTAATGGGATAGAAAGTGAGAGCCAAAAAGCTGAGCCCGAGAAGACTGGT
CGTGGACGCAAAAGAAAGGCTGCTTCTCAGTACAACAATACTAAGGCTAAGAGAGCGGTTGCTGCTATGAT
TTCAAGATCTAAAGAAGATGGTGAGACCATCAACTCAGATCTGACAGAGGAAGAAACAGTCATCAAACTGC
AGAATGAACTTTGTCCTCTTCTCACTGGTGGACAGTTAAAGTCTTATCAGCTTAAAGGTGTCAAATGGCTA
ATATCATTGTGGCAGAATGGTTTGAATGGAATATTAGCTGATCAAATGGGACTTGGAAAGACGATTCAAAC
GATCGGTTTCTTATCACATCTGAAAGGGAATGGGTTGGATGGTCCATATCTAGTCATTGCTCCACTGTCTA
CACTTTCAAATTGGTTCAATGAGATTGCTAGGTTCACGCCTTCCATCAATGCAATCATCTACCATGGGGAT
AAAAATCAAAGGGATGAGCTCAGGAGGAAGCACATGCCTAAAACTGTTGGTCCCAAGTTCCCTATAGTTAT
TACTTCTTATGAGGTTGCCATGAATGATGCTAAAAGAATTCTGCGGCACTATCCATGGAAATATGTTGTGA
TTGATGAGGGCCACAGGTTGAAAAACCACAAGTGTAAATTGTTGAGGGAACTAAAACACTTGAAGATGGAT
AACAAACTTCTGCTGACAGGAACACCTCTGCAAAATAATCTTTCTGAGCTTTGGTCTTTGTTAAATTTTAT
TCTGCCTGACATCTTTACATCACATGATGAATTTGAATCATGGTTTGATTTTTCTGAAAAGAACAAAAACG
AAGCAACCAAGGAAGAAGAAGAGAAAAGAAGAGCTCAAGTTGTTTCCAAACTTCATGGTATACTACGACCA
TTCATCCTTCGAAGAATGAAATGTGATGTTGAGCTCTCACTTCCACGGAAAAAGGAGATTATAATGTATGC
TACAATGACTGATCATCAGAAAAAGTTCCAGGAACATCTGGTGAATAACACGTTGGAAGCACATCTTGGAG
AGAATGCCATCCGAGGTCAAGGCTGGAAGGGAAAGCTTAACAACCTGGTCATTCAACTTCGAAAGAACTGC
AACCATCCTGACCTTCTCCAGGGGCAAATAGATGGTTCATATCTCTACCCTCCTGTTGAAGAGATTGTTGG
ACAGTGTGGTAAATTCCGCTTATTGGAGAGATTACTTGTTCGGTTATTTGCCAATAATCACAAAGTCCTTA
TCTTCTCCCAATGGACGAAACTTTTGGACATTATGGATTACTACTTCAGTGAGAAGGGGTTTGAGGTTTGC
AGAATCGATGGCAGTGTGAAGCTGGATGAAAGGAGAAGACAGATTAAAGATTTCAGTGATGAGAAGAGCAG
CTGTAGTATATTTCTCCTGAGTACCAGAGCTGGAGGACTCGGAATCAATCTTACTGCTGCTGATACATGCA
TCCTCTATGACAGCGACTGGAACCCTCAAATGGACTTGCAAGCCATGGACAGATGCCACAGAATCGGGCAG
ACGAAACCTGTTCATGTTTATAGGCTTTCCACGGCTCAGTCGATAGAGACCCGGGTTCTGAAACGAGCGTA
CAGTAAGCTCAAGCTGGAACATGTGGTTATTGGCCAAGGGCAGTTTCATCAAGAACGTGCCAAGTCTTCAA
CACCTTTAGAGGAAGAGGACATACTGGCGTTGCTTAAGGAAGATGAAACTGCTGAAGATAAGTTGATACAA
ACCGATATAAGCGATGCGGATCTTGACAGGTTACTTGACCGGAGTGACCTGACAATTACTGCACCGGGAGA
GACACAAGCTGCTGAAGCTTTTCCAGTGAAGGGTCCAGGTTGGGAAGTGGTCCTGCCTAGTTCGGGAGGAA
TGCTGTCTTCCCTGAACAGTTAG
- The *Arabidopsis* DDM1 protein sequence:

MVSLRSRKVIPASEMVSDGKTEKDASGDSPTSVLNEEENCEEKSVTVVEEEILLAKNGDSSLISEAMAQEE
EQLLKLREDEEKANNAGSAVAPNLNETQFTKLDELLTQTQLYSEFLLEKMEDITINGIESESQKAEPEKTG
RGRKRKAASQYNNTKAKRAVAAMISRSKEDGETINSDLTEEETVIKLQNELCPLLTGGQLKSYQLKGVKWL
ISLWQNGLNGILADQMGLGKTIQTIGFLSHLKGNGLDGPYLVIAPLSTLSNWFNEIARFTPSINAIIYHGD
KNQRDELRRKHMPKTVGPKFPIVITSYEVAMNDAKRILRHYPWKYVVIDEGHRLKNHKCKLLRELKHLKMD

NKLLLTGTPLQNNLSELWSLLNFILPDIFTSHDEFESWFDFSEKNKNEATKEEEEKRRAQVVSKLHGILRP
FILRRMKCDVELSLPRKKEIIMYATMTDHQKKFQEHLVNNTLEAHLGENAIRGQGWKGKLNNLVIQLRKNC
NHPDLLQGQIDGSYLYPPVEEIVGQCGKFRLLERLLVRLFANNHKVLIFSQWTKLLDIMDYYFSEKGFEVC
RIDGSVKLDERRRQIKDFSDEKSSCSIFLLSTRAGGLGINLTAADTCILYDSDWNPQMDLQAMDRCHRIGQ
TKPVHVYRLSTAQSIETRVLKRAYSKLKLEHVVIGQGQFHQERAKSSTPLEEEDILALLKEDETAEDKLIQ
TDISDADLDRLLDRSDLTITAPGETQAAEAFPVKGPGWEVVLPSSGGMLSSLNS

### Example 9

### Identification of Repressors for Methylases

Constructs reporting DRM1, DRM2, CMT3 and MET1 transcription are generated by coupling control sequences thereof to a reporter such as a fluorescent protein. These transgenic lines are further mutagenized and candidate repressor genes are isolated by map-based cloning. Such repressors of DNA-methyltransferase transcription are then expressed under a strong 35S promoter or pathogen-inducible promoters (*e.g*., WRKY6 or PR1) and stable transgenic lines generated to confer enhanced disease resistance to pathogens. By constitutively enhancing the expression of repressors of DNA-methyltransferase transcription increased resistance to bacterial and fungal pathogens is achieved in a variety of plants, including crops. The positive regulators of DCL4 transcription, obtained as described above, are further overexpressed, conditionally or constitutively, in these transgenic lines to confer, additionally, enhanced resistance to virulent viruses.

Furthermore, the same transgenic lines reporting transcriptional activities of DNA-methyltransferases are used to screen for chemical compounds that trigger down-regulation of GFP mRNA, as described above. Molecules that repress GFP mRNA levels are further used to confer antibacterial and antifungal resistance in a variety of plant species including crops. Cocktails of chemical agents that promote DCL4 transcription (see Example 2) and inhibit transcription of DNA-methyltransferases will be used to confer broad spectrum resistance to unrelated pathogens.

Sequences from DRM1, DRM2, CMT3 and MET1 predicted promoters are:
DRM1 promoter sequence: 
AGCTATGTAATTTAATAGAATTTGGGTTGTACATAACTACATATGTTCAAGTATGAAGAAATAGATATAAA
ATCAAGCATGAAAGACAACACAAATGTTAAATGAGCAAAACCAAGAAGGCAAGAACAAATATAGGGCCTTC
GTGGAAACCTTTTGTGCGACATATGGAAACCCATTAGGCTAGCGATGTAGTTGGCCCAAGAAACCGGCTTT
GACTCAGAAGATATAGTTATTGATTTTCGGCTTCGTCAATCAACAACACTGTAATTGTAATGACAATAGTT
GGTGCCGACAAAAAATAATAATGACAATAGTTGGGCTTAGGTTTATAAGTTCATTTTTCTAAAAGTTAATT
GGTGAAAATCAATTGCAAACAATATATTACTCTCTTTTCTTAGTAGTCTTCTATATAAGATTCTGTTTGAT
CATGAGATAAAAATAAAAATAAATACTCTTTTTAATCTGTGGGTAAAAGGTAAAAGAGACATGTTATGGTT
GGATCTGACGGCCCACGTGTCGCTCGCACTCCGATCTCTTTTCACTTTTGGTCCCAGTAAGGCTGTCCGTA
TGGAGACATCTTCCCATGCCTTTGGACATTTGTGAAAACAAGATATTATTATTAGAACAACTGAACAAGAT
ATTGCAAGTGTTACTTTTATTTAATTTCACTGTGGTAAGATAAAATTTGAAAATTTACTTGTTGCTCTGAT
CTTGATGCAAGTAACCTCAAGTTTTGCCCATTCTTGGAGAATGTAAATATAACTTCGATCCCCAAAATGTG
CCTCCTGTCATGTTGGAATAACTGGTCAGATTTTCAAAAGGTGACCATTTGTCTGTCCATAATCATCAATC
CCTTATATTCTATTCCACTTCTTAAAGTTTTTGTTCTATTGTTAAAACGAGTTGGTTTGGTTTGGATCATT
TGAAATGAATGGGTGAATGCATGAATTCTAAGAGTTTGTCATGATACTTAGGCTTCACATAAAATTCTACA
TATGGTTAAGAAGAAATTAGGTATTCTGAATTTGACGATATTTCAATAATTACCAATTTGTTACCTTGTGA
TAATTTCACGAAGCTCGAGGCTAGAATACTTTATTTTATAGGTCCCACTTCAATGACTCATCATCCTTATC
TAGATTTGTGTCACATTCCATCTAGCACTTTTTTTTATTTGCACACCCTCCCCACTCCTTTTCTTTTGTGA
TCCTAAAATTAAGTTCAAAAATTATTTTAATTTTGGAATCTTCAGATTATAAGAAGAAAAAAAACATTGAA
TCTTACATAAATACTTAAGTAGATTTGGGATTACCGGATTAGTAGTGACAAAATTAACTAAGAAATATTAT
TCAATAATAAAACAACCAGTAAAATAAAGTCACCAAACTTTTTAAATGGCGTGGCCGGTAGTGAAAAAACA
 
AGAAAAAAATTAATAATGTAAATAAAAATCAAGATATTTTGATAAGGTGTCTATAAAAGTCATATGCCACC
ACCAAAAGT
 DRM2 promoter sequence: 
AGTTATATATTACCAATCTTTGGCTTGTCCAACTTTTGGTTAGCCTCTATTTCCAGGTGAGAGTGGAGTTG
ACCAGCTAGTTGAGATAATAAAGGTACTTCAATTTGGTTAAACAAACACACATAATCCTAGCCATTGCTAT
ATTGAACATAGAGTGGATCATTGATTATATGGAATGAAGAGGCTCCATTTCCTGCTATTGATTGCCATCAT
TTTGTTTACTGTGTGTGTTACAGGTTCTTGGAACACCAACACGGGAGGAAATCAAATGCATGAATCCAAAC
TACACAGAATTCAAATTCCCGCAAATAAAGGCTCATCCTTGGCACAAAGTAAGCAAACACATCATCAGTTT
TTCCTTAACATTGATCTCCATATATTCTTACGATTGAAAAATCTGTTGTTGGTTCTTAAGATATTCCATAA
GCGTACACCTCCAGAAGCTGTAGACCTTGTCTCAAGACTTCTCCAGTATTCTCCAAACCTCAGATCAACCG
CTGTAAGTCAATGAAGTGATTACCATAATAACATTATGTTTGATATATCTGGTTGGTTGATTCAAACTTAC
TTGTTATTGTAGATGGAGGCGATAGTTCACCCGTTCTTCGATGAGCTACGTGATCCCAATACACGTCTTCC
TAATGGTCGTGCCTTGCCTCCTCTCTTCAACTTTAAACCTCAAGGTCTGTCTCCTCCAAATATGCTTTTGT
TTGTTTCCCAATGCTCCGTTTTAACAAAGACTAAAAGTGTGTGCTTCTTGTTAAATATGTAGAGCTAAAAG
GAGCAAGTTTAGAGTTGTTGTCCAAGCTTATACCTGACCACGCCCGAAAACAATGTTCCTTCCTCGCTCTC
TAAATCTCTTCCTCTCTCTCTATATATATGTGTGTGTGTGTGTATGTACACATGCATATAATATGCTTATC
GTTTCTAAGTAATGGAGATAGCTTCTCAGGATTATCATTAGCTTTCATCTTTCATGTATCTTTGTTGTTTA
TTGTCTTATCACAACCTTTGTACTTTATTACATACAATGATTAGTGTAATGTATGTGACGGTCTTTGACTC
GCCGGTCGCTACAGTTATGTTGGATACTAAATTATAAAATAAACTTCTCGCTCGTCACGTGTCATTGCATG
CATCCAAAGCTCATGCTTCAAAGCTTAGCCAAACTTATTTTTAAAAAAGCCTATGTTCTGTGTAAAAGTGT
CATTTACGAGAGTTTCTTGTTTAAGTTTAACCAATTTCACTTCCTCAAACGAAATACGGTAATTGGTAATA
TCCTCTAAACATGAATTATCATTGACTATAAAAATTAGTTTCGCAAATTGCCTCTAAGCACCACAAGTGTC
ACGTAACGTGTCATTTATTCATGTTTATTGATTTAGTTAATTAAAAACATTGTAGTTTAATTATTGAAGTA
GTACAAAGAATAGGGACTAAATTGCAATACTCTGAATTTGTTTTTTCTTTTTTAGAATCATCCGACTTTT
TGTTTCACG
 CMT3 promoter sequence: 
ACATAGTGGACCCATGACAAGAAATAAGGCCCAAAAGTTGGCTAATTTCAGCCATCACGACAAAGGCTAC
TTCTCATTTCGACATCCATATTATTCTATTCTTGTACACTTCTCTTTCATTCACTCGTCATTAATATGCCT
TCAATCTAATATATTTAAGGACACAATTATACACACGTCCATATATAAACTTATATTGTTGCCTTGTTGGA
TATATATAAAAGTTATTGTAATTAGTAGTTACTAGTTAGAGTAATTTTCAGGTCTAATAGTGATGAGATTA
ACGTATTTTCTTCTTTAAATTTCAGTTATTTGCAAATAATGTCACTGCTCGATTTCCTGGAATAAAGGAGC
TAATAAGAGTATCATGTTCTCCTTCATGTTACCATTTCACGTTTGCACGTTTTTTTTTTTTTTATTATGAA
CAAGATTACGATCACACGTTCGGTTTGTTGTTTAGTCGTTCCAAGTGTATAGGAGTTTTATCCACACAAAA
AAAATTTTGATAGGAATGTGTAATTCCATAAAGATTTCCATCGTTACACACTAAAGTTTATCTTACGCGC
CACGCCGTTTGTGGAGTAACCGAGTAACTAATTTACTACTAGTCTTGATGTTGGTAGGATTAAATAAAAGA
ATCATGGAGATAGGATACGATTGTGTTTGAATAATTCAATATGTTTATATCTGGAAAGATCTACAAATTTG
ATATGATCACTAAGATTGTGGGAATTTATTCAAATCCAAAACACATAGTTACAAATCATATACAAATATGG
AAAATAAATAATGTCTAATAACATTTGTGTAACATCATCTTTAATGCGAGTCATTTAGAAATGTAACTTTT
TTTCTTTCTGATATGTTTATTTCTGGAAATATATACAAAATTGACCTGATCACTAAGATTGTGGGAATAAT
TCCAAACCAATATAGAAAGTGATAATTTATTAAAAACTGAACATTTGTGTAATCTTTTTTTTTTTTTTTT
AATGTAATGTCAAATTATATTCAAAAAAAACATATACTTTTTTACAACTTGTGCTTCATGTGTAAGAAAGG
GAGAGTGCTAAAAAAAAATTCTGAAATTGTCCAAAAATGATTAGATCTTTCGTAGAGTCGATGTTGACTACC
CCGGGAATGAACCCATTTGTGTAATCTTTTAACATTTATTAAAAACAAATCATAGATAGCTAAGATTGTGG
GAAGAATTCCAAACGAAATTAAATTCACACTAACACATTTACTAAAATCATAGATAGATATAGAAAGAAAA
AACTTTTCTACTAATTTTTTTGACATTTGTGTACTCATTATAGCGAGAGAAAGAGATGGGCCTCAAAACTT
TTATTAGGCCCAAACGTTTTAAATTCATTATAAAAATAAAACAATTCCACGAAAATTTCGAAACCCATCAC
TTTGGCGCTTATGTGACGCGCTTATTTCGCCCTCAATCTCAGATTATTTAGTCCTCACACTCGTCACACCC
CCGCTTCCT
 MET1 promoter sequence:

CTAAAAAGAGTATTCAAAAACCCAAACATTAATTTAATATCCAAAATATTAATTATATGATATTATTTTAT
TTGATTTTAAATATATAGTAAACTGCGAGTTGTATATGTTTTCTTGATATTATTTATATTGTTTAGTGTTT
AAAATTATACACTTGTATTTTGATTGTTAATTTTAGAGTTTCACCTGTAGTATACCATCTTATATTAATAT
CGATTTAAACCCGTCAATTCTAGGATTTTCCAGCTTGTATTAAAAATTGAATCACATCATACACATAAAAA
AATCTAATATGTTATTAATTATTGTTGTATATAAGATTATAAATTCTTAAAATAATATGCATGAAATTGAA
TATAAATATTTAAATTATGACCCAGTACTTAGTAATAAATTTTTTTAAATCTATTTTTGACCCGTTATAAT
ATTTTTTTATGTATTGAACAGTTTATATTCGTTTTTAAAAGTTTAAATTATGGCATATGCGAAAAAACTCT
AATTATTTTTTTATAACGATGATATTATTTTTTCGCAAAAATAGAATCATATAAAGATGAGAGGTGAACTA
TAATAATTAATAAAAAAATTAATATGATAATTTAGATATTAAATCTAATTTGTTGATTTTAATTGGTTAATT

TTTTGGAAATTAATAATGTATTTCATTTTTAATGAAATTTAATTAATTAAATTAGTATTTGACTTTTTAA
TTTTTAAAGAGATGAATTACTTTACTCTTTAAATTTTATTTCTAATGGCATACATATGTAATTACTTACAA
AAAGTAAGGTTACATTTAAAATGTACTTCCCAAATAATATAGTAGGATCATGGTAAAATGTTAGTTCTCGA
AAGAAAAAAATATTGTTATAAATCATAAACCTAACGAGCTAACTAAAATAGCGGCATCCTACCAATTTGAGA
TTTTTCGTATATATATTAAAATTATCCATTTGATAAACACTTCATGATAAAGTATTAGTTTTGAAAATAAA
AATATTGTTCTTGTTATAAGAAAAAACACACACATAAAAGTATTATTGGAGGATCTCATTGTTAAGTTGTT
AACCCTCAACATTTCGTCTAAAAATCAGACTTTTTTCTATCAAAAAAATATCTATACTTTGTAGTCAAATA
AAAATCTTAATCAAAATAATACTCGTATACTTTGACTGTTGACTGATGGAAAGATATTAGAATATAAACAT
TAGAGATAGAAACAAATCTGTAAAAATCTTAAAATTAGGATTATTTATACGGAATATTCCCCAAAAGATAA
AATCATTGAATCATAAAAAGCCATTTATGGTAGCCCTAAATATTCAGGCGCGGCTTTTTTCTTATTTCGT
TTTCATTTTAAAAAAGTTTTTAGCGCCGTTTATTGCCGCGCGTCGTTTTCGCTCCTCGTCTCGTCTCCTTT
TATTATTACCCCCTCTCTCTCTCTCCCACTCTTCCTCTCAAATCACACATCACTGCTTTCTTCAACCTCTC
TATCTCTCA

### Example 10

### The DNA-Demethylase ROS1 Positively Regulate Plant Resistance to Pathogens

The role of DNA-glycosylases in resistance to pathogens was tested. *Arabidopsis* encodes four DNA-glycosylases, among which ROS1 and DEMETER (DME) are the most characterized. Both ROS1 and DME were recently shown to excise 5-methylcytosine *in vitro* when expressed in *E*. *coli.* These findings revealed that DNA-glycosylases encode active demethylases that could direct the possible active DNA demethylation of specific defense-related genes discussed above. We challenged single DNA glycosylase mutants with virulent Pto DC3000. Only the *ros1-4* single mutant was more susceptible to this pathogen as revealed by enhanced bacterial growth and disease symptoms. Five week-old Col-0, La-er, *dml2-1, dml3-1, ros1-4* and *dme* mutant plants were syringe inoculated with Pto DC3000 at a concentration of 10⁵ cfu/ml and bacterial growth measured 4 dpi. The *ros1* mutant plants display more pronounced bacterial disease symptoms. Five week-old Col-0 and ros1-4 mutant plants were inoculated and pictures taken 4 dpi. Additionally, we found that induction of the SA-defense marker gene *PR1* was delayed in the *ros1-4-* as compared to Col-0-infected plants. Five week-old Col-0 and *ros1-4* mutant plants were syringe infiltrated with Pto DC3000 at a concentration of 2 x 10⁷ cfu/ml and PR1 mRNA levels analyzed over a 12 hour timecourse experiment by semi-quantitative RT-PCR analysis. These results suggest that ROS1 might demethylate defense-related genes to promote resistance to pathogens.

Therefor, constitutive or conditional overexpression of the *Arabidopsis* ROS1 protein is used to elevate resistance to pathogens. ROS1 coding sequence is expressed transgenically in plants using methods known by those skilled in the art using either constitutive promoters or, preferably, pathogen-responsive promoters that are known to be broadly responsive to bacterial, fungal and viral pathogens. Examples of such promoters include, but are not restricted to, WRKY6 and PR1. The method allows inducible, enhanced resistance, which is desirable because it is not, or is less, detrimental to plant development and physiology in non-infected conditions.

Accordingly, from this disclosure, those skilled in the art will appreciate that constructs are prepared according to this invention wherein, in one embodiment, a constitutive or pathogen responsive promoter (including but not limited to, for example, the WRKY6 promoter, the PR1 promoter and the like) is operatively linked to a nucleic acid sequence which encodes *Arabidopsis* ROS1 protein to confer enhance resistance to unrelated pathogens in various plant species, including crops.
- The *Arabidopsis* ROS1 (At2g36490) coding sequence is: 
ATGGAGAAACAGAGGAGAGAAGAAAGCAGCTTTCAACAACCTCCATGGATTCCTCAGACACCCATGAAGCC
ATTTTCACCGATCTGCCCATACACGGTGGAGGATCAATATCATAGCAGTCAATTGGAGGAAAGGAGATTTG
TTGGGAACAAGGATATGAGTGGTCTTGATCACTTGTCTTTTGGGGATTTGCTTGCTCTAGCTAACACTGCA
TCCCTCATATTCTCTGGTCAGACTCCAATACCTACAAGAAACACAGAGGTTATGCAAAAAGGTACTGAAGA
AGTGGAGAGTTTGAGCTCAGTGAGTAACAATGTTGCTGAACAGATCCTCAAGACTCCTGAAAAACCTAAGA
GGAAGAAGCATCGGCCAAAGGTTCGTAGAGAAGCTAAACCCAAGAGGGAGCCTAAACCACGAGCTCCGAGG
AAGTCTGTTGTCACCGATGGTCAAGAAAGCAAAACACCAAAGAGGAAATATGTGCGGAAGAAGGTTGAAGT
CAGTAAGGATCAAGACGCTACTCCGGTTGAATCATCAGCAGCTGTTGAAACTTCAACTCGTCCTAAGAGGC
TCTGTAGACGAGTCTTGGATTTTGAAGCCGAAAATGGAGAAAACCAGACCAACGGTGACATTAGAGAAGCA
GGTGAGATGGAATCAGCTCTTCAAGAGAAGCAGTTAGATTCTGGGAATCAAGAGTTAAAAGATTGCCTTCT
TTCGGCTCCTAGCACGCCCAAGAGAAAGCGCAGCCAAGGTAAAAGAAAGGGAGTTCAACCAAAGAAAAATG
GCAGTAATCTAGAAGAAGTCGATATTTCGATGGCGCAAGCTGCAAAGAGAAGACAAGGACCAACTTGTTGC
GACATGAATCTATCAGGGATTCAGTATGATGAGCAATGTGACTACCAGAAAATGCATTGGTTGTATTCCCC
AAACTTGCAACAGGGAGGGATGAGATATGATGCCATTTGCAGCAAAGTATTCTCTGGACAACAGCACAATT
ATGTTTCTGCCTTTCACGCTACGTGCTACAGTTCCACATCTCAGCTCAGTGCTAATAGAGTCCTAACCGTT
GAAGAAAGACGAGAAGGTATCTTTCAAGGAAGGCAAGAGTCTGAGCTAAATGTTCTCTCGGATAAGATAGA
CACGCCGATCAAGAAGAAAACAACAGGCCATGCTCGATTCCGGAATTTGTCTTCAATGAATAAACTTGTGG
AAGTTCCTGAGCATTTAACCTCAGGATATTGTAGCAAGCCACAGCAAAATAATAAGATTCTTGTTGATACG
CGGGTGACTGTGAGCAAAAAGAAGCCAACCAAGTCTGAGAAATCACAAACCAAACAGAAAAATCTTCTTCC
GAATCTTTGCCGTTTTCCACCTTCATTTACTGGTCTTTCTCCAGATGAACTTTGGAAACGACGTAACTCGA
TCGAAACAATCAGTGAGCTATTGCGTCTATTAGACATCAACAGGGAGCATTCTGAAACTGCTCTCGTTCCT
TACACAATGAATAGCCAGATTGTACTCTTTGGTGGTGGCGCTGGAGCAATTGTGCCTGTAACTCCTGTTAA
AAAACCACGCCCACGACCAAAGGTTGATCTAGACGATGAGACAGACAGAGTGTGGAAACTGCTATTGGAGA
ATATTAATAGCGAAGGTGTTGACGGATCAGACGAGCAGAAGGCGAAATGGTGGGAGGAAGAACGTAATGTG
TTTCGAGGACGAGCTGACTCATTTATTGCAAGGATGCACCTTGTACAAGGGGATCGACGTTTTACGCCTTG
GAAGGGATCCGTCGTGGATTCTGTTGTTGGAGTATTTCTCACTCAAAATGTTTCAGACCATCTCTCAAGTT
CGGCTTTCATGTCGTTGGCTTCCCAGTTCCCTGTCCCTTTTGTACCGAGCAGTAACTTTGACGCTGGAACA
AGCTCGATGCCTTCTATTCAAATAACGTACTTGGACTCAGAGGAAACGATGTCAAGCCCACCCGATCACAA
TCACAGTTCTGTTACTTTGAAAAATACACAGCCTGATGAGGAGAAGGATTATGTACCTAGCAATGAAACCT
CCAGAAGCAGTAGTGAGATTGCCATCTCAGCCCATGAATCAGTTGACAAAACCACGGATTCAAAGGAGTAT
GTTGATTCAGATCGAAAAGGCTCAAGTGTAGAGGTTGATAAGACGGATGAGAAGTGTCGTGTCCTGAACCT
GTTTCCATCTGAAGATTCTGCACTTACATGTCAACATTCGATGGTGTCTGATGCTCCTCAAAATACAGAGA
GAGCAGGATCAAGCTCAGAGATCGACTTAGAAGGAGAGTATCGTACTTCCTTTATGAAGCTCCTACAGGGG
GTACAAGTCTCTCTAGAAGATTCCAATCAAGTATCACCAAATATGTCTCCGGGTGATTGTAGCTCAGAAAT
TAAGGGTTTCCAGTCAATGAAAGAGCCCACAAAATCCTCTGTTGATAGTAGTGAACCTGGTTGTTGCTCTC
AGCAAGATGGGGATGTTTTGAGTTGTCAGAAACCTACCTTAAAAGAAAAAGGAAAAAAGGTTTTGAAGGAG
GAAAAAAAAGCGTTTGACTGGGATTGTTTAAGAAGAGAAGCCCAAGCTAGAGCAGGAATTAGAGAAAAAAC
AAGAAGTACAATGGACACCGTGGATTGGAAGGCAATACGAGCAGCAGATGTTAAGGAAGTTGCTGAAACAA
TCAAGAGTCGCGGGATGAACCATAAACTTGCAGAACGTATACAGGGCTTCCTTGATCGACTGGTAAATGAC
CATGGAAGTATCGATCTTGAATGGTTGAGAGATGTTCCACCAGATAAAGCAAAAGAATATCTTCTGAGCTT
TAACGGATTGGGACTGAAAAGTGTGGAGTGTGTGCGGCTTCTAACACTTCACCATCTTGCCTTTCCAGTTG
ATACAAATGTTGGGCGCATAGCCGTCAGACTTGGATGGGTGCCCCTTCAGCCGCTCCCAGAGTCACTTCAG
TTGCATCTTCTGGAAATGTATCCTATGCTTGAATCTATTCAAAAGTATCTTTGGCCCCGTCTCTGCAAACT
CGACCAAAAAACATTGTATGAGTTGCACTACCAGATGATTACTTTTGGAAAGGTCTTTTGCACAAAGAGCA
AACCTAATTGCAATGCATGTCCGATGAAAGGAGAATGCAGACATTTTGCCAGTGCGTTTGCAAGTGCAAGG
CTTGCTTTACCAAGTACAGAGAAAGGTATGGGGACACCTGATAAAAACCCTTTGCCTCTACACCTGCCAGA
GCCATTCCAGAGAGAGCAAGGGTCTGAAGTAGTACAGCACTCAGAACCAGCAAAAAAGGTCACATGTTGTG
AACCAATCATCGAAGAGCCTGCTTCACCGGAGCCAGAAACCGCAGAAGTATCAATAGCTGACATAGAGGAG
GCGTTTTTTGAGGATCCAGAAGAAATTCCTACCATCAGGCTAAACATGGATGCATTTACCAGTAACTTGAA
GAAGATAATGGAACACAACAAGGAACTTCAAGACGGAAACATGTCCAGCGCTTTAGTTGCACTTACTGCTG
AAACTGCTTCTCTTCCAATGCCTAAGCTCAAGAATATCAGCCAGTTAAGGACAGAACACCGAGTTTACGAA
CTTCCAGACGAGCATCCTCTTCTAGCTCAGTTGGAAAAGAGAGAACCTGATGATCCATGTTCTTATTTGCT
TGCTATATGGACGCCAGGTGAGACGGCTGATTCTATTCAACCGTCTGTTAGTACGTGCATATTCCAAGCAA
ATGGTATGCTTTGTGACGAGGAGACTTGTTTCTCCTGCAACAGCATCAAGGAGACTAGATCTCAAATTGTG
 
AGAGGGACAATTTTGATTCCTTGTAGAACAGCGATGAGGGGTAGTTTTCCTCTAAATGGAACGTACTTTCA
AGTAAATGAGGTGTTTGCGGATCATGCATCCAGCCTAAACCCAATCAATGTCCCAAGGGAATTGATATGGG
AATTACCTCGAAGAACGGTCTATTTTGGTACCTCTGTTCCTACGATATTCAAAGGTTTATCAACTGAGAAG
ATACAGGCTTGCTTTTGGAAAGGGTACGTATGTGTACGTGGATTTGATCGAAAGACGAGGGGACCGAAGCC
TTTGATTGCAAGATTGCACTTCCCGGCGAGCAAACTGAAGGGACAACAAGCTAACCTCGCCTAATCCGTTG
GCAAGCAAACAAATACAAGCTTATGGTTAAGAGTGAGAGAGCACACTGTTCCAATCTAGTTAATGTAAGAA
AGTGAAAACGTAAAGTTAACAGTCCTAGAGTTGTACAAGGTTTCTAAATCCCATTTTAGTTTCGTCTTAAA
TTTGTATCAAACACTTGTCACAAAAAACAGACCCGTAGCTGTGTAAACTCTCTGTTCCCTTCGTTTGGTTT
ATATCTGAATTTACGGTT
- The *Arabidopsis* ROS1 protein sequence is:

MEKQRREESSFQQPPWIPQTPMKPFSPICPYTVEDQYHSSQLEERRFVGNKDMSGLDHLSFGDLLALANTA
SLIFSGQTPIPTRNTEVMQKGTEEVESLSSVSNNVAEQILKTPEKPKRKKHRPKVRREAKPKREPKPRAPR
KSVVTDGQESKTPKRKYVRKKVEVSKDQDATPVESSAAVETSTRPKRLCRRVLDFEAENGENQTNGDIREA
GEMESALQEKQLDSGNQELKDCLLSAPSTPKRKRSQGKRKGVQPKKNGSNLEEVDISMAQAAKRRQGPTCC
DMNLSGIQYDEQCDYQKMHWLYSPNLQQGGMRYDAICSKVFSGQQHNYVSAFHATCYSSTSQLSANRVLTV
EERREGIFQGRQESELNVLSDKIDTPIKKKTTGHARFRNLSSMNKLVEVPEHLTSGYCSKPQQNNKILVDT
RVTVSKKKPTKSEKSQTKQKNLLPNLCRFPPSFTGLSPDELWKRRNSIETISELLRLLDINREHSETALVP
YTMNSQIVLFGGGAGAIVPVTPVKKPRPRPKVDLDDETDRVWKLLLENINSEGVDGSDEQKAKWWEEERNV
FRGRADSFIARMHLVQGDRRFTPWKGSVVDSVVGVFLTQNVSDHLSSSAFMSLASQFPVPFVPSSNFDAGT
SSMPSIQITYLDSEETMSSPPDHNHSSVTLKNTQPDEEKDYVPSNETSRSSSEIAISAHESVDKTTDSKEY
VDSDRKGSSVEVDKTDEKCRVLNLFPSEDSALTCQHSMVSDAPQNTERAGSSSEIDLEGEYRTSFMKLLQG
VQVSLEDSNQVSPNMSPGDCSSEIKGFQSMKEPTKSSVDSSEPGCCSQQDGDVLSCQKPTLKEKGKKVLKE
EKKAFDWDCLRREAQARAGIREKTRSTMDTVDWKAIRAADVKEVAETIKSRGMNHKLAERIQGFLDRLVND
HGSIDLEWLRDVPPDKAKEYLLSFNGLGLKSVECVRLLTLHHLAFPVDTNVGRIAVRLGWVPLQPLPESLQ
LHLLEMYPMLESIQKYLWPRLCKLDQKTLYELHYQMITFGKVFCTKSKPNCNACPMKGECRHFASAFASAR
LALPSTEKGMGTPDKNPLPLHLPEPFQREQGSEVVQHSEPAKKVTCCEPIIEEPASPEPETAEVSIADIEE
AFFEDPEEIPTIRLNMDAFTSNLKKIMEHNKELQDGNMSSALVALTAETASLPMPKLKNISQLRTEHRVYE
LPDEHPLLAQLEKREPDDPCSYLLAIWTPGETADSIQPSVSTCIFQANGMLCDEETCFSCNSIKETRSQIV
RGTILIPCRTAMRGSFPLNGTYFQVNEVFADHASSLNPINVPRELIWELPRRTVYFGTSVPTIFKGLSTEK
IQACFWKGYVCVRGFDRKTRGPKPLIARLHFPASKLKGQQANLA

A construct reporting ROS1 transcription is generated as described above, and further mutagenized. Mutants displaying enhanced reporter levels are isolated. The candidate enhancements of ROS1 transcription are then expressed under a strong 35S promoter or pathogen-inducible promoters (*e.g.*, PR1, WRKY6) and stable transgenic lines generated to confer enhanced disease resistance to pathogens. By constitutively enhancing the expression of positive regulators of ROS1 increased resistance to bacterial and fungal pathogens is achieved in a variety of plants, including crops. The positive regulators of DCL4 transcription, obtained as described above, are further overexpress, conditionally or constitutively, in the same transgenic lines to confer, additionally, enhanced resistance to viral pathogens.

Furthermore, the same transgenic lines reporting ROS1 transcription is used to screen for chemical compounds that enhance GFP expression, as described above. Molecules that enhance GFP mRNA levels may be used to confer antibacterial and antifungal resistance in a variety of plant species including crops. Cocktails of chemical agents that promote DCL4 transcription as well as ROS1 transcription are used to confer broad spectrum resistance to unrelated pathogens.

Sequences from ROS1 predicted promoter are:
ROS I promoter sequence:

ATAATCCGTTCCCAACTTTTATCCACTATTATTCGTCTCAGTTTCTAGGATAGATATGTCCACACAAAAA
AGCTCTTGATTTTTTTTTTTTTTTTTACAAATTCCAAATTTCTTTGCTCATAACCCAATCATTAGGTTATG
ACCACCATTGACTCACTCATAAGTCATAAGTCATAGGCTCATAACCAATCCAACAAGTTGTTAAGATTGAC
AACAACGATTCACTAAGATTCCAACCAAGTCCATGAAATAAATGATTTACAATACTCATTTCTCATGTACG
TCTCTTTGAAGGTTTCTTGCATGACAGGAAATCAAAGGTTAGCACACTAATTACTCTTTTTTTCACACACA

TTCACAGTTTCACACATATGGTGCAGTATTTTGACTCCTATCGTACTAGACTAAAACATTTGGAATGATCA
AAAACGAAAGACTCGTTGGGCAACTAGCCTAATAATCACTCTACTACACTAGCTCCCATATCAGTGGAAAA
TAATAATTCTAAAACGAATCATTTAACTTCTGCATATGTAAACGAAAACGTGTAAATTTATGAGATTACGT
AAAAATTAGCAAAATAATATATTATTGATCAAAATTATAAACGTGGATTACATAACATGTTATTTGTTTAA
ATCATAATTTGATGATAAATTTATAAATAAAGTTCTAATTATTTTATATCTAAAGCAAAATTAAGATTATT
TTATAATTTCTATTAATTATAAAATTAGTTAGTTCATATAATTTTAAATAGTTACGTAAACGAGAAAAAAT
ACGAAATTTTAAAGAGAAAAAGATAACAGAAAAGACGATGATGACGATGACGATAACAACGACAATATTAT
TAACTTTTTAAATCATCTTTCCCATAGTCTAGGAGATTTTGTAGAAAAGAATCATTATTTTAAAATAAAA
TTCCGTAAAACTTTTCCCGCCAACCAAACGAACTTTCGCCCTACATAAACAAACGGTTATGAAAAATAGTG
AAACACACAACAACACATGTTATATCCTCTTCTTTATACGTTAGGCCAAAAAAGCTTTTTCTATATTACTC
TTTAACTTCATCGATTCCAAGAGAAGAAACGAAGCATCAGTGATCTTATCCTCTCATAGCTACCACCGAAC
TAACTCTCTCCATCACCACCATAACCATTGATTCTACTGGTAATGAATTTTGTTTTTTTCTTACTTTTTTT
TACATTTTGTTGTGAATCTAAAAAGTCTCTCTTTCACCTAACGAACGGATTAATCGTTCATGTCGCCACTC
ACCCAAAATCAATGACTTCCGGAGATCTCTCTTTCTCTAAAACCCCAGAAAAAAGTGGATCTGATCATTTT
ATAAAATCGTGATTTTTAAAAAAAATTGGTGATCTCTTTTATTGAAGAAATTATTGAACTTTTTGCAGTGG
AAAAAATAGAAAGTTCCAAGCTTTTTCTCAAATGGTTCTGATTTAAGTAAGAGTGAAGAAAAGTAAAAATA
GAGTCAGAA

## Claims

1. A method to enhance the resistance of a plant to a pathogen which method comprises modifying said plant to contain a nucleic acid construct which comprises constitutive or pathogen responsive control sequences operatively linked to a nucleotide sequence the expression of which is upregulated when resistance response to said pathogen is elicited,
wherein the nucleotide sequence is the nucleotide sequence encoding the DNA glycosylase ROS1.

## Patentansprüche

1. Ein Verfahren zur Verbesserung der Resistenz einer Pflanze gegen ein Pathogen, wobei das Verfahren die Veränderung der Pflanze umfasst, so dass sie ein Nukleinsäurekonstrukt enthält, welches konstitutive Kontrollsequenzen oder Kontrollsequenzen als Antwort auf das Pathogen umfasst, die operativ mit einer Nukleotidsequenz verbunden sind, deren Ausdruck hochreguliert wird, wenn eine Resistenzantwort auf das Pathogen hervorgerufen wird,
wobei die Nukleotidsequenz die Nukleotidsequenz ist, die DNA Glycosylase ROS1 kodiert.

## Revendications

1. Procédé pour augmenter la résistance d'une plante à un pathogène, lequel procédé comprend la modification de la plante afin de contenir une construction d'acide nucléique qui comprend des séquences de contrôle constitutives ou en réponse au pathogène liées de manière opérationnelle à une séquence de nucléotides dont l'expression est régulée à la hausse lorsque la réponse de résistance audit pathogène est suscitée, dans lequel la séquence de nucléotides est la séquence de nucléotides codant pour l'ADN glycosylase ROS1.
